(19) Europäisches Patentamt
European Patent Office
Office européen des brevets

(11) EP 4 700 018 A1

(12) EUROPEAN PATENT APPLICATION
published in accordance with Art. 153(4) EPC

(43) Date of publication:
**25.02.2026 Bulletin 2026/09**

(21) Application number: **24791929.3**

(22) Date of filing: **12.04.2024**

(51) International Patent Classification (IPC):
*C07D 401/04* (2006.01) *C07D 401/12* (2006.01)
*C07D 213/78* (2006.01) *C07D 213/82* (2006.01)
*C07D 317/68* (2006.01) *C07C 257/18* (2006.01)
*C07C 259/18* (2006.01) *C07C 257/20* (2006.01)
*C07C 261/04* (2006.01) *A61K 31/44* (2006.01)
*A61K 31/36* (2006.01) *A61K 31/167* (2006.01)
*A61K 31/277* (2006.01) *A61P 29/00* (2006.01)
*A61P 25/04* (2006.01) *A61P 19/02* (2006.01)

(86) International application number:
**PCT/CN2024/087393**

(87) International publication number:
**WO 2024/217344 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.04.2023 CN 202310422192**

(71) Applicant: **Shanghai Institute of Materia Medica, Chinese Academy of Sciences**
**Shanghai 201203 (CN)**

(72) Inventors:
- **GAO, Zhaobing**
  **Shanghai 201203 (CN)**
- **YANG, Chunhao**
  **Shanghai 201203 (CN)**
- **TAN, Cun**
  **Shanghai 201203 (CN)**
- **ZHENG, Yueming**
  **Shanghai 201203 (CN)**
- **FU, Linshu**
  **Shanghai 201203 (CN)**
- **XU, Haiyan**
  **Shanghai 201203 (CN)**
- **CHEN, Xiaoyan**
  **Shanghai 201203 (CN)**
- **HU, Youhong**
  **Shanghai 201203 (CN)**
- **XIONG, Bing**
  **Shanghai 201203 (CN)**
- **HE, Qian**
  **Shanghai 201203 (CN)**
- **WEI, Aihuan**
  **Shanghai 201203 (CN)**
- **ZHANG, Xiaofei**
  **Shanghai 201203 (CN)**
- **WANG, Linlin**
  **Shanghai 201203 (CN)**

(74) Representative: **Kador & Partner Part mbB**
**Corneliusstraße 15**
**80469 München (DE)**

# (54) NAV1.8 INHIBITORS, PREPARATION METHOD THEREFOR AND USE THEREOF

(57) Provided in the present invention are Nav1.8 inhibitors shown as formula I, a preparation method therefor and the use thereof. The compounds provided by the present invention have good Nav1.8 selective inhibitory activity, good pharmacokinetic properties and good druggability, can be used as Nav1.8 inhibitors for preventing and/or treating diseases related to abnormal Nav1.8 channel expression activity, and thus have significant clinical application value.

EP 4 700 018 A1

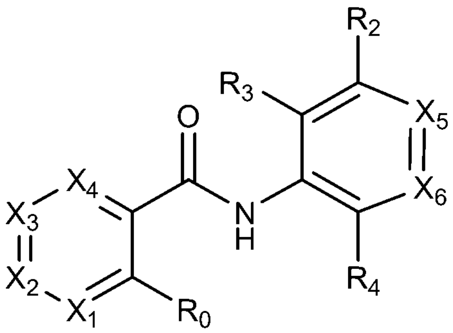

I

## Description

### TECHNICAL FIELD

The present invention relates to the technical field of sodium channel inhibitor synthesis, in particular to a class of Nav1.8 inhibitors, their preparation method and use in preparing drugs for preventing and/or treating diseases associated with abnormal expression of Nav1.8 channel.

### BACKGROUND

Voltage-gated sodium channels (VGSCs or Nav) mediate the selective transmembrane flow of sodium ions and play a key role in mediating the initiation, conduction, and transmission of action potentials in excitable cells such as neurons (Catterall et al., Pharmacol Rev. 2005, 57(4): 397-409.). The Nav family includes 9 subtypes, namely Nav1.1 to Nav1.9, which regulate various important physiological and pathological processes in the human body by acting on Nav (Black et al., Neuron. 2013, 80(2): 280-91; Catterall et al., Annu Rev Pharmacol Toxicol. 2014, 54: 317-38; Bennett et al., Physiol Rev. 2019, 99(2):1079-1151.). Nav1.1, Nav1.2, Nav1.3, and Nav1.6 are expressed in both the central and peripheral nervous systems, Nav1.4 is primarily expressed in skeletal muscle, Nav1.5 is primarily expressed in cardiomyocytes, and Nav1.7, Nav1.8, and Nav1.9 are primarily expressed in the peripheral nervous system. These 9 subtypes have similar functional behaviors but differ in specific aspects of voltage dependence and kinetic behavior.

As a subtype of Nav, Nav1.8 is primarily expressed in neurons that transmit pain signals in the dorsal root ganglion (DRG). Nav1.8 channel has high activation and inactivation voltages, making it the main component of the rise of the action potential (other Nav channel subtypes are already in a nonfunctional inactivated state) (Goodwin et al., Nat Rev Neurosci. 2021, 22(5): 263-274.).

It has been confirmed that Nav1.8 in the Nav family is closely related to the occurrence of many diseases:

1) Nav1.8 is involved in the occurrence of pain: The characteristics of slow inactivation and rapid activation of Nav1.8 channel make it involved in the physiological and pathological processes of membrane potential depolarization and high-frequency neuronal discharge, such as pain (Alsaloum et al., Nat Rev Neurol. 2020, 16(12): 689-705.). Human genetic studies have shown that mutations in the Nav1.8 gene cause small fiber neuralgia and erythropoiesis (Faber et al., Proc Natl Acad Sci USA. 2012, 109(47): 19444-9; Kaluza et al., Pflugers Arch. 2018, 470(12): 1787-1801.). In rodents, gene knockout or knockdown of the Nav1.8 channel gene could alleviate various inflammatory pain and neuropathic pain; and administration of Nav1.8 inhibitors such as A-803467 could effectively alleviate pain responses (Jarvis et al., Proc Natl Acad Sci USA. 2007, 104(20): 8520-5.). In a mouse model of diabetic neuropathic pain, methylglyoxal directly enhanced Nav1.8 channel function, and gene knockout or knockdown of Nav1.8 channel could effectively alleviate methylglyoxal-induced neuropathic pain (Bierhaus et al., Nat Med. 2012, 18(6): 926-33.). In a rat model of STZ-induced diabetic neuropathy, intraperitoneal or intraplantar administration of the Nav1.8 inhibitor A-803467 could dose-dependently alleviate the animals' pain behavioral responses (Mert et al., J Am Assoc Lab Anim Sci. 2012, 51(5): 579-85.).

2) Nav1.8 is involved in the development of multiple sclerosis: Multiple sclerosis (MS) is an inflammatory demyelinating disease originating in the central nervous system, and its exact pathogenesis remains to be elucidated. The Purkinje fibers in the cerebellum of normal people do not express Nav1.8 channel. However, the expression of Nav1.8 in the cerebellum of patients with multiple sclerosis is upregulated, and the expression level of the channel increases in a disease-dependent manner. The single nucleotide polymorphism (SNP) of the Nav1.8 encoding gene is also related to the severity of MS (Craner et al., J Neuropathol Exp Neurol. 2003, 62(9):968-75; Roostaei et al., Neurology. 2016, 86(5):410-7.). Mice (L7-1.8TG) with knock-in (overexpression) of Nav1.8 in the cerebellar Purkinje fibers exhibited multiple sclerosis behaviors. Administration of the Nav1.8 selective inhibitor PF-01247324 could alleviate the MS behaviors of L7-1.8TG transgenic mice (Shields et al., Ann Neurol. 2012, 71(2): 186-94; Shields et al., PLoS One. 2015, 10(3): e0119067.).

3) Nav1.8 is involved in the development of osteoarthritis inflammation: osteoarthritis is a degenerative bone and joint disease characterized by cartilage wear and pain. The direct binding of phosphorylated cAMP response element binding protein (CREB) to the promoter of the Nav1.8 encoding gene could promote the transcription of Nav1.8 protein and upregulate the expression level of the Nav1.8 channel (Zhu et al., Elife. 2020, 9: e57656.).

4) Nav1.8 is involved in the occurrence of cardiovascular diseases: in the cardiovascular system, Nav1.8 channel has been shown to be expressed in cardiac nerves such as Purkinje fibers, and some studies have also suggested that Nav1.8 is also expressed in cardiomyocytes (Verkerk et al., Circ Res. 2012, 111(3): 333-43.). Human genetic studies have found that Nav1.8 gene mutations are associated with Brugada syndrome (Hu et al., J Am Coll Cardiol. 2014, 64(1): 66-79.). Inhibiting Nav1.8 channel can improve cardiac remodeling, and Nav1.8 channel is considered to be potential therapeutic targets for cardiovascular diseases such as arrhythmias, atrial fibrillation, and heart failure

(Dybkova et al., Cardiovasc Res. 2018, 114(13): 1728-1737.).
5) Nav1.8 is involved in the development of pathological cough: Nav1.8 channel is expressed in the cough-related vagal plexus. During pathological cough, Nav1.8 phosphorylation levels and expression levels increase, indicating that it is involved in the cough reflex (Muroi et al., Lung. 2014, 192(1): 15-20.).
6) Nav1.8 is involved in the development of itch behavior: In mammalian itch perception, itch factors such as histamine released by lymphocytes and mast cells, etc., can activate Nav1.8 channel. Knocking out Nav1.8 in mice can effectively alleviate histamine- and endothelin-induced itch behavior (Riol-Blanco et al., 2014, 510(7503): 157-61.).
7) In addition, congenital mutations in human Nav1.8 have been reported to cause epilepsy, convulsions and other diseases (Kambouris et al., Ann Clin Transl Neurol. 2016, 4(1): 26-35.).

As mentioned above, given the high relevance of Nav1.8 in the treatment of pain (such as small fiber neuralgia, erythromelalgia, and diabetic neuropathy) and other related diseases (such as multiple sclerosis, osteoarthritis, arrhythmias, atrial fibrillation, pruritus associated with heart failure, epilepsy, convulsions, and pathological cough), inhibitors of the Nav1.8 channel are considered promising for the treatment, prevention, or control of diseases associated with Nav1.8 channel participation or dysfunction.

Inhibitors specifically targeting Nav1.8 that have entered the clinical trials currently include VX-548 (Phase III), HRS-4800 (Phase II), VX-150 (Phase I), and JMKX-000623 (Phase I), but no products are currently marketed. Therefore, the development of new Nav1.8 inhibitors holds significant scientific and clinical significance for the treatment of diseases associated with dysregulated Nav1.8 expression.

## SUMMARY OF THE INVENTION

One object of the present invention is to provide a compound of Formula I having selective Nav1.8 inhibitory activity, or a tautomer, stereoisomer, deuterated substance, or pharmaceutically acceptable salt thereof.

A second object of the present invention is to provide a method for preparing the compound of Formula I.

A third object of the present invention is to provide a pharmaceutical composition comprising the compound of Formula I, or a tautomer, stereoisomer, deuterated substance, or pharmaceutically acceptable salt thereof.

A fourth object of the present invention is to provide the compound of Formula I, or a tautomer, stereoisomer, deuterated substance, or pharmaceutically acceptable salt thereof, or the aforementioned pharmaceutical composition, for use in the preparation of a Nav1.8 inhibitor.

A fifth object of the present invention is to provide the compound of Formula I, or a tautomer, stereoisomer, deuterated substance, or pharmaceutically acceptable salt thereof, or the aforementioned pharmaceutical composition, for use in the preparation of a medicament for preventing and/or treating diseases associated with abnormal expression of Nav1.8 channel.

In one aspect, the present invention provides a compound of Formula I, or a tautomer, stereoisomer, deuterated substance, or pharmaceutically acceptable salt thereof,

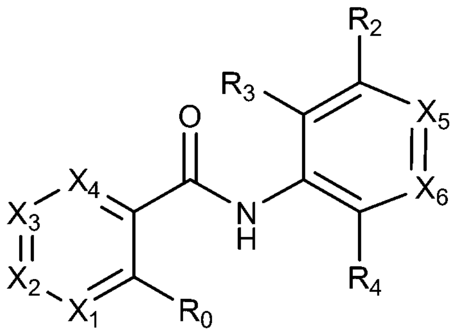

I

wherein:

$R_0$ is

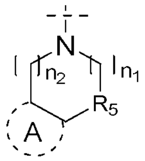 or 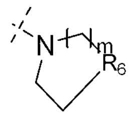,

Ring A is selected from substituted or unsubstituted C6-C10 aryl, and substituted or unsubstituted 5-10 membered heteroaryl, wherein the substituted means being substituted by 1, 2, 3 or 4 substituents selected from the group consisting of halogen, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkoxy;

n, is 0, 1, 2, 3 or 4; $n_2$ is 0 or 1; m is 0, 1, 2, 3 or 4;

$R_5$ is O, S, C=O, $CR_{5a}$,$R_{5b}$ or $NR_{5c}$, wherein $R_{5a}$ and $R_{5b}$ are each independently selected from hydrogen and halogen; $R_{5c}$ is selected from hydrogen, substituted or unsubstituted C1-C4 alkyl, and substituted or unsubstituted C3-C6 cycloalkyl, wherein the substituted means being substituted by 1, 2, or 3 substituents selected from the group consisting of halogen, C1-C4 alkyl, halogenated C1-C4 alkyl, C1-C4 alkoxy, and halogenated C1-C4 alkoxy;

$R_6$ is O, S, C=O, $CR_{6a}R_{6b}$, or $NR_{6c}$, wherein $R_{6a}$ and $R_{6b}$ are each independently selected from hydrogen and halogen; or $CR_{6a}R_{6b}$ forms substituted or unsubstituted C3-C6 cycloalkyl, substituted or unsubstituted 3-8 membered heterocyclyl (as one ring of an azaspirocycle, with the carbon atom to which $R_{6a}$ and $R_{6b}$ are attached serving as the spiro atom), [substituted or unsubstituted C3-C6 cycloalkyl] fused [substituted or unsubstituted phenyl]; [substituted or unsubstituted C3-C6 cycloalkyl] fused [substituted or unsubstituted 5-6 membered heteroaryl], [substituted or unsubstituted 3-8 membered heterocyclyl] fused [substituted or unsubstituted phenyl], [substituted or unsubstituted 3-8 membered heterocyclyl] fused [substituted or unsubstituted 5-6 membered heteroaryl], [substituted or unsubstituted cyclopentenyl] fused [substituted or unsubstituted phenyl], or [substituted or unsubstituted cyclopentenyl] fused [substituted or unsubstituted 5-6 membered heteroaryl]; $R_{6c}$ is selected from hydrogen, substituted or unsubstituted C1-C4 alkyl, and substituted or unsubstituted C3-C6 cycloalkyl, wherein the substituted means being substituted by 1, 2 or 3 substituents selected from the group consisting of halogen, C1-C4 alkyl, halogenated C1-C4 alkyl, C1-C4 alkoxy, and halogenated C1-C4 alkoxy,

$X_1$ is N or $CR_a$, $X_2$ is N or $CR_b$, $X_3$ is N or $CR_c$, $X_4$ is N or $CR_d$, and at most two N atoms are present in $X_1$, $X_2$, $X_3$, and $X_4$;

$R_a$, $R_b$, $R_c$, and $R_d$, at each occurrence, are independently selected from hydrogen, halogen, cyano, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkoxy; in particular, $R_a$ is hydrogen; $R_b$ is selected from $-CF_3$, methyl, cyclopropyl, isopropyl, and -Cl; $R_c$ is selected from -Cl, $-CF_3$, cyano, and ethynyl; $R_d$ is selected from hydrogen, -Cl, and -F;

One of $X_5$ and $X_6$ is $CR_1$, and the other is N or $CR_e$;

$R_e$, at each occurrence, is selected from hydrogen, halogen, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkoxy; preferably selected from -Cl, -F, Br, hydrogen, methoxy, and methyl;

$R_1$ is

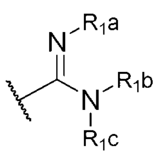, 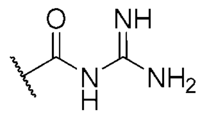, 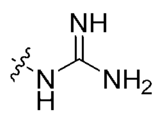 or 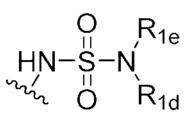;

or $R_1$ and $R_e$, together with the carbon to which they are attached, form

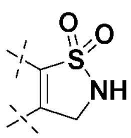;

wherein, $R_{1a}$ is selected from hydrogen, -CN, -OH, and C1-C6 alkoxy; $R_{1b}$, $R_{1c}$, $R_{1e}$, and $R_{1d}$ are each independently selected from hydrogen, C1-C6 alkyl, and C3-C6 cycloalkyl;

$R_2$, $R_3$, and $R_4$ are each independently selected from hydrogen, halogen, substituted or unsubstituted C1-C3 alkyl, substituted or unsubstituted C1-C3 alkoxy, and substituted or unsubstituted C3-C6 cycloalkyl, wherein the substituted means being substituted by 1, 2, or 3 substituents selected from the group consisting of C1-C4 alkyl, halogen, -CN, $-NO_2$ and $-NH_2$.

In some embodiments, $R_0$ is

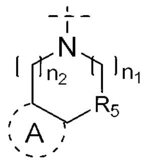

,

and is selected from substituted or unsubstituted indolinyl, substituted or unsubstituted isoindolinyl, substituted or unsubstituted 1,2,3,4-tetrahydroquinolinyl, substituted or unsubstituted tetrahydroisoquinolinyl, substituted or unsubstituted 4,5,6,7-tetrahydrothieno[3,2-c]pyridinyl, and substituted or unsubstituted 4,5,6,7-tetrahydrothieno[2,3-c]pyridinyl, wherein the substituent for the substituted is selected from the group consisting of halogen (fluorine, chlorine, bromine), C1-C4 alkyl, C1-C4 alkoxy, phenyl, and halogenated C1-C4 alkyl (trifluoromethyl).

In some embodiments, $R_0$ is

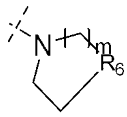

,

and is selected from substituted or unsubstituted tetrahydropyrrolyl, substituted or unsubstituted piperidinyl, substituted or unsubstituted morpholinyl, substituted or unsubstituted thiomorpholinyl, substituted or unsubstituted piperazinyl, substituted or unsubstituted azepanyl, substituted or unsubstituted 1,4-oxazepanyl, substituted or unsubstituted azaspiro [2.5]octanyl, substituted or unsubstituted spiro[indene-1,4'-piperidinyl] (

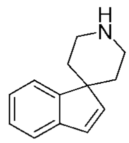

), substituted or unsubstituted 4,5-dihydrospiro[piperidin-4,7'-thieno[2,3-c]pyranyl] (

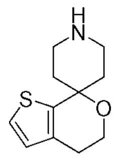

), and substituted or unsubstituted 3H-spiro[isobenzofuran-1,4'-piperidinyl] (

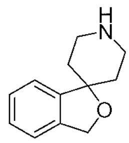

), wherein substituent for the substituted is selected from the group consisting of halogen (fluorine, chlorine, bromine), C1-C4 alkyl, C1-C4 alkoxy, phenyl and halogenated C1-C4 alkyl (trifluoromethyl).

Preferably, $R_0$ is selected from the following groups:

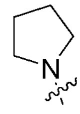 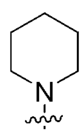 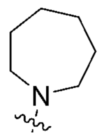 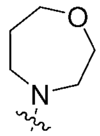 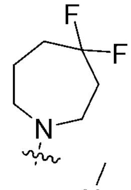 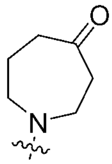 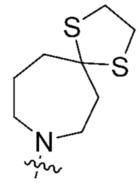

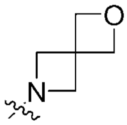 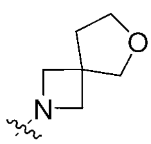 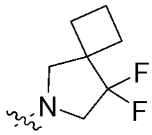 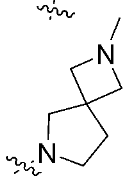 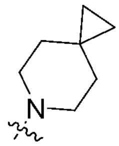 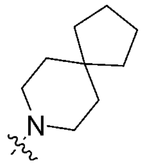

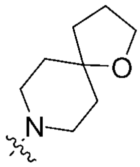 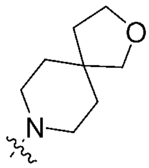 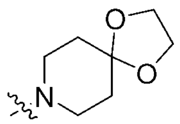 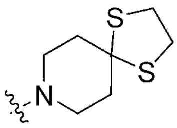 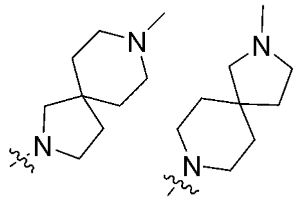 .

In some embodiments, the compound of formula I is selected from the compounds of following formula I-al, formula I-a2, formula I-b1, formula I-b2, formula I-c1, formula I-c2, and formula I-d:

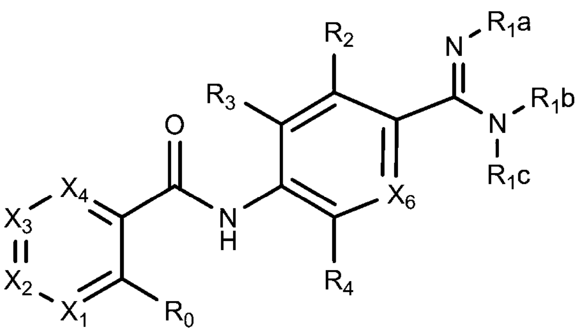

I-a1

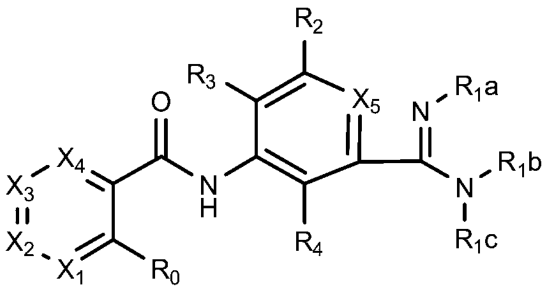

I-a2

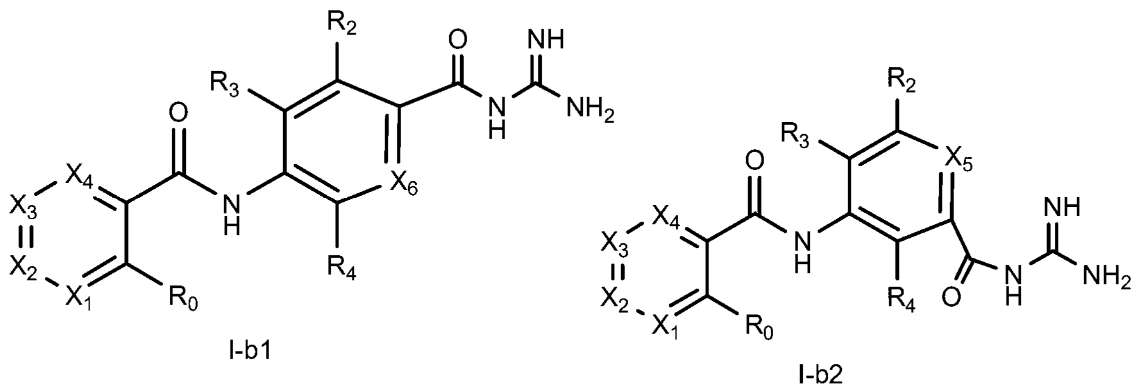

I-b1

I-b2

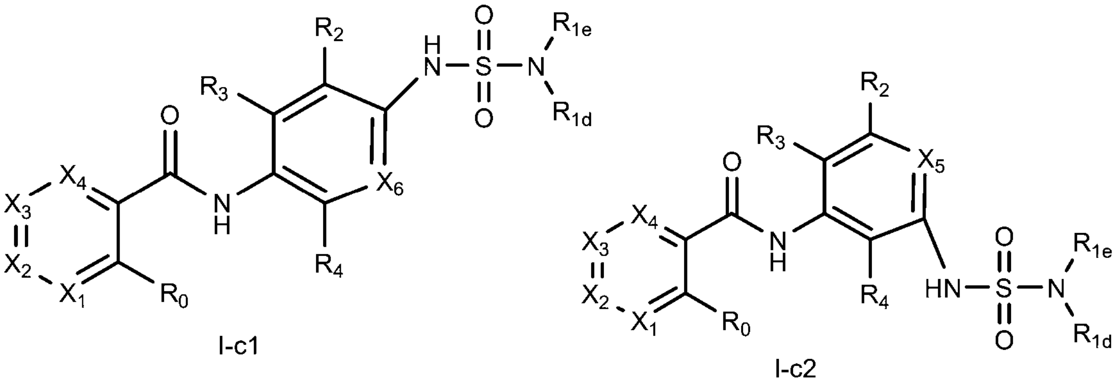

I-c1

I-c2

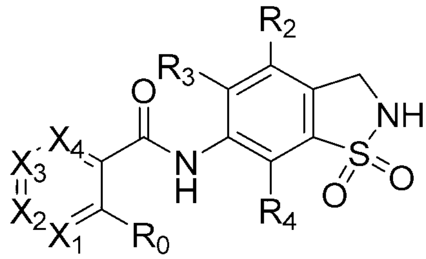

1-d

wherein, $X_5$ and $X_6$ are N or $CR_e$, and $X_1$, $X_2$, $X_3$, $X_4$, $R_0$, $R_{1a}$, $R_{1b}$, $R_{1c}$, $R_{1d}$, $R_{1e}$, $R_2$, $R_3$, $R_4$, and $R_e$ are defined as above.

In some embodiments, the compound of Formula I is selected from the compounds of following Formula II:

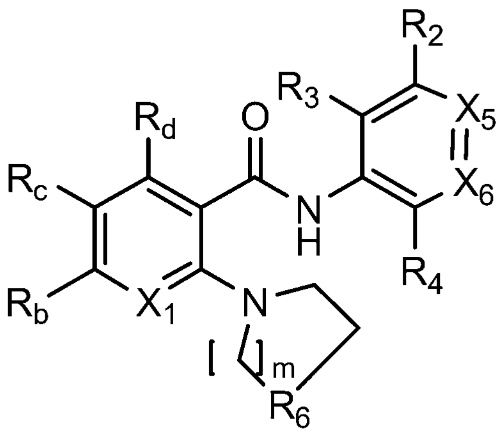

II

wherein, $X_1$ is C or N; m, $R_6$, $R_b$, $R_c$, $R_d$, $R_2$, $R_3$, $R_4$, $X_5$, and $X_6$ are defined as above.

In Formula II,

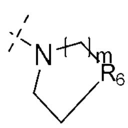

are defined the same as above.

In some embodiments, the compound of Formula II is selected from the compounds of following Formula II-a1, Formula II-a2, Formula II-b1, Formula II-b2, Formula II-c1, Formula II-c2, and Formula II-d:

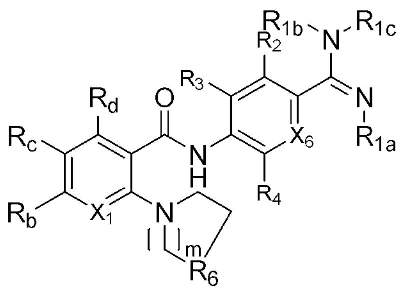

II-a1

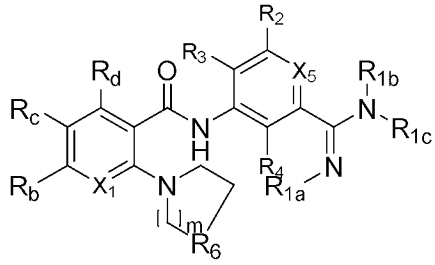

II-a2

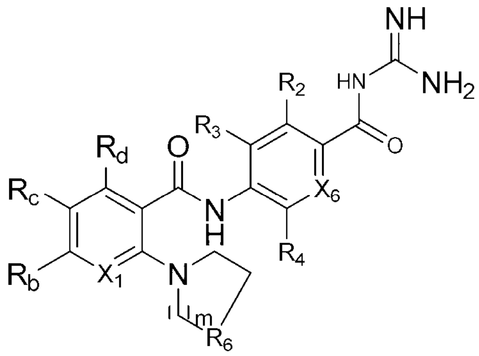

II-b1

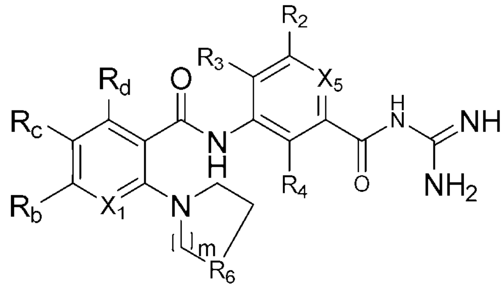

II-b2

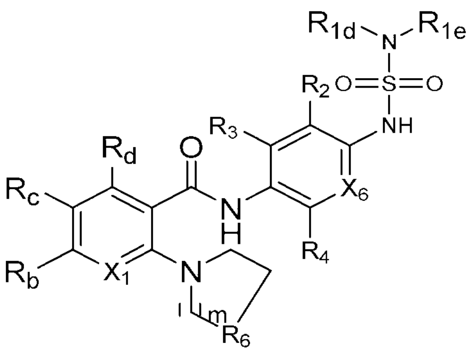

II-c1

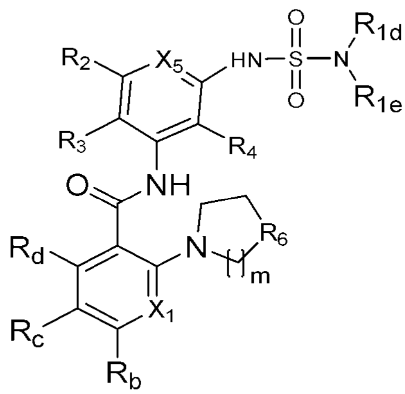

II-c2

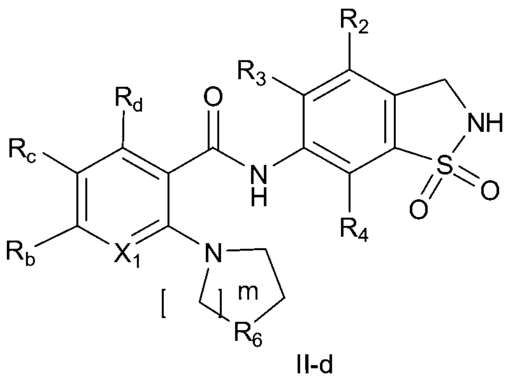

II-d

wherein, $X_1$, m, $R_6$, $R_b$, $R_c$, $R_d$, $R_2$, $R_3$, $R_4$, $X_5$, $X_6$, $R_{1a}$, $R_{1b}$, $R_{1c}$, $R_{1d}$, and $R_{1e}$ are defined as above.

In Formula II-a to Formula II-d,

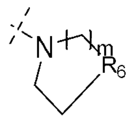

is defined the same as above.

In some embodiments, the compounds of Formulas II-a1 and II-a2 are selected from the compounds of following Formula II-a1-1 or Formula II-a2-1:

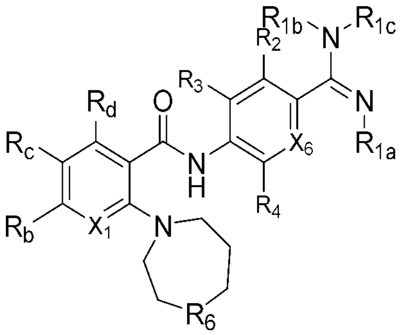

**II-a1-1**

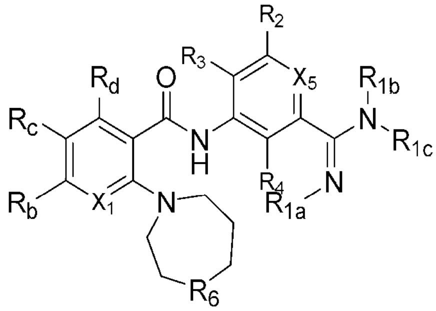

**II-a2-1**

wherein,

$R_{1a}$ is hydrogen, -CN, -OH, or C1-C6 alkoxy, preferably -OH;

$R_{1b}$ and $R_{1c}$ are each independently selected from hydrogen, and C1-C6 alkyl, preferably hydrogen;

$R_6$ is $-CH_2$, -C=O, or $-CF_2$;

$R_b$, $R_c$, and $R_d$ are each independently selected from hydrogen, halogen, cyano, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkoxy; preferably, $R_b$, $R_c$, and $R_d$ are each independently selected from hydrogen, chlorine, fluorine, C1-C4 alkyl, C2-C4 alkynyl, C3-C6 cycloalkyl, and trifluoromethyl;

$X_1$ is N or CH ;

$X_5$ and $X_6$ are N or $CR_e$, wherein $R_e$, at each occurrence, is selected from hydrogen, halogen, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkyloxy; preferably, Re, at each occurrence, is selected from hydrogen, fluorine, chlorine, bromine, methoxy, and methyl;

$R_2$, $R_3$, and $R_4$ are each independently selected from hydrogen, halogen, substituted or unsubstituted C1-C3 alkyl, substituted or unsubstituted C1-C3 alkoxy, and substituted or unsubstituted C3-C6 cycloalkyl, wherein the substituted means being substituted by 1, 2, or 3 substituents selected from the group consisting of methyl, -F, -CN, $-NO_2$, and $-NH_2$.

In some preferred embodiments, the compound of formula II-a2-1 is selected from the compounds of following formula II-a2-1-A:

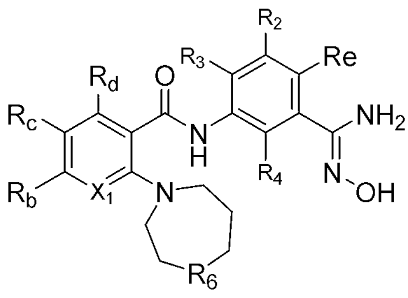

**II-a2-1-A**

$R_e$ is hydrogen, F, Cl, Br, C1-C6 alkyl, or C1-C6 alkoxy, preferably, is hydrogen, F, Cl, or Br, more preferably, is hydrogen or F, especially, is F;

$R_6$ is $-CH_2$, -C=O, or $-CF_2$;

$R_b$, $R_c$, and $R_d$ are each independently selected from hydrogen, halogen, cyano, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkyloxy; preferably, $R_b$, $R_c$, and $R_d$ are each independently selected from hydrogen, chlorine, fluorine, C1-C4 alkyl, C2-C4 alkynyl, C3-C6 cycloalkyl, and trifluoromethyl;

$X_1$ is N or CH;

$R_2$, $R_3$, and $R_4$ are defined as above.

A person skilled in the art would understand that

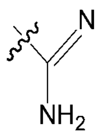 and 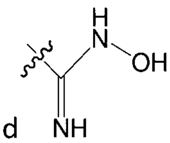

are tautomers, and therefore, compounds containing the structure of

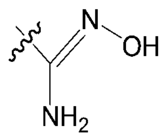

of the present invention can also be considered as containing the structure of

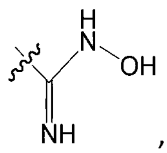 ,

and the two are equivalent.

Similarly,

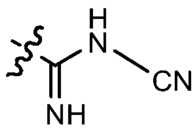

is equivalent to

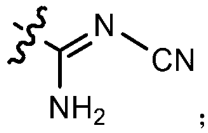 ; 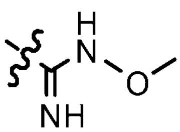

is equivalent to

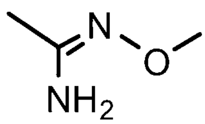 ,

and so on.

Exemplary compounds of the present invention include, but are not limited to:

| | |
|---|---|
| 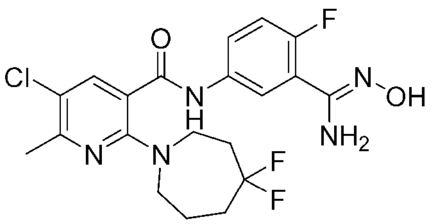<br>I-1 | 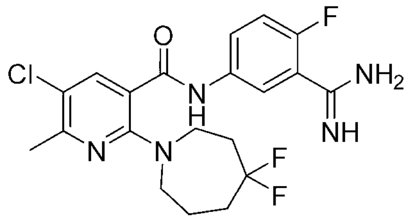<br>I-2 |
| 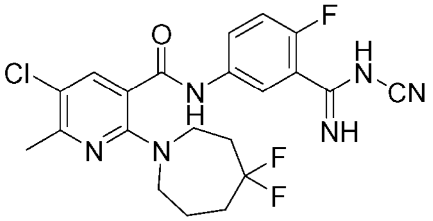<br>I-3 | 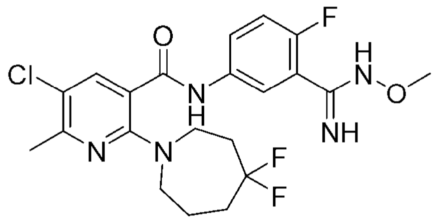<br>I-4 |
| 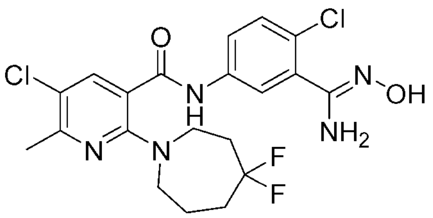<br>I-5 | 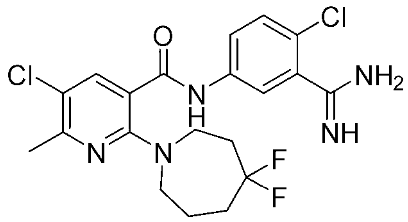<br>I-6 |
| 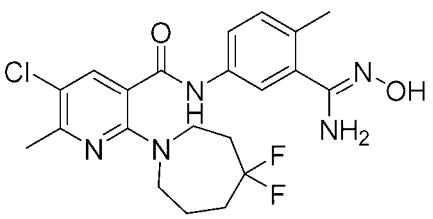<br>I-7 | 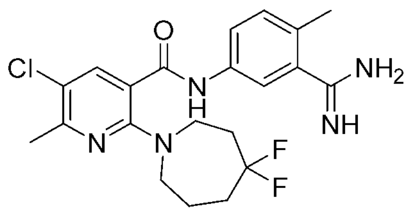<br>I-8 |
| 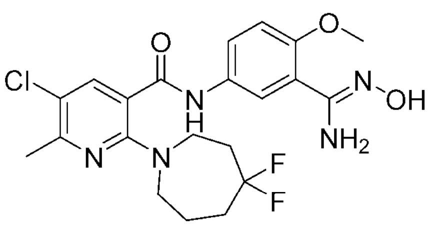<br>I-9 | 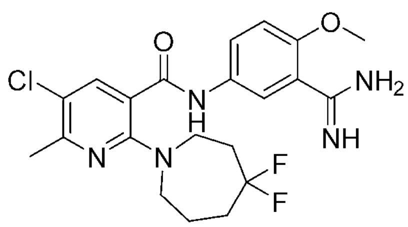<br>I-10 |

| | |
|---|---|
| 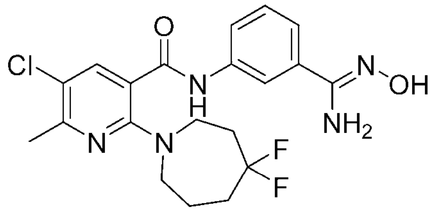 I-11 | 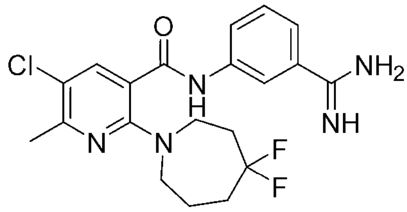 I-12 |
| 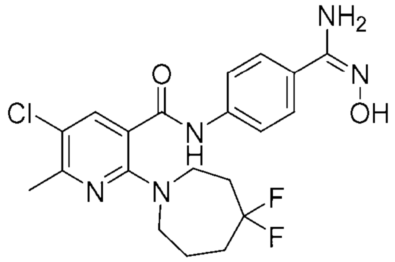 I-13 | 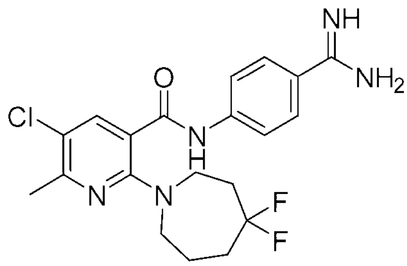 I-14 |
| 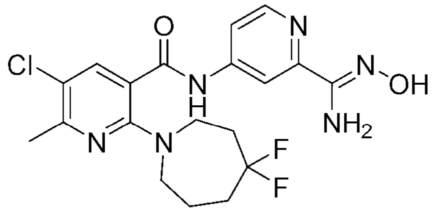 I-15 | 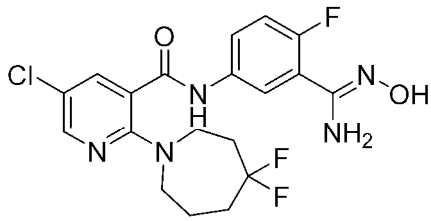 I-16 |
| 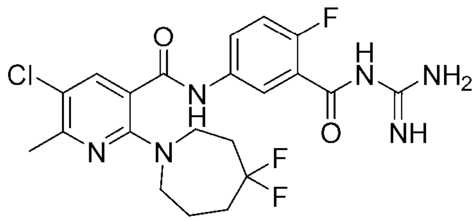 I-17 | 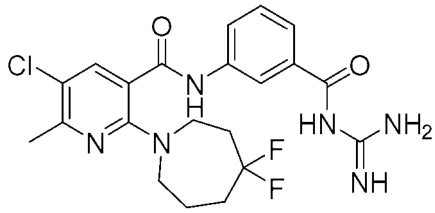 I-18 |
| 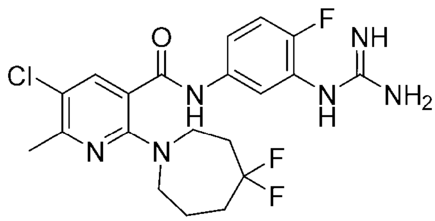 I-19 | 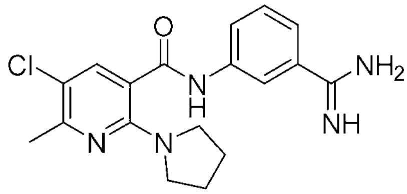 I-20 |
| 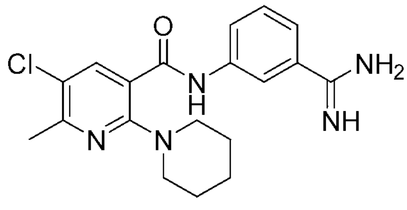 I-21 | 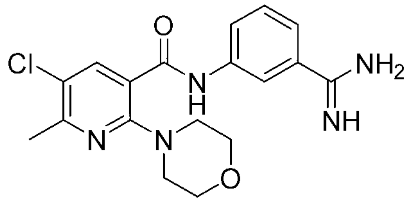 I-22 |

| | |
|---|---|
| 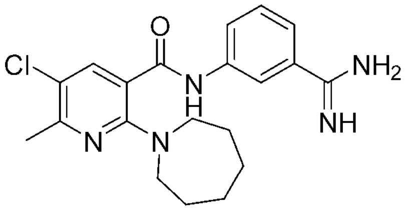 I-23 | 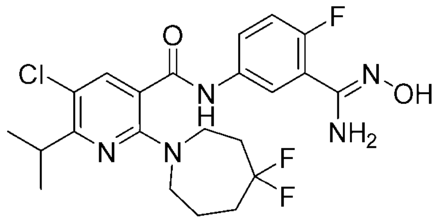 I-24 |
| 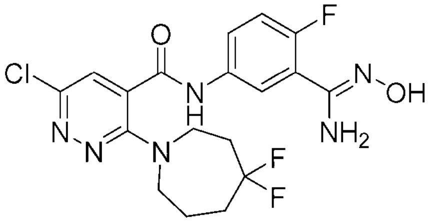 I-25 | 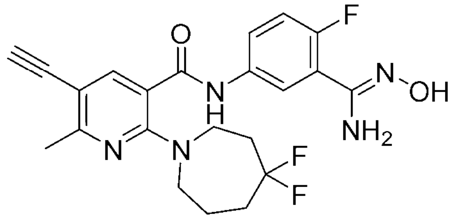 I-26 |
| 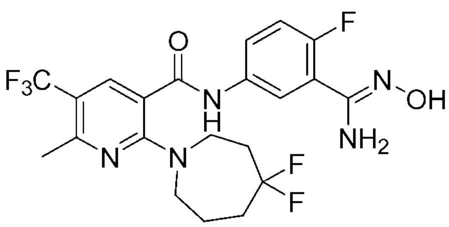 I-27 | 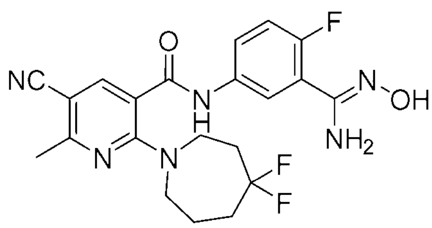 I-28 |
| 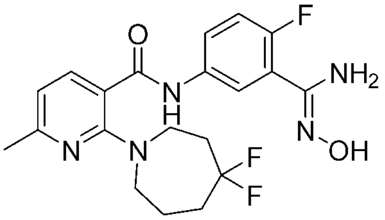 I-29 | 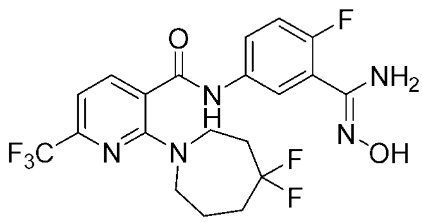 I-30 |
| 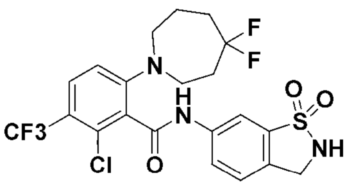 I-31 | 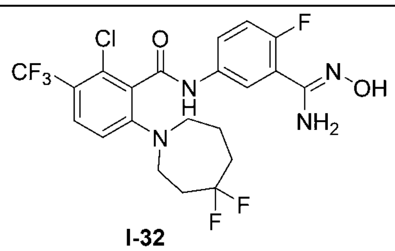 I-32 |
| 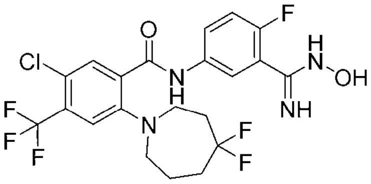 I-33 | 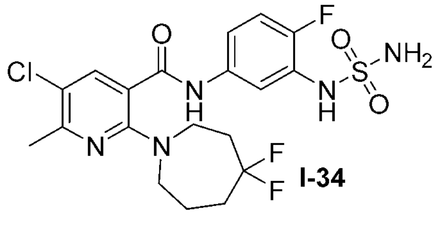 I-34 |

| | |
|---|---|
| 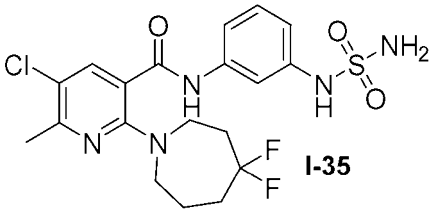 I-35 | 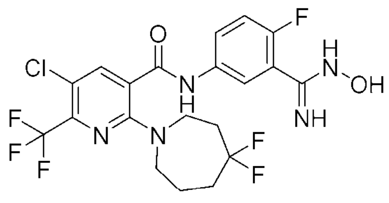 I-36 |
| 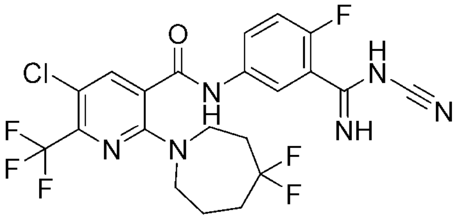 I-37 | 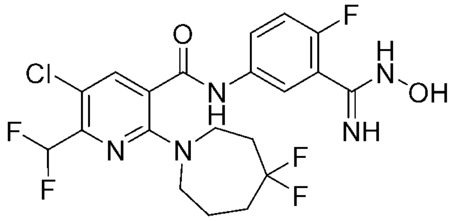 I-38 |
| 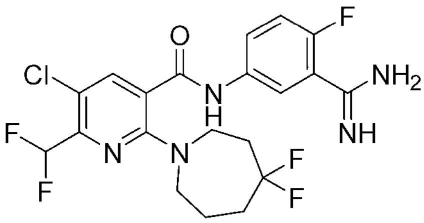 I-39 | 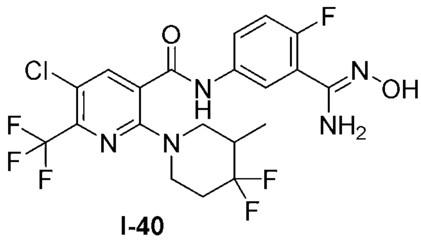 I-40 |
| 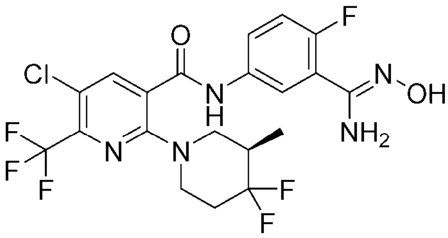 | 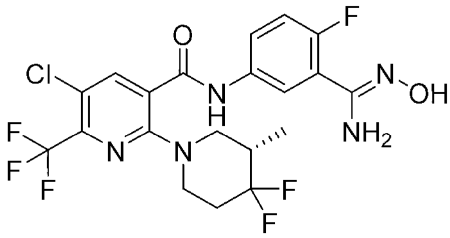 |
| 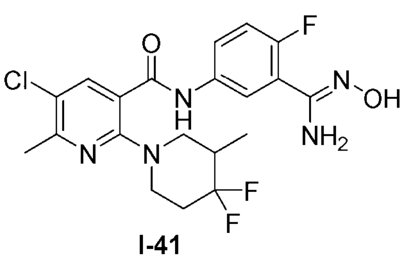 I-41 | 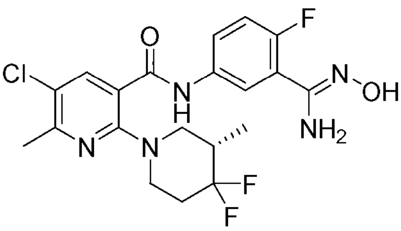 |
| 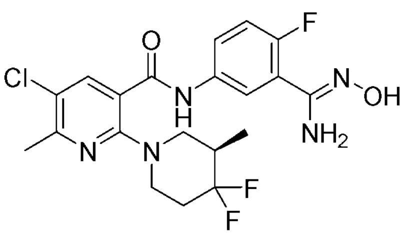 | 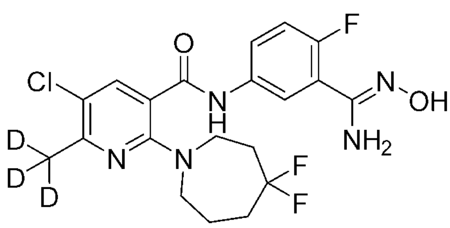 I-42 |

| | |
|---|---|
| 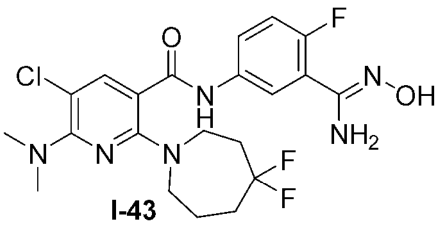 I-43 | 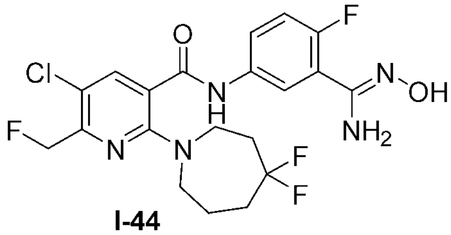 I-44 |
| 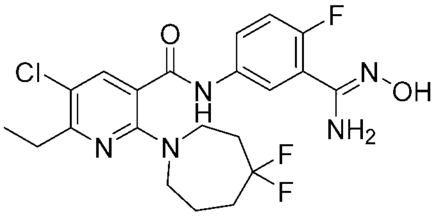 I-45 | 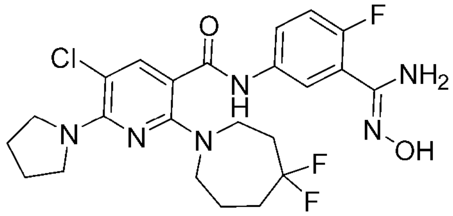 I-46 |
| 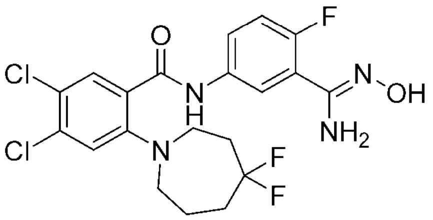 I-47 | 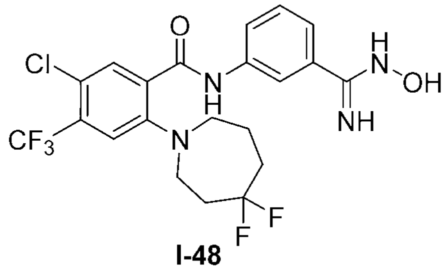 I-48 |
| 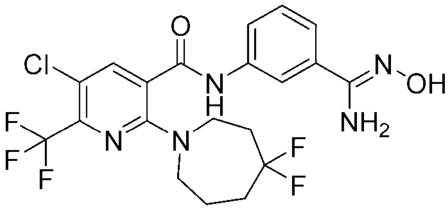 I-49 | |

.

The terms used in the present invention are defined as follows:
"Halogen" may be fluorine, chlorine, bromine, or iodine.

"C1-C6 alkyl" refers to a chain alkyl group having 1-6 carbon atoms; specific examples thereof include, but are not limited to, methyl, ethyl, propyl, n-propyl, isopropyl, butyl, n-butyl, isobutyl, tert-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, and the like. C1-C4 alkyl has similar meanings to the above.

"Halogenated C1-C6 alkyl" refers to a C1-C6 alkyl group as defined above of which one or more hydrogen atoms are replaced by a halogen.

"C2-C6 alkenyl" refers to an alkenyl group having 2-6 carbon atoms; specific examples thereof include vinyl, propenyl, isopropenyl, butenyl, isobutenyl, pentenyl, isopentenyl, and hexenyl, etc.

"Halogenated substituted C2-C6 alkenyl" refers to a C2-C6 alkenyl group as defined above of which one or more hydrogen atoms are replaced by a halogen.

"C2-C6 alkynyl" refers to an alkynyl group having 2-6 carbon atoms; specific examples thereof include ethynyl, propynyl, isopropynyl, butynyl, isobutynyl, pentynyl, isopentenyl, and hexynyl, etc.

"Halogenated substituted C2-C6 alkynyl" refers to a C2-C6 alkynyl group as defined above of which one or more hydrogen atoms are replaced by a halogen.

"C1-C6 alkoxy" refers to an RO- group, where R is a C1-C6 alkyl group as defined above. Specific examples of the alkoxy include methoxy, ethoxy, n-propoxy, and isopropoxy, etc. C1-C4 alkoxy has similar meanings to the above.

"Halogenated C1-C6 alkoxy" refers to an alkoxy group as defined above of which at least one hydrogen atom is replaced by a halogen; specific example thereof includes trifluoromethoxy, etc.

"C2-C6 alkenyloxy" refers to an RO- group, where R is a C2-C6 alkenyl group as defined above; specific examples of the alkenyloxy include ethyleneoxy and propenyloxy.

"Halogenated C2-C6 alkenyloxy" refers to the alkenyloxy group as defined above of which at least one hydrogen atom is replaced by a halogen.

"C2-C6 alkynyloxy" refers to an RO- group, where R is a C2-C6 alkynyl group as defined above; specific examples of the alkynyloxy include ethynyloxy and propynyloxy.

"Halogenated C2-C6 alkynyloxy" refers to an alkynyloxy group as defined above of which at least one hydrogen atom is replaced by a halogen.

"C3-C6 cycloalkyl" refers to a fully saturated monovalent cyclic hydrocarbon group containing 3-6 ring carbon atoms; specific examples thereof include cyclopropyl, cyclobutyl, cyclopentyl, and cyclohexyl. A C4-C8 cycloalkyl group has similar meanings to the above.

"C3-C6 cycloalkoxy" refers to an RO- group, where R is a C3-C6 cycloalkyl group as defined above.

"3- to 8-membered heterocyclyl" refers to a 3- to 8-membered non-aromatic cyclic group containing 1 to 3 heteroatoms selected from N, O, and S. The specific examples thereof include oxetanyl, tetrahydroimidazolyl, and tetrahydrofuranyl. A 5- to 6-membered heterocycly has similar meanings to the above.

"C6- to C10 aryl" refers to a monocyclic or polycyclic aryl containing 6 to 10 carbon atoms. Specific example thereof includes phenyl.

"5- to 10-membered heteroaryl" refers to an aromatic group containing 5 to 10 ring atoms and 1 to 4 heteroatoms selected from nitrogen, oxygen, sulfur, S(=O), and $S(=O)_2$. Specific examples thereof include pyridyl, pyridazinyl, pyrimidinyl, and pyrrolyl. A 5- to 6-membered heteroaryl group has similar meanings to the above.

"Deuterated substance" refers to a compound structure of which one or more hydrogens ($^1H$) are replaced by deuterium ($^2H$/D), where the deuterium content can range from 20% to 100%, preferably 90% to 100%.

"Pharmaceutically acceptable salts" refer to salts that retain the biological efficacy of the free acid or base of a specific compound with no adverse biological effects, such as addition salts of an acid (including organic and inorganic acids) or a base (including organic and inorganic bases). Pharmaceutically acceptable salts of the present invention can be synthesized from parent compounds containing acid or base groups by conventional chemical methods. Generally, they are prepared by reacting the parent compound in its free acid or base form with a stoichiometric amount of a base or acid, in water, an organic solvent, or a mixture of both.

The compounds of the present application and the pharmaceutically acceptable salts, esters, and prodrugs thereof may have isomers, such as, but not limited to, stereoisomers, tautomers, and mixtures thereof. These isomers are also included within the scope of the claims of the present invention.

In another aspect, the present invention provides a method for preparing the compound of Formula I above, which is achieved via the following reaction routes.

Route 1:

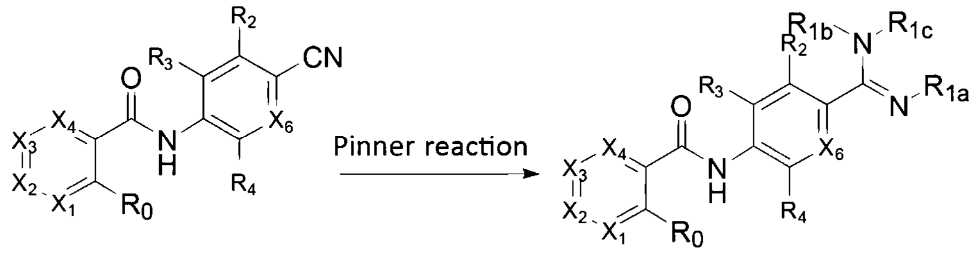


**III-a1** **I-a1**

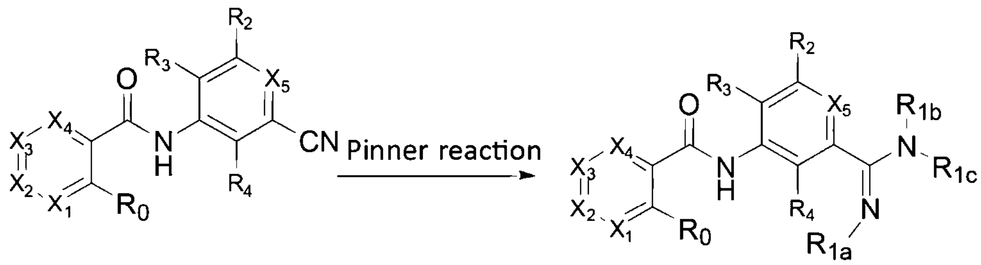


**III-a2** **I-a2**

**III-a1** or **III-a2** undergoes a Pinner reaction to form a Pinner salt, which is then reacted with $NR_{1a}$ and $NR_{1b}R_{1c}$ to prepare **I-a1** or **I-a2;**
wherein, each of the substituents is defined as above.

Specifically, this route is applicable to the preparations in Examples 1 to 36, particularly in Example 3, where a cyanamide was first added, and after the reaction was complete, an ammonia/methanol system was added to obtain **I-3.**

Route 2:

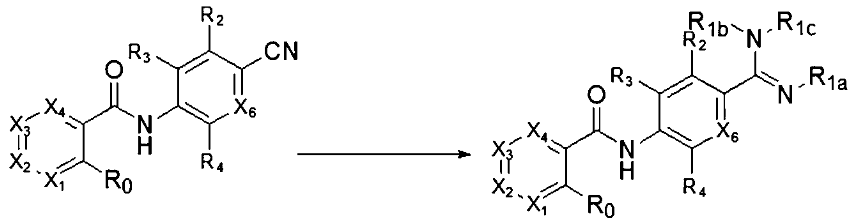

**III-a1** **I-a1** $R_{1a}$=OH,$R_{1b}$=H,$R_{1c}$=H

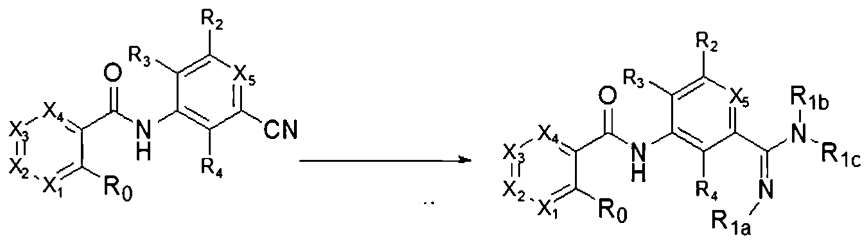

**III-a2** **I-a2**

$R_{1a}$=OH,$R_{1b}$=H,$R_{1c}$=H

**III-a1** or **III-a2** is reacted with hydroxylamine or a salt of hydroxylamine (e.g., in the presence of an anhydrous solvent

and a base) to obtain **I-a1 or I-a2;**
wherein, $R_{1a}$ is OH, $R_{1b}$ is H, $R_{1c}$ is H, and the remaining substituents are defined as above.

The anhydrous organic solvent includes, but is not limited to, one or more of anhydrous methanol, anhydrous ethanol, anhydrous propanol, anhydrous butanol, anhydrous tetrahydrofuran, anhydrous ethyl acetate, etc.; the base is an organic base, including, but not limited to, anhydrous triethylamine, anhydrous N,N-dimethylethylamine, anhydrous 1,8-diazabicyclo[5.4.0]undec-7-ene, 1,5-diazabicyclo[4.3.0]non-5-ene, triethylenediamine, sodium methoxide, potassium ethoxide, potassium tert-butoxide, etc.; the salts of hydroxylamine include, but are not limited to, hydroxylamine hydrochloride, hydroxylamine sulfate, hydroxylamine phosphate, etc.

Route 3:

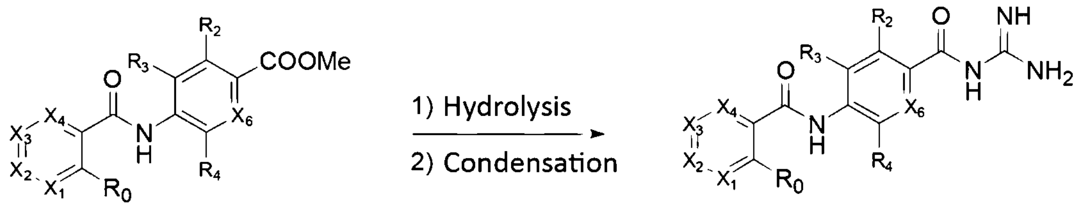


**III-a3** **I-b1**

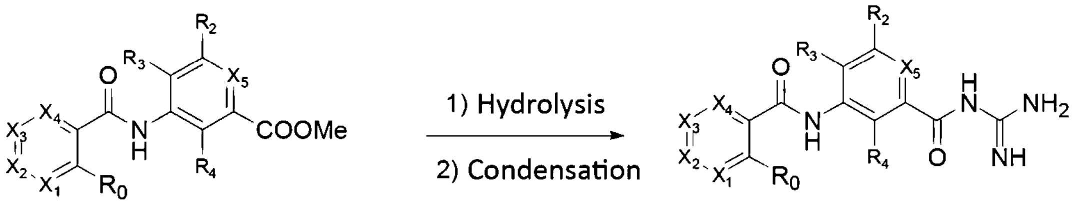


**III-a4** **I-b2**

**III-a3** or **III-a4** is hydrolyzed to produce an acid, which is then converted to an acid chloride (e.g., in the presence of thionyl chloride, oxalyl chloride, or phosphorus trichloride), the acid chloride is then reacted with guanidine via condensation to produce **I-b1** or **I-b2.**

Each of the substituents is defined as above.

Route 5:

Route 4:

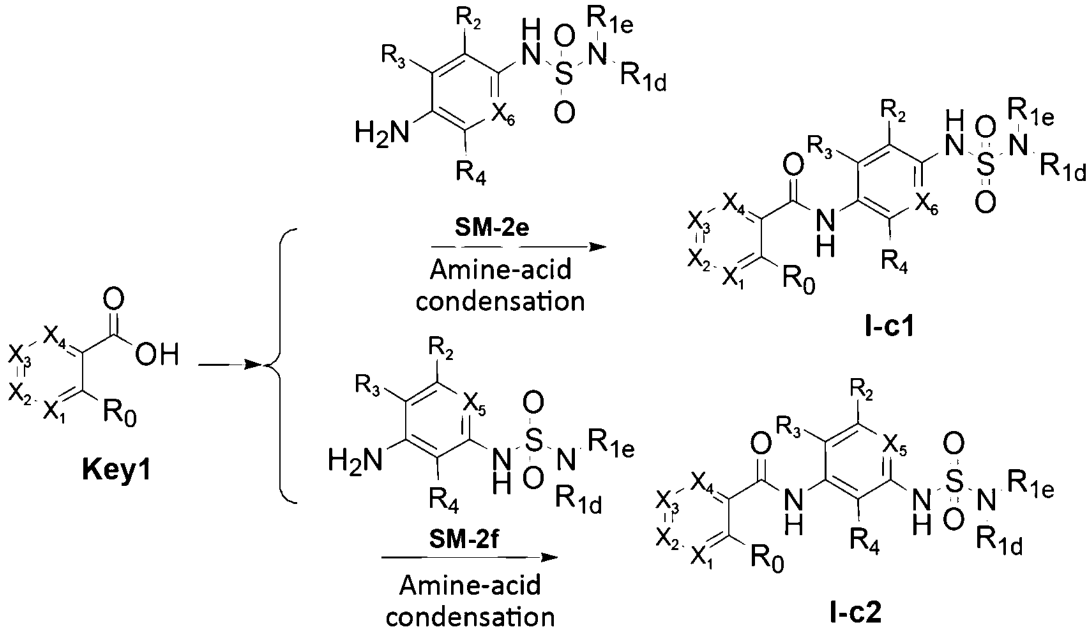


**Key1** is reacted with **SM-2e** or **SM-2f** via amine-acid condensation to produce **I-c1** or **I-c2;** wherein, each of the substituents is defined as above.

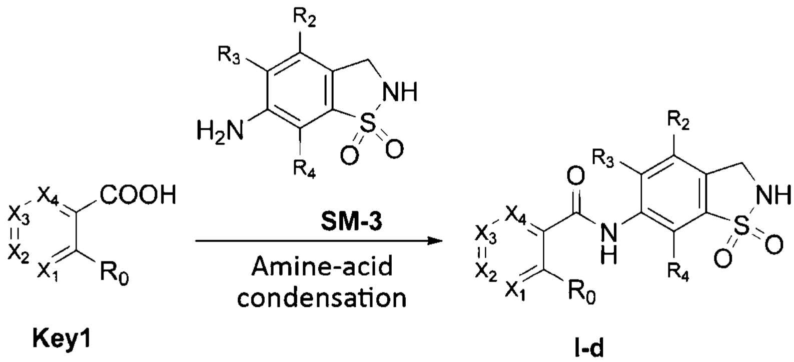


**Key1** is reacted with **SM-3** via amine-acid condensation to produce **I-d;** wherein, each of the substituents is defined as above.

The amine-acid condensation is one of the following Schemes 1 and 2.

Scheme 1:

**Key1** and the reactants (**SM-2e** or **SM-2f, SM-3**) undergo amine-acid condensation in the presence of a condensing agent, such as HATU or HBTU, and a base, such as triethylamine, diisopropylethylamine, pyridine, potassium carbonate and cesium carbonate, in a solvent, such as acetonitrile, tetrahydrofuran, N,N-dimethylformamide and dimethyl sulfoxide, at a temperature of 0 to 100°C to produce the product (**I-c1** or **I-c2, I-d**).

Scheme 2:

**Key1** is prepared into an acid chloride in the presence of thionyl chloride, oxalyl chloride, or phosphorus trichloride, the acid chloride is then reacted with the reactants (**SM-2e** or **SM-2f, SM-3**) in the presence of a base, such as triethylamine, diisopropylethylamine, pyridine, potassium carbonate and cesium carbonate, in a solvent, such as dichloromethane, ethyl acetate, acetonitrile, tetrahydrofuran, N,N-dimethylformamide and dimethyl sulfoxide, to produce the product (**I-c1** or **I-c2, I-d**).

In some embodiments, the preparation routes of III-a1, III-a2, III-a3, and III-a4 are as follows:

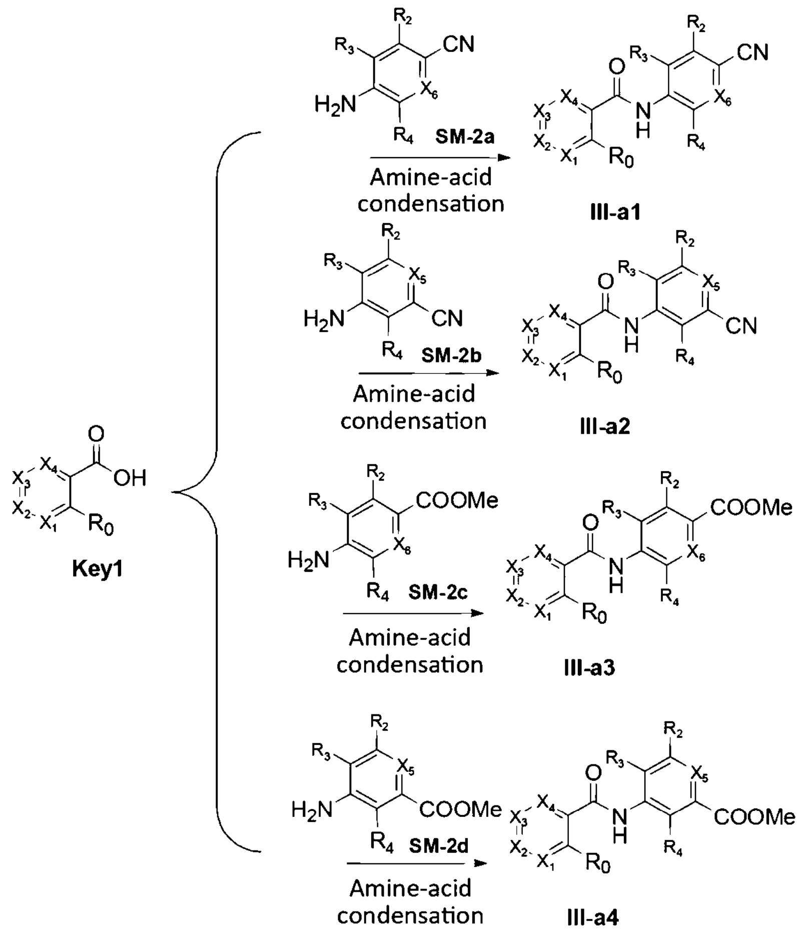


**Key1** is reacted with **SM-2a, SM-2b, SM-2c,** or **SM-2d** via amine-acid condensation to prepare **III-a1, III-a2, III-a3,** or **III-a4,**
wherein, each of the substituents is defined as above.

The amine-acid condensation is described in the schemes above.

In some embodiments, the intermediate **Key1** is prepared as follows:

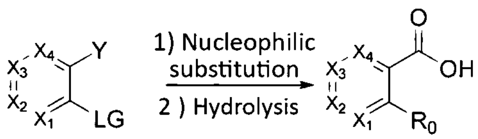


SM1

Key1

LG is a leaving group
Y is an ester group or a cyano group

.

Specifically, in the presence of a base such as potassium carbonate or cesium carbonate and in an appropriate solvent such as acetonitrile, tetrahydrofuran, N,N-dimethylformamide, dimethyl sulfoxide, or the like, **SM1** (wherein **LG** is a nucleophilic leaving group such as a halogen or $S(O)_2Me$, and Y is an ester group or a cyano group) and

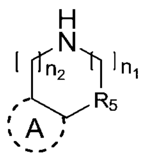 or 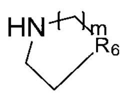 ,

or salts thereof are heated to undergo nucleophilic substitution, followed by hydrolysis to obtain **Key1.** The reaction is typically carried out at a temperature ranging from 50°C to 140°C,
wherein, the other substituents are defined as above.

In another aspect, the present invention provides a pharmaceutical composition comprising one or more selected from the compounds of Formula I, the tautomers, stereoisomers, deuterated substance, and pharmaceutically acceptable salts thereof, and optionally, a pharmaceutically acceptable excipient.

The pharmaceutical composition of the present invention may contain at least one pharmaceutically acceptable carrier selected from the group consisting of a diluent, an adjuvant, an excipient, a preservative, a filler, a binder, a disintegrant, a wetting agent, an emulsifier, a suspending agent, a sweetener, a flavoring agent, a fragrance, an antibacterial agent, an antifungal agent, a lubricant, a dispersant, a temperature-sensitive material, an adhesive, a stabilizer, and a suspending agent, depending on the administration manner and the nature of the dosage form.

In another aspect, the present invention provides the compound of Formula I, or the tautomer, stereoisomer, deuterated substance, or pharmaceutically acceptable salt thereof, or the above pharmaceutical composition, for use in preparation of a Nav1.8 inhibitor.

In another aspect, the present invention provides a method for inhibiting Nav1.8 channel activity in an individual, comprising administering to the individual one or more selected from the compounds of Formula I, the tautomers, stereoisomers, deuterated substances, and pharmaceutically acceptable salts thereof, or the above pharmaceutical composition.

In another aspect, the present invention provides the compound of Formula I, or the tautomer, stereoisomer, deuterated substance, or pharmaceutically acceptable salt thereof, or the above pharmaceutical composition, for use in preparation of a medicament for preventing and/or treating diseases associated with abnormal expression of Nav1.8 channel.

In another aspect, the present invention provides a method for preventing and/or treating diseases associated with abnormal expression of Nav1.8 channel, comprising administering to a subject in need thereof one or more selected from the compounds of Formula I, the tautomers, stereoisomers, deuterated substances, and pharmaceutically acceptable salts thereof, or the above pharmaceutical composition. The diseases associated with abnormal expression of Nav1.8 channel include pains, etc.; wherein the pains include but are not limited to nociceptive pain, inflammatory pain (including but not limited to rheumatoid arthritis pain or vulvar pain), neuropathic pain (including but not limited to post-herpetic neuralgia, idiopathic small fiber neuralgia), musculoskeletal pain (including but not limited to osteoarthritis pain, back pain, cold pain, burn pain or toothache), postoperative pain (including but not limited to bunionectomy pain, abdominoplasty pain, etc.), internal pain, functional pain, pain related to muscle or bone injury, pelvic pain, abdominal pain, chest pain, lumbar neuralgia, preoperative pain, intraoperative pain, postoperative pain, intestinal pain (including but not limited to inflammatory bowel disease pain, Crohn's disease pain or interstitial cystitis), acute or chronic pain, migraine, trigeminal neuralgia, pancreatitis, renal colic, cancer pain, pain caused by chemotherapy or drug therapy, diabetic neuralgia, post-herpetic neuralgia, back pain, phantom limb pain, sciatica, small fiber neuralgia, erythromelalgia, etc. Diseases associated with abnormal expression of Nav1.8 channel also include pruritus, acute or chronic pruritus, asthma, multiple sclerosis, arrhythmias, atrial fibrillation, heart failure, Brugada syndrome, kidney stones, epilepsy, convulsions, Charcot-Marie-Tooth syndrome, and incontinence, etc.

The present invention has the following beneficial effects: the compounds of the present invention exhibit excellent selective Nav1.8 inhibitory activity, excellent pharmacokinetic properties, and good drugability. As Nav1.8 inhibitors, they can be used to prevent and/or treat diseases associated with abnormal expression of Nav1.8 channel and thus have important clinical applications.

Although the present invention has been described in detail above, the aforementioned embodiments are merely illustrative and are not intended to limit the present invention. Furthermore, the present invention is not intended to be bound by any theory presented in the aforementioned prior art, summary of the invention, and the following examples.

## DETAILED EMBODIMENTS

The present invention will be further illustrated below with reference to the following examples. It should be noted that the following examples are provided for illustrative purposes only and are not intended to limit the scope of the present invention.

**Reagents and materials:**

Unless otherwise specified, all reagents were commercially available. The silica gel for column chromatography purification was 200-300 mesh column chromatography silica gel produced by Qingdao Ocean Chemical Plant. The chromatography silica gel plates for purification (model HSGF-254, thickness 0.15-0.2 mm) were produced by Yantai Chemical Experimental Plant.

**Analytic Instruments:**

$^1$H-NMR spectra were recorded on Bruker Avance III 600, Bruker Avance III 500, Bruker AM-400, or GEMINI-300 nuclear magnetic resonance spectrometer. Chemical shifts were expressed in δ (ppm), and proton coupling was labeled as singlet (s), doublet (d), triplet (t), quartet (q), multiplet (m), and broad (br).

Mass spectra were recorded on a Finnigan/MAT-95 mass spectrometer or an Agilent 1200-6110 single quadrupole liquid chromatograph-mass spectrometer.

**Example 1:** Preparation of 5-chloro-2-(4,4-difluoroazepan-1-yl)-N-(4-fluoro-3-(N'-hydroxyamidino)phenyl)-6-met hylnicotinamide (**I-1**)

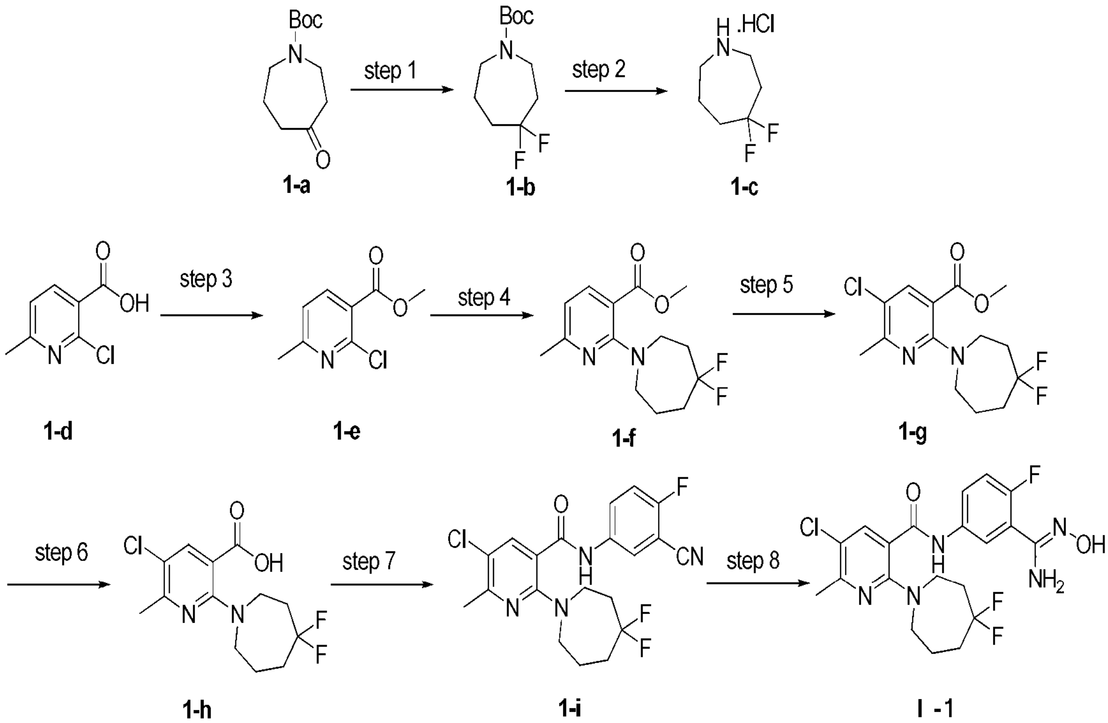


1-a 1-b 1-c 1-d 1-e 1-f 1-g 1-h 1-i I -1

Step 1: Tert-butyl 4-oxoazepane-1-carboxylate (**1-a**) (10.8 g, 50.6 mmol) was dissolved in dichloromethane (60 mL) and cooled to 0°C, and diethylaminosulfur trifluoride (14.7 mL, 111.4 mmol) diluted in dichloromethane (20 mL) was added dropwise thereto under inert gas. The mixture was allowed to react at room temperature for 48 hours. After **1-a** was reacted completely, the reaction mixture was poured into ice water, adjusted to pH 8-9 by adding saturated sodium bicarbonate solution, and extracted three times with dichloromethane. The combined organic layer was washed once with saturated brine, dried over anhydrous sodium sulfate, and concentrated in vacuum to remove the organic solvent, and obtain a crude product. The crude product was then purified by silica gel (200-300 mesh) column chromatography using PE:EA = 100:1-10:1 (v/v) to obtain 9.043 g of tert-butyl 4,4-difluoroazepane-1-carboxylate (**1-b**) as a reddish-brown liquid in a yield of 75.9%. $^1$H NMR (400MHz, $CDCl_3$) δ: 3.48~3.30(m, 4H), 2.16~1.97 (m, 4H), 1.83~1.74 (m, 2H), 1.44 (s, 9H).

Step 2: Tert-butyl 4,4-difluoroazepane-1-carboxylate (**1-b**) (9.043 g, 38.4 mmol) was added with a solution of hydrochloric acid in dioxane (4N, 60 mL) and reacted at room temperature until the starting material was reacted completely. The reaction mixture was directly evaporated to dryness by rotary evaporation to obtain 7.183 g of 4,4-difluoroazepane hydrochloride (**1-c**) as a white solid in a yield of 93.7%. $^1$H NMR (400 MHz, $CDCl_3$) δ: 9.88 (s, 2H), 3.39-3.29 (m, 4H), 2.61-2.47 (m, 2H), 2.36-2.21 (m, 2H), 2.08-1.98 (m, 2H); MS (ESI) m/z $[M+H]^+$= 136.2.

Step 3: 2-chloro-6-methylnicotinic acid (**1-d**) (3 g, 17.5 mmol) was dissolved in dichloromethane (20 mL), added with a catalytic amount of N,N-dimethylformamide and placed at 0°C. Oxalyl chloride (2.2 mL, 26.2 mmol) was slowly added dropwise thereto under nitrogen. The reaction was allowed to proceed at room temperature until the starting material was reacted completely. Methanol (7.1 mL, 175.0 mmol) was added and reacted for 1 hour. After the reaction was complete, the reaction system was concentrated, adjusted to pH 8-9 with saturated sodium bicarbonate solution, and extracted three times with dichloromethane. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using PE:EA = 20:1 (v/v) to obtain 2.472 g of methyl 2-chloro-6-methylnicotinate (**1-e**) as a colorless liquid in a yield of 71.4%. $^1$H NMR (400 MHz, $CDCl_3$) δ: 8.09 (d, *J* = 7.8 Hz, 1H), 7.20-7.14 (m, 1H), 3.94 (s, 3H), 2.59 (s, 3H); MS (ESI) m/z $[M+H]^+$ = 186.0.

Step 4: Methyl 2-chloro-6-methylnicotinate (**1-e**) (3.7 g, 19.9 mmol), 4,4-difluoroazepane hydrochloride (**1-c**) (4.1 g, 23.9 mmol), and potassium carbonate (8.253 g, 59.8 mmol) were added to N,N-dimethylformamide (100 mL) and reacted overnight at 90°C. After the reaction was complete, the reaction solution was adjusted to about pH 5-6 with 1N HCl. and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using PE:EA=30:1 (v/v) to obtain 4.52 g of methyl 2-(4,4-difluoroazepan-1-yl)-6-methylnicotinate (**1-f**) as a colorless liquid in a yield of 84.3%. $^1$H NMR (400MHz, $CDCl_3$) δ: 7.81(d, *J* = 7.8Hz, 1H), 6.52(d, *J* = 7.7 Hz, 1H), 3.85(s, 3H), 3.76~3.69(m, 2H), 3.30(t, *J* = 5.6Hz, 2H), 2.44~2.34(m, 5H), 2.07~1.91(m, 4H); MS(ESI) m/z $[M+H]^+$ =285.2.

Step 5: Methyl 2-(4,4-difluoroazepan-1-yl)-6-methylnicotinate (**1-f**) (200 mg, 0.703 mmol) and N-chlorosuccinimide (187.9 mg, 1.41 mmol) were dissolved in N,N-dimethylformamide (4 mL) and reacted at room temperature. After the reaction was complete, the reaction solution was added with water (20 mL) and extracted three times with ethyl acetate. The combined organic layer was washed once with saturated brine, dried over anhydrous sodium sulfate, and concentrated in vacuum to remove the organic solvent to obtain a crude product. The crude product was then purified by silica gel (200-300 mesh) column chromatography using PE:EA = 20:1 (v/v) to obtain 127 mg of methyl 5-chloro-2-(4,4-difluoroazepan-1-yl)-6-methylnicotinate (**1-g**) as a yellowish liquid in a yield of 56.6%. $^1$H NMR (400MHz, $CDCl_3$) δ: 7.84(s, 1H), 3.86 (s, 3H), 3.74~3.67(m, 2H), 3.26(t, *J* = 5.6Hz, 2H), 2.48(s, 3H), 2.44~2.29(m, 2H), 1.98~1.91(m, 4H); MS(ESI) m/z $[M+H]^+$ =319.9.

Step 6: Solid sodium hydroxide (4.34 g, 108.6 mmol) was dissolved in water (15 mL), and then the sodium hydroxide solution was added dropwise to a solution of methyl 5-chloro-2-(4,4-difluoroazepan-1-yl)-6-methylnicotinate (**1-g**) (3.462 g, 10.86 mmol) in methanol (15 mL) and reacted under reflux. After the reaction was complete, the reaction solution was concentrated to remove excess methanol, and adjusted to pH 5-6 with 2N HCl to precipitate a solid, which was filtered to obtain 3.25 g of 5-chloro-2-(4,4-difluoroazepan-1-yl)-6-methylnicotinic acid (**1-h**) as a yellowish solid in a yield of 98.2%. $^1$H NMR (400 MHz, $CDCl_3$) δ: 8.33 (s, 1H), 3.50-3.43 (m, 2H), 3.33 (t, *J* = 5.6 Hz, 2H), 2.62 (s, 3H), 2.52-2.37 (m, 2H), 2.30-2.17 (m, 2H), 2.07-2.00 (m, 2H); MS (ESI) m/z $[M+H]^+$ = 305.9.

Step 7: 5-chloro-2-(4,4-difluoroazepan-1-yl)-6-methylnicotinic acid (**1-h**) (600 mg, 2.0 mmol) was dissolved in thionyl chloride (10 mL), refluxed at 80°C until the starting material completely converted to an acid chloride, and removed the excess thionyl chloride by evaporation to obtain a residue. The residue was dissolved in dichloromethane (6 mL), and 5-amino-2-fluorobenzonitrile (268 mg, 2.0 mmol) and triethylamine (547 μL, 3.9 mmol) were added and the resultant was allowed to react at room temperature. After the reaction was complete, the dichloromethane was evaporated, and the resultant was added with water (40 mL), extracted three times with ethyl acetate. The combined organic layer was washed once with saturated brine, dried over anhydrous sodium sulfate, and concentrated in vacuum to remove the organic solvent to obtain a crude product. The crude product was then purified by silica gel (200-300 mesh) column chromatography using PE:EA = 10-1:1 (v/v) to obtain 730 mg of 5-chloro-N-(3-cyano-4-fluorophenyl)-2-(4,4-difluoroazepan-1-yl)-6-methylnicotinami de (**I-i**) as a yellow solid in a yield of 87.7%. $^1$H NMR (400MHz, $CDCl_3$) δ:10.24 (s, 1H), 8.16(s, 1H), 8.13~8.10(m, 1H), 7.79~7.72(m, 1H), 7.23(t, *J* = 8.6 Hz, 1H), 3.61~3.54(m, 2H), 3.37(t, *J* = 5.6 Hz, 2H), 2.57(s, 3H), 2.47~2.36(m, 2H), 2.25~2.12(m, 2H), 1.98~1.89(m, 2H); MS(ESI) m/z $[M+H]^+$= 424.1.

Step 8: 5-chloro-N-(3-cyano-4-fluorophenyl)-2-(4,4-difluoroazepan-1-yl)-6-methylnicotinami de (**1-i**) (200 mg, 0.47 mmol) and hydroxylamine hydrochloride (66 mg, 0.95 mmol) were dissolved in anhydrous ethanol (5 mL), added with triethylamine (197 μL, 1.42 mmol), and reacted at 80°C overnight. After the reaction was complete, the mixture was concentrated to obtain a crude product. The crude product was then purified by silica gel (200-300 mesh) column chromatography using DCM:MeOH=40:1(v/v) to obtain 138 mg of 5-chloro-2-(4,4-difluoroazepan-1-yl)-N-(4-fluoro-3-(N'-hydroxyamidino)phenyl)-6-met hylnicotinamide (**I-1**) as a white solid in a yield of 63.9%. $^1$H NMR (600 MHz, DMSO-$d_6$) δ: 10.47(s, 1H), 9.61(s, 1H), 7.80(dd, *J* = 6.4, 2.8 Hz, 1H), 7.70~7.64(m, 2H), 7.19(t, *J* = 9.6 Hz, 1H), 5.77(s, 2H), 3.60~3.55(m, 2H), 3.38(t, *J* = 6.0 Hz, 2H), 2.41(s, 3H), 2.32~2.22(m, 2H), 1.97~1.88(m, 2H), 1.82~1.78(m, 2H); MS(ESI) m/z $[M+H]^+$ = 457.2.

**Example 2:** Preparation of N-(3-amidino-4-fluorophenyl)-5-chloro-2-(4,4-difluoroazepan-1-yl)-6-methylnicotina mide (**I-2**)

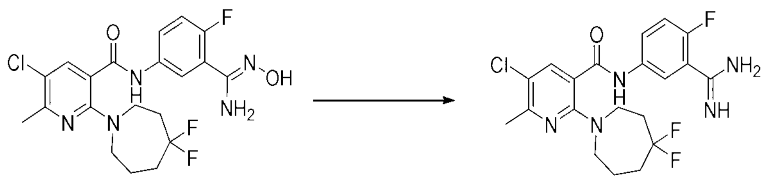

I-1

I-2

5-Chloro-2-(4,4-difluoroazepan-1-yl)-N-(4-fluoro-3-(N'-hydroxyamidino)phenyl)-6-me thylnicotinamide (**I-1**) (310 mg, 0.68 mmol) obtained in Example 1 was dissolved in acetic acid (3 mL), added with acetic anhydride (70 μL, 1.35 mmol) and reacted at room temperature for half an hour. Triethylsilane (1085 μL, 6.8 mmol) and palladium dichloride (120 mg, 0.68 mmol) were then added and reacted at 70°C. After the reaction was complete, the reaction mixture was adjusted to be alkaline with 2N sodium hydroxide solution, added with water (20 mL) and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using DCM:MeOH = 40-10:1 (v/v) to obtain 30 mg of N-(3-amidino-4-fluorophenyl)-5-chloro-2-(4,4-difluoroazepan-1-yl)-6-methylnicotina mide (**I-2**) as a white solid in a yield of 10.1%. $^1$H NMR (800 MHz, Methanol-$d_4$) δ: 8.18(dd, $J$ = 6.0, 2.8Hz, 1H), 7.87~7.83(m, 1H), 7.71(s, 1H), 7.39(t, $J$ = 9.4Hz, 1H), 3.72~3.68(m, 2H), 3.46(t, $J$ = 6.0 Hz, 2H), 2.49(s, 3H), 2.36~2.30(m, 2H), 2.05~1.94(m, 2H), 1.94~1.89(m, 2H); MS(ESI) m/z $[M+H]^+$ = 440.2.

**Example 3:** Preparation of 5-chloro-N-(3-(N'-cyanoamidino)-4-fluorophenyl)-2-(4,4-difluoroazepan-1-yl)-6-meth ylnicotinamide (**I-3**)

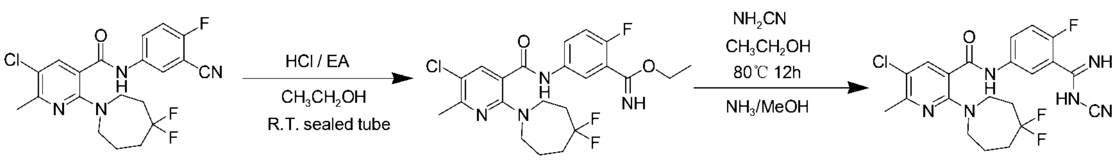


1-i

3-a

I-3

5-chloro-N-(3-cyano-4-fluorophenyl)-2-(4,4-difluoroazepan-1-yl)-6-methylnicotinami de (**1-i**) (150 mg, 0.36 mmol) obtained in Example 1, anhydrous ethanol (42 μL, 0.71 mmol), and dry HCl/ethyl acetate (5 mL) were added to a sealed tube and reacted at room temperature to obtain a Pinner salt intermediate (**3-a**). After the reaction was complete, the reaction system was evaporated to dryness, added with anhydrous ethanol (3 mL), cyanamide (180 mg, 4.26 mmol), and triethylamine (99 μL, 0.71 mmol) and refluxed at 80°C until the intermediate was fully converted. An ammonia methanol solution (3 mL) was then added to the reaction system and the reaction continued until the reaction was complete. After the reaction was complete, the organic solvent was removed by vacuum concentration. The resultant was added with water (30 mL) and extracted three times with ethyl acetate. The combined organic layer was washed once with saturated brine, dried over anhydrous sodium sulfate, and concentrated in vacuum to remove the organic solvent to obtain a crude product. The crude product was then purified by silica gel (200-300 mesh) column chromatography using PE:EA = 4-2:1 (v/v) to obtain 32 mg of 5-chloro-N-(3-(N'-cyanoamidino)-4-fluorophenyl)-2-(4,4-difluoroazepan-1-yl)-6-meth ylnicotinamide (**I-3**) as a white solid in a yield of 19.4%. $^1$H NMR (600MHz, DMSO-$d_6$) δ: 10.65(s, 1H), 9.10~8.76(m, 2H), 8.08~7.64(m, 3H), 7.39(s, 1H), 3.62~3.59(m, 2H), 3.40(t, $J$ = 6.0 Hz, 2H), 2.45 (s, 3H), 2.37~2.26(m, 2H), 2.02~1.94(m, 2H), 1.87~1.81(m, 2H); MS(ESI) m/z $[M+H]^+$ =466.1.

**Example 4:** Preparation of 5-chloro-2-(4,4-difluoroazepan-1-yl)-N-(4-fluoro-3-(N'-methoxyamidino)phenyl)-6-me thylnicotinamide (**I-4**)

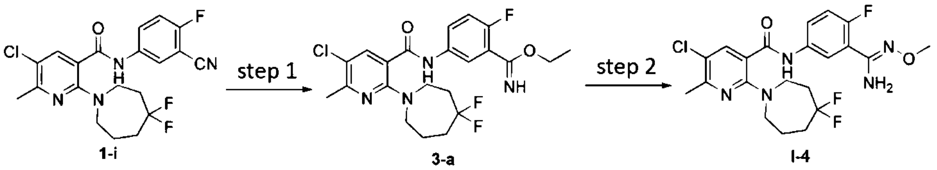


1-i
3-a
I-4

Step 1: 5-chloro-N-(3-cyano-4-fluorophenyl)-2-(4,4-difluoroazepan-1-yl)-6-methylnicotinami de (**1-i**) (150 mg, 0.36 mmol) obtained in Example 1, anhydrous ethanol (42 μL, 0.71 mmol), and dry HCl/ethyl acetate (5 mL) were added to a sealed tube and reacted at room temperature to obtain a Pinner salt intermediate (**3-a**).

Step 2: After the reaction was complete, the reaction system was evaporated to dryness, added with anhydrous ethanol (3 mL), methoxyamine hydrochloride (296 mg, 3.55 mmol), and triethylamine (49 μL, 3.55 mmol) and refluxed at 80°C. After the reaction was complete, the organic solvent was removed by vacuum concentration to obtain a crude product. The crude product was then purified by silica gel (200-300 mesh) column chromatography using PE:EA= 10-4:1 (v/v) to obtain 43 mg of 5-chloro-2-(4,4-difluoroazepan-1-yl)-N-(4-fluoro-3-(N'-methoxyamidino)phenyl)-6-me thylnicotinamide (**I-4**) as a white solid in a yield of 25.7%. $^1$H NMR (600 MHz, $CDCl_3$) δ: 9.62(s, 1H), 7.91(s, 1H), 7.87~7.82(m, 1H), 7.76(dd, $J$ = 6.4, 2.8 Hz, 1H), 7.08(dd, $J$ = 10.8, 9.0 Hz, 1H), 5.09(s, 2H), 3.86(s, 3H), 3.57~3.52(m, 2H), 3.35(t, $J$ = 6.0 Hz, 2H), 2.50(s, 3H), 2.40~2.31(m, 2H), 2.12~2.04(m, 2H), 1.91~1.86(m, 2H); MS(ESI) m/z $[M+H]^+$ = 470.8.

**Example 5:** Preparation of 5-chloro-2-(4,4-difluoroazepan-1-yl)-N-(4-chloro-3-(N'-hydroxyamidino)phenyl)-6-me thylnicotinamide (**I-5**)

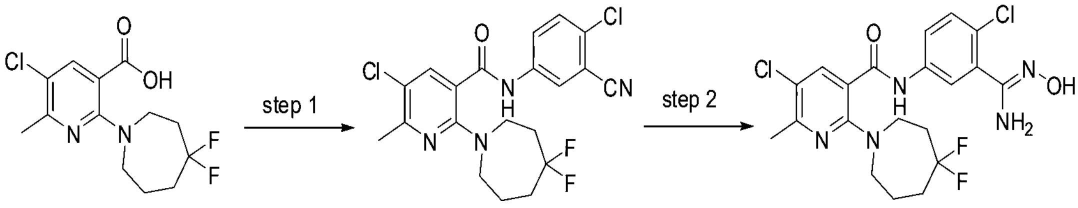


1-h
5-a
I -5

Step 1: Following the process of Step 7 of Example 1, 5-chloro-2-(4,4-difluoroazepan-1-yl)-6-methylnicotinic acid (**1-h**) (200 mg, 0.66 mmol) was reacted with 5-amino-2-chlorobenzonitrile (100 mg, 0.66 mmol) to obtain 115 mg of 5-chloro-N-(3-cyano-4-chlorophenyl)-2-(4,4-difluoroazepan-1-yl)-6-methylnicotinami de (**5-a**) as a yellow solid in a yield of 39.9%. $^1$H NMR (400 MHz, $CDCl_3$) δ: 10.25(s, 1H), 8.16(d, $J$ = 13.4 Hz, 2H), 7.75~7.69(m, 1H), 7.50(d, $J$ = 7.8 Hz, 1H), 3.63~3.48 (m, 2H), 3.46~3.31(m, 2H), 2.57(s, 3H), 2.49~2.36(m, 2H), 2.26~2.13(m, 2H), 1.97~1.89(m, 2H); MS(ESI) m/z $[M+H]^+$ = 440.0.

Step 2: Following the process of Step 8 of Example 1, 5-chloro-N-(3-cyano-4-chlorophenyl)-2-(4,4-difluoroazepan-1-yl)-6-methylnicotinami de (**5-a**) was used as a starting material to obtain 80 mg of 5-chloro-2-(4,4-difluoroazepan-1-yl)-N-(4-chloro-3-(N'-hydroxyamidino)phenyl)-6-me thylnicotinamide (**I-5**) as a white solid in a yield of 67.6%. $^1$H NMR (500 MHz, DMSO-$d_6$) δ: 10.59(s, 1H), 9.49(s, 1H), 7.76(d, $J$ = 2.6 Hz, 1H), 7.75~7.72(m, 2H), 7.44(d, J = 8.6 Hz, 1H), 5.85(s, 2H), 3.62~3.59(m, 2H), 3.42~3.39(m, 2H), 2.44(s, 3H), 2.35~2.26(m, 2H), 2.01~1.92(m, 2H), 1.86~1.81(m, 2H); MS(ESI) m/z $[M+H]^+$ = 472.9.

**Example 6:** Preparation of N-(3-amidino-4-chlorophenyl)-5-chloro-2-(4,4-difluoroazepan-1-yl)-6-methylnicotina mide (**I-6**)

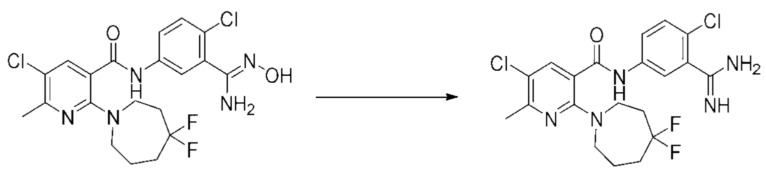

I-5
I-6

Following the process of Example 2, 5-chloro-2-(4,4-difluoroazepan-1-yl)-N-(4-chloro-3-(N'-hydroxyamidino) phenyl)-6-me thylnicotinamide (**I-5**) (430 mg, 0.91 mmol) was used as a starting material to obtain 40 mg of N-(3-amidino-4-chlorophenyl)-5-chloro-2-(4,4-difluoroazepan-1-yl)-6-methylnicotina mide (**I-6**) as a white solid in a yield of 9.6%. $^1$H NMR (500 MHz, Methanol-$d_4$) δ: 8.15(d, *J* = 2.6 Hz, 1H), 7.78(dd, *J* = 8.8, 2.6 Hz, 1H), 7.70(s, 1H), 7.61(d, *J* = 8.8 Hz, 1H), 3.72~3.66(m, 2H), 3.45(t, *J* = 6.0 Hz, 2H), 2.49(s, 3H), 2.38~2.29(m, 2H), 2.04~1.95(m, 2H), 1.93~1.88(m, 2H); MS(ESI) m/z[M+H]$^+$=457.2.

**Example 7:** Preparation of 5-chloro-2-(4,4-difluoroazepan-1-yl)-N-(3-(N'-hydroxyamidino)-4-methylphenyl)-6-me thylnicotinamide (**I-7**)

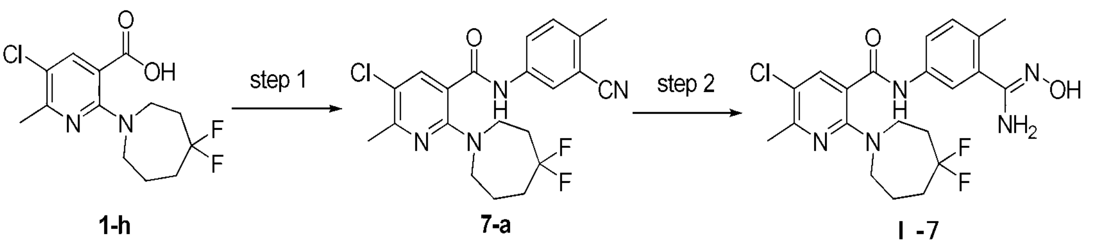


1-h 7-a I -7

Step 1: Following the process of Step 7 of Example 1, 5-chloro-2-(4,4-difluoroazepan-1-yl)-6-methylnicotinamide (**1-h**) (300 mg, 0.98 mmol) was reacted with 5-amino-2-methylbenzonitrile (130 mg, 0.98 mmol) to obtain 300 mg of 5-chloro-N-(3-cyano-4-methylphenyl)-2-(4,4-difluoroazepan-1-yl)-6-methylnicotinami de (**7-a**) as a white solid in a yield of 72.7%. $^1$H NMR (400 MHz, $CDCl_3$) δ: 9.90(s, 1H), 8.09 (s, 1H), 8.03(d, *J* = 2.4 Hz, 1H), 7.64(dd, *J* = 8.4, 2.4 Hz, 1H), 7.31(d, *J* = 8.4 Hz, 1H), 3.61~3.54(m, 2H), 3.41~3.34(m, 2H), 2.55(s, 3H), 2.53(s, 3H), 2.46~2.34(m, 2H), 2.23~2.11(m, 2H), 1.97~1.88(m, 2H); MS(ESI) m/z [M+H]$^+$= 420.0.

Step 2: Following the process of Step 8 of Example 1, 5-chloro-N-(3-cyano-4-methylphenyl)-2-(4,4-difluoroazepan-1-yl)-6-methylnicotinami de (**7-a**) (300 mg, 0.72 mmol) was used as a starting material to obtain 100 mg of 5-chloro-2-(4,4-difluoroazepan-1-yl)-N-(3-(N'-hydroxyamidino)-4-methylphenyl)-6-me thylnicotinamide (**I-7**) as a white solid in a yield of 30.9%. $^1$H NMR (500 MHz, DMSO-$d_6$) δ: 10.38(s, 1H), 9.31(s, 1H), 7.68(s, 1H), 7.60(d, *J* = 9.8 Hz, 2H), 7.17(d, *J* = 8.0 Hz, 1H), 5.71(s, 2H), 3.63~3.59(m, 2H), 3.43(t, *J* = 6.0 Hz, 2H), 2.44(s, 3H), 2.30~2.29(m, 5H), 2.02~1.93(m, 2H), 1.86~1.79(m, 2H); MS(ESI) m/z [M+H]$^+$= 453.2. **Example 8:** Preparation of *N*-(3-amidino-4-methylphenyl)-5-chloro-2-(4,4-difluoroazepan-1-yl)-6-methylnicotina mide (**I-8**)

Following the process of Example 2, 5-chloro-2-(4,4-difluoroazepan-1-yl)-N-(3-(N'-hydroxyamidino)-4-methylphenyl)-6-me thylnicotinamide (**I-7**) (325 mg, 0.72 mmol) was used as a starting material to obtain 22 mg of *N*-(3-amidino-4-methylphenyl)-5-chloro-2-(4,4-difluoroazepan-1-yl)-6-methylnicotina mide (**I-8**) as a white solid in a yield of 7.05%. $^1$H NMR (500 MHz, Methanol-$d_4$) δ: 7.99(d, *J* = 2.4 Hz, 1H), 7.68(s, 1H), 7.66 (dd, *J* = 8.4, 2.4 Hz, 1H), 7.40(d, *J* = 8.4 Hz, 1H), 3.72~3.66(m, 2H), 3.46(t, *J* = 6.0 Hz, 2H), 2.49(s, 3H), 2.44(s, 3H), 2.38~2.28(m, 2H), 2.05~1.94(m, 2H), 1.93~1.87(m, 2H); MS(ESI) m/z [M+H]$^+$ = 436.9.

**Example 9:** Preparation of 5-chloro-2-(4,4-difluoroazepan-1-yl)-N-(3-(N'-hydroxyamidino)-4-methoxyphenyl)-6-methylnicotinamide (**I-9**)

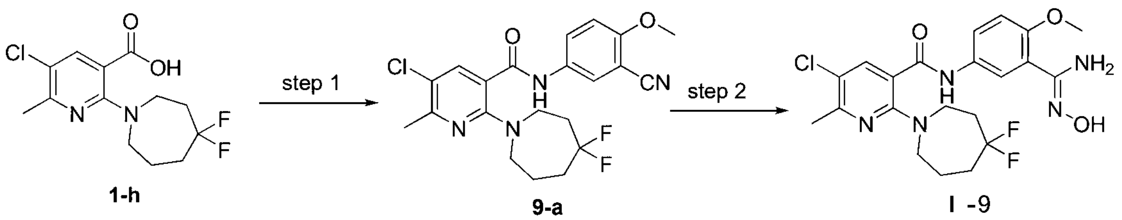


1-h 9-a I -9

Step 1: Following the process of Step 7 of Example 1, 5-chloro-2-(4,4-difluoroazepan-1-yl)-6-methylnicotinic acid (**1-h**) (300 mg, 0.98 mmol) was reacted with 5-amino-2-methoxybenzonitrile (146 mg, 0.98 mmol) to obtain 309 mg of 5-chloro-N-(3-cyano-4-methoxyphenyl)-2-(4,4-difluoroazepan-1-yl)-6-methylnicotina mide (**9-a**) as a yellow solid in a yield of 70.1%. $^1$H NMR (400 MHz, $CDCl_3$) δ: 9.84(s, 1H), 8.07(s, 1H), 7.92(d, *J* = 2.8Hz, 1H), 7.76(dd, *J* = 9.0, 2.8 Hz, 1H),

6.99(d, $J$ = 9.0 Hz, 1H), 3.94(s, 3H), 3.61~3.54(m, 2H), 3.38(t, $J$ = 5.8 Hz, 2H), 2.55(s, 3H), 2.47~2.32(m, 2H), 2.23~2.09(m, 2H), 1.97~1.88(m, 2H); MS(ESI) m/z $[M+H]^+$ =435.9.

Step 2: Following the process of Step 8 of Example 1, 5-chloro-N-(3-cyano-4-methylphenyl)-2-(4,4-difluoroazepan-1-yl)-6-methoxynicotina mide (**9-a**) (200 mg, 0.46 mmol) was used as a starting material to obtain 50 mg of 5-chloro-2-(4,4-difluoroazepan-1-yl)-N-(3-(N'-hydroxyamidino)-4-methoxyphenyl)-6-methylnicotinamide (**I-9**) as a white solid in a yield of 23.3%. $^1H$ NMR (500 MHz, DMSO-$d_6$) δ: 10.31(s, 1H), 9.41(s, 1H), 7.73 (dd, $J$ = 2.7, 1.0Hz, 1H), 7.67(d, $J$ = 9.4 Hz, 2H), 7.05(d, $J$ = 9.0 Hz, 1H), 5.61(s, 2H), 3.78 (s, 3H), 3.63~3.58(m, 2H), 3.42(t, $J$ = 6.0 Hz, 2H), 2.44(s, 3H), 2.34~2.25 (m, 2H), 2.02~1.92 (m, 2H), 1.87~1.80(m, 2H); MS(ESI) m/z $[M+H]^+$ = 468.8.

**Example 10**: Preparation of N-(3-amidino-4-methoxyphenyl)-5-chloro-2-(4,4-difluoroazepan-1-yl)-6-methylnicoti namide (**I-10**)

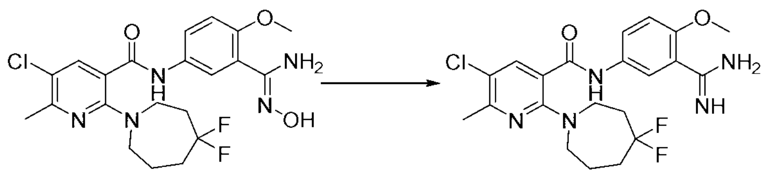

I-9 I-10

Following the process of Example 2, 5-chloro-2-(4,4-difluoroazepan-1-yl)-N-(3-(N'-hydroxyamidino)-4-methoxyphenyl)-6-methylnicotinamide (**I-9**) (240 mg, 0.53 mmol) was used as a starting material to obtain 14 mg of N-(3-amidino-4-methoxyphenyl)-5-chloro-2-(4,4-difluoroazepan-1-yl)-6-methylnicoti namide (**I-10**) as a white solid in a yield of 5.8%. $^1H$ NMR (500 MHz, Methanol-$d_4$) δ: 8.01(d, $J$ = 2.8 Hz, 1H), 7.77(dd, $J$ = 9.0, 2.8 Hz, 1H), 7.69(s, 1H), 7.25(d, $J$ = 9.0 Hz, 1H), 3.94(s, 3H), 3.72~3.66(m, 2H), 3.48(t, $J$ = 6.0 Hz, 2H), 2.49(s, 3H), 2.38~2.27(m, 2H), 2.05~1.95(m, 2H), 1.94~1.87(m, 2H); MS(ESI) m/z $[M+H]^+$ =453.2.

**Example 11**: Preparation of 5-chloro-2-(4,4-difluoroazepan-1-yl)-N-(3-(N'-hydroxyamidino)phenyl)-6-methylnicoti namide (**I-11**)

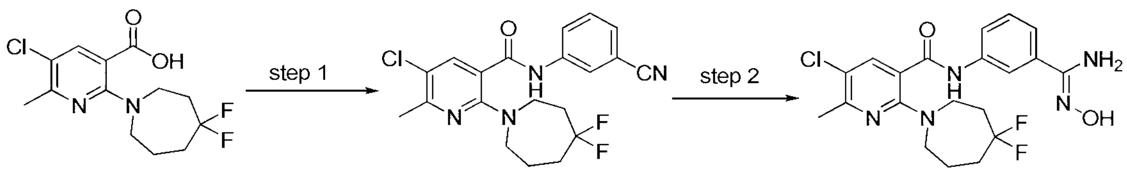


1-h 11-a I -11

Step 1: Following the process of Step 7 of Example 1, 5-chloro-2-(4,4-difluoroazepan-1-yl)-6-methylnicotinamide (**I-h**) (100 mg, 0.33 mmol) was reacted with m-aminobenzonitrile (39 mg, 0.33 mmol) to obtain 51 mg of 5-chloro-N-(3-cyanophenyl)-2-(4,4-difluoroazepan-1-yl)-6-methylnicotinamide (**11-a**) as a white solid in a yield of 38.3%. $^1H$ NMR (400 MHz, $CDCl_3$) δ: 10.05(s, 1H), 8.12(s, 2H), 7.75(d, $J$ = 8.0 Hz, 1H), 7.52~7.40(m, 2H), 3.61~3.54(m, 2H), 3.38(t, $J$ = 5.8 Hz, 2H), 2.56(s, 3H), 2.47~2.33(m, 2H), 2.25~2.10(m, 2H), 1.96~1.89 (m, 2H); MS(ESI) m/z $[M+H]^+$ = 406.0.

Step 2: Following the process of Step 8 of Example 1, 5-chloro-N-(3-cyanophenyl)-2-(4,4-difluoroazepan-1-yl)-6-methylnicotinamide (**11-a**) (100 mg, 0.25 mmol) was used as a starting material to obtain 50 mg of 5-chloro-2-(4,4-difluoroazepan-1-yl)-N-(3-(N'-hydroxyamidino)phenyl)-6-methylnicoti namide (**I-11**) as a white solid in a yield of 46.3%. $^1H$ NMR (600 MHz, DMSO-$d_6$) δ: 10.46(s, 1H), 9.65(s, 1H), 8.01(s, 1H), 7.71 (s, 1H), 7.68(d, $J$ = 7.8 Hz, 1H), 7.39~7.32(m, 2H), 5.77(s, 2H), 3.63~3.60(m, 2H), 3.43(t, $J$ = 6.0 Hz, 2H), 2.44(s, 3H), 2.34~2.27(m, 2H), 2.01~1.93(m, 2H), 1.86~1.82(m, 2H); MS(ESI) m/z $[M+H]^+$ = 439.0.

**Example 12**: Preparation of N-(3-amidinophenyl)-5-chloro-2-(4,4-difluoroazepan-1-yl)-6-methylnicotinamide (**I-12**)

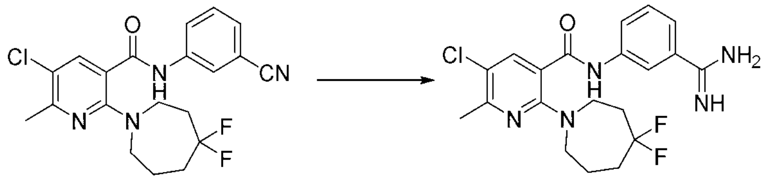

11-a I-12

5-Chloro-N-(3-cyanophenyl)-2-(4,4-difluoroazepan-1-yl)-6-methylnicotinamide (**11-a**) (100 mg, 0.247 mmol) prepared in Example 11, anhydrous methanol (3 mL), and dry HCl/methyl acetate (10N, 4 mL) were added to a sealed tube and reacted overnight at room temperature to obtain a Pinner salt. After the reaction was complete, the reaction system was evaporated to dryness, followed by the addition of anhydrous methanol (2 mL), an ammonia-methanol solution (3 mL), and ammonium chloride (26 mg, 0.49 mmol) and the reaction was continued under reflux. After the reaction was complete, the reaction mixture was concentrated, added with water (20 mL), adjusted to pH 8-9 with a 2N sodium hydroxide solution, and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using DCM:MeOH=20-10:1 (v/v) to obtain 49 mg of N-(3-amidinophenyl)-5-chloro-2-(4,4-difluoroazepan-1-yl)-6-methylnicotinamide (**I-12**) as a white solid in a yield of 47.1%. $^1H$ NMR (600 MHz, Methanol-$d_4$) $\delta$: 8.29 (t, *J* = 2.0 Hz, 1H), 7.85 (d, *J* = 8.0 Hz, 1H), 7.72 (s, 1H), 7.61 (t, *J* = 8.0 Hz, 1H), 7.55 (d, *J* = 7.8 Hz, 1H), 3.73~3.68 (m, 2H), 3.47 (t, *J* = 6.0 Hz, 2H), 2.50 (s, 3H), 2.39~2.29 (m, 2H), 2.03~1.95 (m, 2H), 1.93~1.90 (m, 2H); MS(ESI) m/z $[M+H]^+$ = 423.0.

**Example 13:** Preparation of 5-chloro-2-(4,4-difluoroazepan-1-yl)-N-(4-(N'-hydroxyamidino)phenyl)-6-methylnicoti namide (**I-13**)

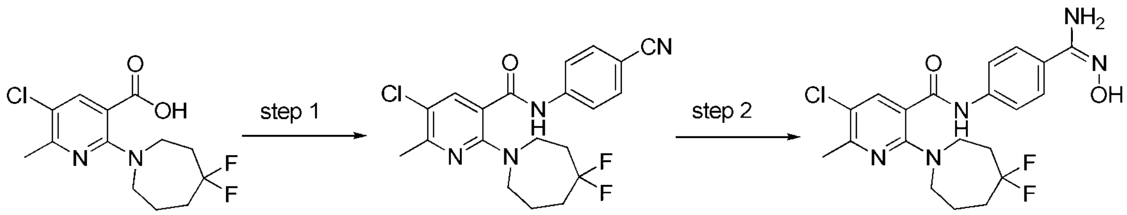


1-h 13-a I -13

Step 1: Following the process of Step 7 of Example 1, 5-chloro-2-(4,4-difluoroazepan-1-yl)-6-methylnicotinamide (**1-h**) obtained in Example 1 (200 mg, 0.66 mmol) was reacted with 4-aminobenzonitrile (78 mg, 0.66 mmol) to obtain 90 mg of 5-chloro-N-(4-cyanophenyl)-2-(4,4-difluoroazepan-1-yl)-6-methylnicotinamide (**13-a**) as a yellow solid in a yield of 33.8%. $^1H$ NMR (400 MHz, $CDCl_3$) $\delta$: 10.26 (s, 1H), 8.15 (s, 1H), 7.81~7.74(m, 2H), 7.70~7.63(m, 2H), 3.61~3.54(m, 2H), 3.40~3.33 (m, 2H), 2.57(s, 3H), 2.49~2.34(m, 2H), 2.26~2.11(m, 2H), 1.97~1.87(m, 2H); MS(ESI) m/z $[M+H]^+$ =406.0.

Step 2: Following the process of Step 8 of Example 1, 5-chloro-N-(4-cyanophenyl)-2-(4,4-difluoroazepan-1-yl)-6-methylnicotinamide (**13-a**) (140 mg, 0.35 mmol) was used as a starting material to obtain 28 mg of 5-chloro-2-(4,4-difluoroazepan-1-yl)-N-(4-(N'-hydroxyamidino)phenyl)-6-methylnicoti namide (**I-13**) as a white solid in a yield of 18.5%. $^1H$ NMR (600 MHz, DMSO-$d_6$) $\delta$: 10.54(s, 1H), 9.59(s, 1H), 7.73(s, 1H), 7.68 (d, *J* = 8.6 Hz, 2H), 7.64(d, *J* = 8.6 Hz, 2H), 5.82(s, 2H), 3.62~3.59(m, 2H), 3.42(t, *J* = 6.0 Hz, 2H), 2.44(s, 3H), 2.34~2.26(m, 2H), 2.01~1.94(m, 2H), 1.85~1.81(m, 2H); MS(ESI) m/z $[M+H]^+$ = 439.2.

**Example 14:** Preparation of N-(4-amidinophenyl)-5-chloro-2-(4,4-difluoroazepan-1-yl)-6-methylnicotinamide (**I-14**)

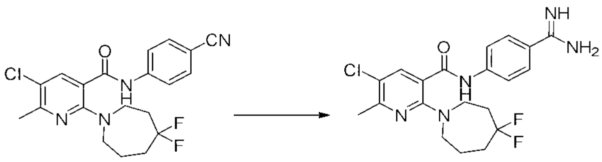

13-a I-14

Following the process of Example 12, 5-chloro-N-(4-cyanophenyl)-2-(4,4-difluoroazepan-1-yl)-6-methylnicotinamide (**13-a**) (150 mg, 0.37 mmol) obtained in Example 13 was used as a starting material to obtain 75 mg of N-(4-amidinophenyl)-5-chloro-2-(4,4-difluoroazepan-1-yl)-6-methylnicotinamide (**I-14**) as a white solid in a yield of 48.0%. $^1$H NMR (600 MHz, Methanol-$d_4$) δ: 7.94 (d, $J$ = 8.4 Hz, 2H), 7.83 (d, $J$ = 8.7 Hz, 2H), 7.72 (s, 1H), 3.71~3.67 (m, 2H), 3.44 (t, $J$ = 6.0 Hz, 2H), 2.49 (s, 3H), 2.37~2.29 (m, 2H), 2.02~1.95 (m, 2H), 1.92~1.88 (m, 2H); MS(ESI) m/z $[M+H]^+$ =422.9.

**Example 15**: Preparation of 5-chloro-2-(4,4-difluoroazepan-1-yl)-N-(2-(N'-hydroxyamidinoimino)pyridin-4-yl)-6-m ethylnicotinamide (**I-15**)

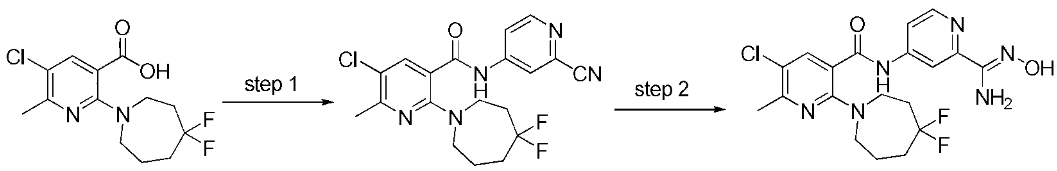


**1-h** **15-a** **I -15**

Step 1: Following the process of Step 7 of Example 1, 5-chloro-2-(4,4-difluoroazepan-1-yl)-6-methylnicotinic acid (**1-h**) (250 mg, 0.82 mmol) was reacted with 4-amino-2-cyanopyridine (98 mg, 0.82 mmol) to obtain 100 mg of 5-chloro-N-(2-cyanopyridine)-2-(4,4-difluoroazepan-1-yl)-6-Methylnicotinamide (**15-a**) as a yellow solid in a yield of 30.0%. $^1$H NMR (400 MHz, $CDCl_3$) δ: 10.72 (s, 1H), 8.62 (d, $J$ = 5.6 Hz, 1H), 8.22~8.15 (m, 2H), 7.66 (dd, $J$ = 5.6, 2.2 Hz, 1H), 3.60~3.53 (m, 2H), 3.38~3.31 (m, 2H), 2.59 (s, 3H), 2.51~2.36 (m, 2H), 2.28~2.16 (m, 2H), 1.99~1.90 (m, 2H); MS(ESI) m/z $[M+H]^+$ = 407.1.

Step 2: Following the process of Step 8 of Example 1, 5-chloro-N-(2-cyanopyridine)-2-(4,4-difluoroazepan-1-yl)-6-methylnicotinamide (**15-a**) (100 mg, 0.25 mmol) was used as a starting material to obtain 93 mg of 5-chloro-2-(4,4-difluoroazepan-1-yl)-N-(2-(N'-hydroxyamidino)pyridin-4-yl)-6-methyln icotinamide (**I-15**) as a white solid in a yield of 86.1%. $^1$H NMR (600 MHz, DMSO-$d_6$) δ: 10.81 (s, 1H), 9.89 (s, 1H), 8.45 (d, $J$ = 5.6 Hz, 1H), 8.26 (s, 1H), 7.80 (s, 1H), 7.65 (d, $J$ = 6.4 Hz, 1H), 5.81 (s, 2H), 3.62~3.59 (m, 2H), 3.38~3.36 (m, 2H), 2.45 (s, 3H), 2.33~2.27(m, 2H), 1.99~1.92 (m, 2H), 1.87~1.80 (m, 2H); MS(ESI) m/z $[M+H]^+$ = 440.2.

**Example 16:** Preparation of 5-chloro-2-(4,4-difluoroazepan-1-yl)-N-(4-fluoro-3-(N'-hydroxyamidino)phenyl)nicotin amide (**I-16**)

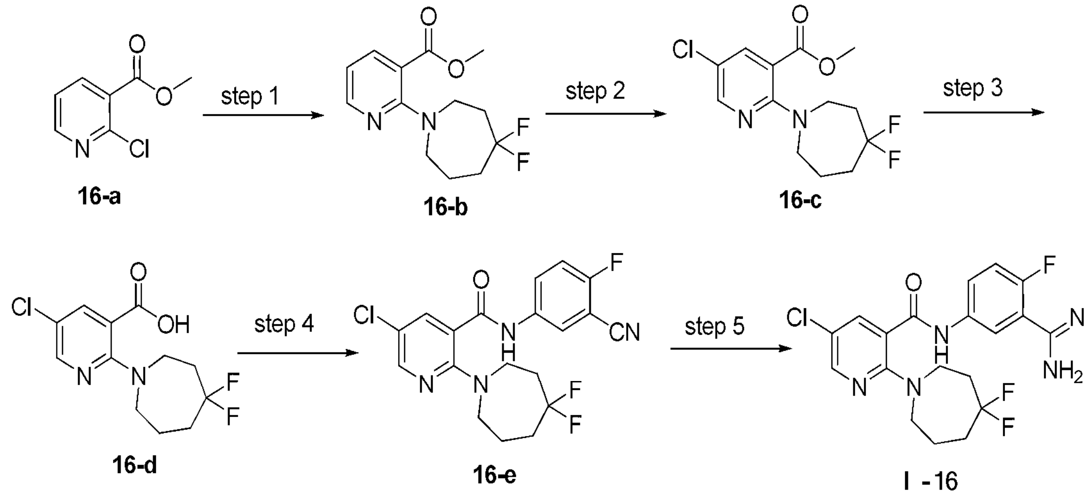


16-a 16-b 16-c

16-d 16-e I -16

Step 1: Following the process of Step 4 of Example 1, methyl 2-chloronicotinate (**16-a**) (1 g, 5.8 mmol) was reacted with 4,4-difluoroazepane hydrochloride (**1-c**) (1.2 g, 7.0 mmol) to obtain 879 mg of methyl 2-(4,4-difluoroazepan-1-yl)nicotinate (**16-b**) as a colorless liquid in a yield of 55.8%. $^1$H NMR (400 MHz, $CDCl_3$) δ: 8.23(dd, *J* = 4.6, 2.0 Hz, 1H), 7.89(dd, *J* = 7.6, 2.0 Hz, 1H), 6.66(dd, *J* = 7.6, 4.6 Hz, 1H), 3.88(s, 3H), 3.76~3.69(m, 2H), 3.30(t, *J* = 5.6 Hz, 2H), 2.45~2.31(m, 2H), 2.03~1.89(m, 4H); MS(ESI) m/z $[M+H]^+$ = 271.0.

Step 2: Following the process of Step 5 of Example 1, methyl 2-(4,4-difluoroazepan-1-yl)nicotinate (**16-b**) (658 mg, 2.4 mmol) was used as a starting material to obtain 523 mg of methyl 5-chloro-2-(4,4-difluoroazepan-1-yl)nicotinate (**16-c**) as a colorless liquid in a yield of 70.5%. $^1$H NMR (400 MHz, $CDCl_3$) δ: 8.16(d, *J* = 2.6 Hz, 1H), 7.86(d, *J* = 2.6 Hz, 1H), 3.88(s, 3H), 3.72~3.65(m, 2H), 3.25(t, *J* = 5.6 Hz, 2H), 2.42~2.27(m, 2H), 2.01~1.90(m, 4H); MS(ESI) m/z $[M+H]^+$ = 305.9.

Step 3: Following the process of Step 6 of Example 1, methyl 5-chloro-2-(4,4-difluoroazepan-1-yl)nicotinate (**16-c**) (460 mg, 1.5 mmol) was used as a starting material to obtain 385 mg of 5-chloro-2-(4,4-difluoroazepan-1-yl)nicotinic acid (**16-d**) as a white solid in a yield of 87.7%. $^1$H NMR (400 MHz, $CDCl_3$) δ: 8.41(d, *J* = 2.6 Hz, 1H), 8.34(d, *J* = 2.6 Hz, 1H), 3.53~3.49(m, 2H), 3.31(t, *J* = 6.0 Hz, 2H), 2.50~2.35(m, 2H), 2.26~2.11(m, 2H), 2.07~1.98(m, 2H); MS(ESI) m/z $[M+H]^+$ = 291.8. Step 4: Following the process of Step 7 of Example 1, 5-chloro-2-(4,4-difluoroazepan-1-yl)nicotinic acid (**16-d**) (300 mg, 1.0 mmol) and 5-amino-2-fluorobenzonitrile (140 mg, 1.0 mmol) were used as starting materials to obtain 352 mg of 5-chloro-N-(3-cyano-4-fluorophenyl)-2-(4,4-difluoroazePan-1-yl)nicotinamide (**16-e**) as a white solid in a yield of 83.4%. $^1$H NMR (400 MHz, DMSO-$d_6$) δ: 10.85(s, 1H), 8.26(d, *J* = 2.6 Hz, 1H), 8.18(dd, *J* = 5.8, 2.6 Hz, 1H), 7.99~7.90(m, 1H), 7.87(d, *J* = 2.6 Hz, 1H), 7.56(t, *J* = 9.1 Hz, 1H), 3.64~3.57(m, 2H), 3.39(t, *J* = 6.0 Hz, 2H), 2.35~2.25(m, 2H), 2.03~1.90(m, 2H), 1.90~1.79(m, 2H); MS(ESI) m/z $[M+H]^+$ = 409.9.

Step 5: Following the process of Step 8 of Example 1, 5-chloro-N-(3-cyano-4-fluorophenyl)-2-(4,4-difluoroazepan-1-yl)nicotinamide (**16-e**) (200 mg, 0.49 mmol) was used as a starting material to obtain 138 mg of 5-chloro-2-(4,4-difluoroazepan-1-yl)-N-(4-fluoro-3-(N'-hydroxyamidino)phenyl)nicotin amide (**I-16**) as a white solid in a yield of 63.9%. $^1$H NMR (500 MHz, DMSO-$d_6$) δ: 10.59(s, 1H), 9.65(s, 1H), 8.24(d, *J* = 2.6 Hz, 1H), 7.84(dd, *J* = 6.4, 2.8 Hz, 1H), 7.81(d, *J* = 2.6 Hz, 1H), 7.74~7.68(m, 1H), 7.23(t, *J* = 9.6 Hz, 1H), 5.83'''5.78(m, 2H), 3.64˜3.60(m, 2H), 3.43(t, *J* = 6.0 Hz, 2H), 2.34~2.23(m, 2H), 2.01~1.92(m, 2H), 1.87~1.83(m, 2H); MS(ESI) m/z $[M+H]^+$ = 442.9.

**Example 17:** Preparation of N-(3-(amidinocarbamoyl)-4-fluorophenyl)-5-chloro-2-(4,4-difluoroazepan-1-yl)-6-met hylnicotinamide (**I-17**)

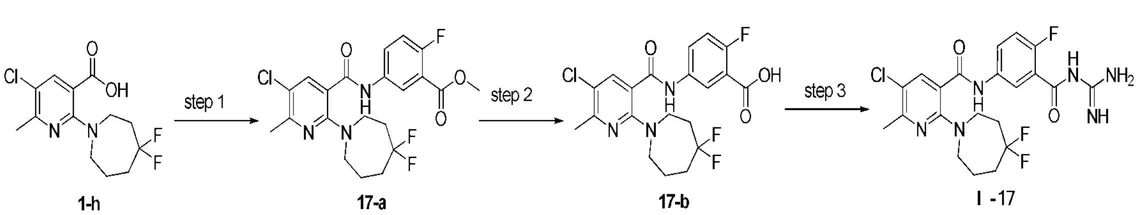


1-h 17-a 17-b I -17

Step 1: Following the process of Step 7 of Example 1, 5-chloro-2-(4,4-difluoroazepan-1-yl)-6-methylnicotinic acid (**1-h**) (500 mg, 1.64 mmol) and methyl 5-amino-2-fluorobenzoate (280 mg, 1.64 mmol) were used as starting materials to obtain 547 mg of methyl 5-(5-chloro-2-(4,4-difluoroazepan-1-yl)6-methylnicotinamido)-2-fluorobenzoate (**17-a**) as a

yellow solid in a yield of 73.1%. $^1$H NMR (400 MHz, $CDCl_3$) δ: 9.86(s, 1H), 8.10(s, 1H), 8.04(dd, *J* = 6.2, 2.8 Hz, 1H), 7.99~7.93(m, 1H), 7.17(t, *J* = 9.6 Hz, 1H), 3.94(s, 3H), 3.61~3.54(m, 2H), 3.42~3.35(m, 2H), 2.55(s, 3H), 2.48~2.33(m, 2H), 2.24~2.10(m, 2H), 1.98~1.87(m, 2H); MS(ESI) m/z $[M+H]^+$ = 457.0.

Step 2: Following the process of Step 6 of Example 1, methyl 5-(5-chloro-2-(4,4-difluoroazepan-1-yl)-6-methylnicotinamido)-2-fluorobenzoate (**17-a**) (400 mg, 0.88 mol) was used as a starting material to obtain 380 mg of 5-(5-chloro-2-(4,4-difluoroazepan-1-yl)-6-methylnicotinamido)-2-fluorobenzoic acid (**17-b**) as a white solid in a yield of 98%. $^1$H NMR (500 MHz, DMSO-$d_6$) δ: 10.61(s, 1H), 8.23(dd, *J* = 6.6, 2.8 Hz, 1H), 7.92~7.86(m, 1H), 7.76(s, 1H), 7.33~7.26(m, 1H), 3.63~3.57(m, 2H), 3.40(t, *J* = 6.0 Hz, 2H), 2.44(s, 3H), 2.35~2.25(m, 2H), 2.03~1.90(m, 2H), 1.88~1.77(m, 2H); MS(ESI) m/z $[M+H]^+$ = 443.0.

Step 3: Thionyl chloride (5 mL) was added to 5-(5-chloro-2-(4,4-difluoroazepan-1-yl)-6-methylnicotinamido)-2-fluorobenzoic acid (**17-b**) (200 mg, 0.45 mmol) and refluxed until the acid was completely converted to an acid chloride. The resultant was then concentrated to remove the thionyl chloride to obtain a crude acid chloride. The crude acid chloride was dissolved in anhydrous tetrahydrofuran (5 mL). A solution of guanidine hydrochloride (216 mg, 2.26 mmol) dissolved in a sodium hydroxide solution (2N, 1.36 mL, 2.7 mmol) was then added dropwise at 0°C and reacted at room temperature for 30 min. After evaporating tetrahydrofuran, the resultant was added with water (30 mL), extracted three times with ethyl acetate. The combined organic layer was washed once with saturated brine, dried over anhydrous sodium sulfate, and concentrated in vacuum to obtain a crude product. The crude product was purified by silica gel (200-300 mesh) column chromatography using PE:EA = 4-1:1 (v/v) and slurried in chloroform to obtain 30 mg of N-(3-(amidinocarbamoyl)-4-fluorophenyl)-5-chloro-2-(4,4-difluoroazepan-1-yl)-6-met hylnicotinamide (**I-17**) as a white solid in a yield of 13.7%. $^1$H NMR (500 MHz, DMSO-$d_6$) δ: 10.46 (s, 1H), 7.98 (dd, *J* = 6.6, 2.8 Hz, 1H), 7.73-7.65 (m, 2H), 7.12 (dd, *J* = 10.2, 8.8 Hz, 1H), 6.78 (s, 2H), 3.64-3.58 (m, 2H), 3.42(t, *J* = 6.0 Hz, 2H), 2.44(s, 3H), 2.34~2.27(m, 2H), 2.01~1.92(m, 2H), 1.87~1.82(m, 2H); MS(ESI) m/z $[M+H]^+$ = 483.9.

**Example 18:** Preparation of N-(3-(amidinocarbamoyl)phenyl)-5-chloro-2-(4,4-difluoroazepan-1-yl)-6-methylnicoti namide (**I-18**)

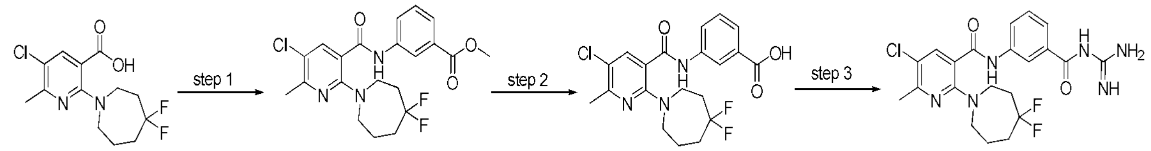


1-h 18-a 18-b I -18

Step 1: Following the process of Step 7 of Example 1, 5-chloro-2-(4,4-difluoroazepan-1-yl)-6-methylnicotinic acid (**1-h**) (500 mg, 1.64 mmol) and methyl 3-aminobenzoate (248 mg, 1.64 mmol) were used as starting materials to obtain 423 mg of methyl 3-(5-chloro-2-(4,4-difluoroazepan-1-yl)-6-methylnicotinamido)benzoate (**18-a**) as a yellow solid in a yield of 58.9%. $^1$H NMR (400 MHz, $CDCl_3$) δ: 9.75(s, 1H), 8.16(s, 1H), 8.09(s, 1H), 7.98(d, *J* = 8.4 Hz, 1H), 7.86~7.80(m, 1H), 7.46(t, *J* = 7.8 Hz, 1H), 3.93(s, 3H), 3.62~3.56(m, 2H), 3.41(t, *J* = 5.8 Hz, 2H), 2.55(s, 3H), 2.47~2.35(m, 2H), 2.21~2.08(m, 2H), 1.96~1.87(m, 2H); MS(ESI) m/z $[M+H]^+$ = 438.9.

Step 2: Following the process of Step 6 of Example 1, methyl 3-(5-chloro-2-(4,4-difluoroazepan-1-yl)-6-methylnicotinamido)benzoate (**18-a**) (300 mg, 0.69 mol) was used as a starting material to obtain 270 mg of 3-(5-chloro-2-(4,4-difluoroazepan-1-yl)-6-methylnicotinamido)benzoic acid (**18-b**) as a white solid in a yield of 93.1%. $^1$H NMR (400 MHz, DMSO-$d_6$) δ: 10.60(s, 1H), 8.34(t, *J* = 2.0 Hz, 1H), 7.94~7.88(m, 1H), 7.77(s, 1H), 7.71~7.64(m, 1H), 7.47(t, *J* = 7.8 Hz, 1H), 3.65~3.58(m, 2H), 3.42(t, *J* = 6.0 Hz, 2H), 2.45(s, 3H), 2.37~2.25(m, 2H), 2.03~1.90(m, 2H), 1.88~1.80(m, 2H); MS(ESI) m/z $[M+H]^+$ = 425.0.

Step 3: Following the process of Step 3 of Example 17, 3-(5-chloro-2-(4,4-difluoroazepan-1-yl)-6-methylnicotinamido)benzoic acid (**18-b**) (250 mg, 0.59 mmol) was used as a starting material to obtain 28 mg of N-(3-(amidinocarbamoyl)phenyl)-5-chloro-2-(4,4-difluoroazepan-1-yl)-6-methylnicoti namide (**I-18**) as a white solid in a yield of 10.2%. $^1$H NMR (500 MHz, DMSO-$d_6$) δ: 12.16(s, 1H), 10.74(s, 1H), 8.85(s, 1H), 8.67(s, 2H), 8.39(t, *J* = 2.0 Hz, 1H), 8.05(d, *J* = 7.8 Hz, 1H), 7.97(dd, *J* = 8.0, 2.1 Hz, 1H), 7.76(s, 1H), 7.55(t, *J* = 8.0 Hz, 1H), 3.64~3.59(m, 2H), 3.41(t, *J* = 6.0 Hz, 2H), 2.44(s, 3H), 2.35~2.26(m, 2H), 2.01~1.91(m, 2H), 1.86~1.81(m, 2H); MS(ESI) m/z$[M+H]^+$ = 466.0.

**Example 19:** Preparation of 5-chloro-2-(4,4-difluoroazepan-1-yl)-N-(4-fluoro-3-guanidinophenyl)-6-methylnicotin amide (**I-19**)

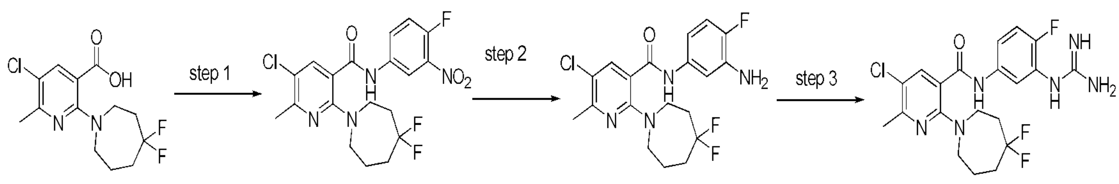


1-h 19-a 19-b I -19

Step 1: Following the process of Step 7 of Example 1, 5-chloro-2-(4,4-difluoroazepan-1-yl)-6-methylnicotinic acid (**1-h**) (400 mg, 1.31 mmol) and 4-fluoro-3-nitroaniline (205 mg, 1.31 mmol) were used as starting materials to obtain 290 mg of 5-chloro-2-(4,4-difluoroazepan-1-yl)-N-(4-fluoro-3-nitrophenyl)-6-methylnicotinamid e (**19-a**) as a yellow solid in a yield of 49.9%. $^1$H NMR (400 MHz, $CDCl_3$) δ: 10.30(s, 1H), 8.45(dd, *J* = 6.4, 2.8 Hz, 1H), 8.16(s, 1H), 7.92~7.84(m, 1H), 7.33~7.28(m, 1H), 3.59~3.55(m, 2H), 3.38~3.35(m, 2H), 2.57(s, 3H), 2.49~2.34(m, 2H), 2.26~2.12(m, 2H), 1.97~1.89(m, 2H); MS(ESI) m/z $[M+H]^+$ = 444.1.

Step 2: 5-chloro-2-(4,4-difluoroazepan-1-yl)-N-(4-fluoro-3-nitrophenyl)-6-methylnicotinamid e (**19-a**) (260 mg, 0.59 mmol) was dissolved in anhydrous ethanol (4 mL). Zinc powder (92 mg, 2.94 mmol) and acetic acid (2 mL) were then added and reacted at 80°C until the starting material was reacted completely. After the reaction was complete, the ethanol was evaporated, and the reaction mixture was adjusted to pH 8-9 by adding saturated sodium bicarbonate solution and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using PE:EA=10-4:1 (v/v) to obtain 227 mg of N-(3-amino-4-fluorophenyl)-5-chloro-2-(4,4-difluoroazepan-1-yl)-6-methylnicotinami de (**19-b**) as a yellow solid in a yield of 93.8%. $^1$H NMR (400 MHz, $CDCl_3$) δ: 9.46(s, 1H), 8.04(s, 1H), 7.38(dd, *J* = 8.0, 2.6 Hz, 1H), 6.99~6.90(m, 1H), 6.73~6.64(m, 1H), 3.82(s, 2H), 3.61~3.53(m, 2H), 3.38(t, *J* = 5.8 Hz, 2H), 2.54(s, 3H), 2.47~2.31(m, 2H), 2.21~2.07 (m, 2H), 1.96~1.87 (m, 2H); MS(ESI) m/z $[M+H]^+$ = 414.2.

Step 3: N-(3-amino-4-fluorophenyl)-5-chloro-2-(4,4-difluoroazepan-1-yl)-6-methylnicotinami de (**19-b**) (100 mg, 0.24 mmol) was dissolved in anhydrous ethanol (4 mL). 50% aqueous cyanamide (0.2 mL, 9.7 mmol) and 4N HCl (0.2 mL, 12.1 mmol) were added and reacted at 80°C in a sealed tube. After the reaction was complete, the reaction solution was concentrated, adjusted to pH 8-9 by adding a sodium hydroxide solution, and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using DCM:MeOH = 40:1-10:1 (v/v) to obtain 72 mg of 5-chloro-2-(4,4-difluoroazepan-1-yl)-N-(4-fluoro-3-guanidinophenyl)-6-methylnicotin amide (**I-19**) as a white solid in a yield of 65.3%. $^1$H NMR (500 MHz, Methanol-*d*$_4$) δ: 7.92~7.85(m, 1H), 7.69(s, 1H), 7.62~7.56(m, 1H), 7.29(t, *J* = 9.4 Hz, 1H), 3.72~3.66(m, 2H), 3.46(t, *J* = 6.0 Hz, 2H), 2.48(s, 3H), 2.37~2.28(m, 2H), 2.05~1.94(m, 2H), 1.94~1.87(m, 2H); MS(ESI) m/z $[M+H]^+$ = 456.2.

**Example 20:** Preparation of N-(3-amidinophenyl)-5-chloro-6-methyl-2-(pyrrolidin-1-yl)nicotinamide (**I-20**)

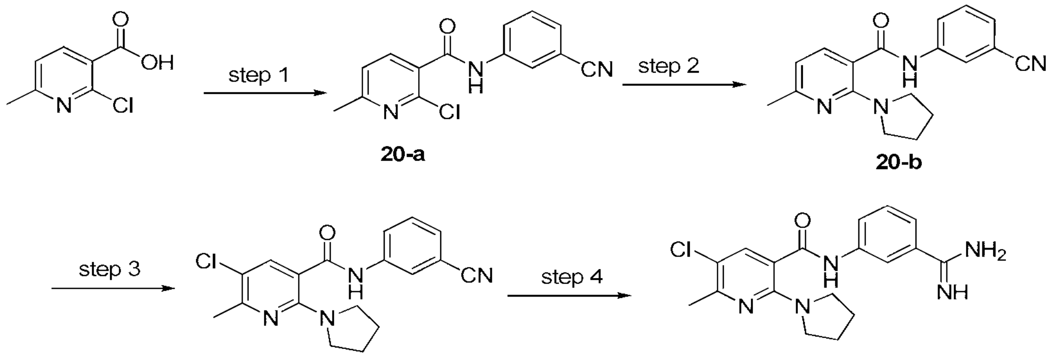


20-a 20-b 20-c I -20

Step 1: Following the process of Step 7 of Example 1, 2-chloro-6-methylnicotinic acid (200 mg, 1.16 mmol) and m-aminobenzonitrile (138 mg, 1.16 mmol) were used as starting materials to obtain 214 mg of 2-chloro-N-(3-cyanophenyl)-6-methylnicotinamide (**20-a**) as a yellowish solid in a yield of 67.5%. $^1$H NMR (400 MHz, $CDCl_3$) δ: 8.56 (s, 1H), 8.15~8.08 (m, 2H), 7.84~7.80 (m, 1H), 7.52~7.45 (m, 2H), 7.28~7.27 (m, 1H), 2.61 (s, 3H); MS(ESI) m/z $[M+H]^+$ = 273.0.

Step 2: Following the process of Step 4 of Example 1, 2-chloro-N-(3-cyanophenyl)-6-methylnicotinamide (**20-a**)

(500 mg, 1.84 mmol) and tetrahydropyrrole (307 μL, 3.68 mmol) were used as starting materials to obtain 400 mg of N-(3-cyanophenyl)-6-methyl-2-(pyrrolidin-1-yl)nicotinamide (**20-b**) as a white solid in a yield of 70.9%. $^1H$ NMR (400 MHz, DMSO-$d_6$) δ: 10.62(s, 1H), 8.19(d, $J$ = 2.4 Hz, 1H), 7.97~7.90(m, 1H), 7.60~7.50(m, 3H), 6.54(d, $J$ = 7.6 Hz, 1H), 3.38~3.35(m, 4H), 2.35(s, 3H), 1.86~1.79(m, 4H); MS(ESI) m/z $[M+H]^+$ = 307.0.

Step 3: Following the process of Step 5 of Example 1, N-(3-cyanophenyl)-6-methyl-2-(pyrrolidin-1-yl)nicotinamide (**20-b**) (75 mg, 0.24 mmol) was used as a starting material to obtain 66 mg of 5-chloro-N-(3-cyanophenyl)-6-methyl-2-(pyrrolidin-1-yl)nicotinamide (**20-c**) as a white solid in a yield of 83.4%. $^1H$ NMR (400 MHz, $CDCl_3$) δ: 9.19(s, 1H), 8.06~8.01(m, 1H), 7.85~7.77(m, 2H), 7.50~7.38(m, 2H), 3.43~3.39(m, 4H), 2.49(s, 3H), 1.95~1.90(m, 4H); MS(ESI) m/z $[M+H]^+$ = 342.0.

Step 4: Following the process of Example 12, 5-chloro-N-(3-cyanophenyl)-6-methyl-2-(pyrrolidin-1-yl)nicotinamide (**20-c**) was used as a starting material to obtain 15 mg of N-(3-amidinophenyl)-5-chloro-6-methyl-2-(pyrrolidin-1-yl) nicotinamide (**I-20**) as a white solid in a yield of 28.6%. $^1H$ NMR (500 MHz, DMSO-$d_6$) δ: 10.84(s, 1H), 9.35(s, 3H), 8.17(s, 1H), 8.01~7.96(m, 1H), 7.67(s, 1H), 7.59(t, $J$ = 8.0 Hz, 1H),7.52~7.47(m, 1H), 3.38~3.35(m, 4H), 2.43(s, 3H), 1.86~1.83(m, 4H); MS(ESI) m/z $[M+H]^+$ = 359.0.

**Example 21:** Preparation of N-(3-amidinophenyl)-5-chloro-6-methyl-2-(piperidin-1-yl)nicotinamide (**I-21**)

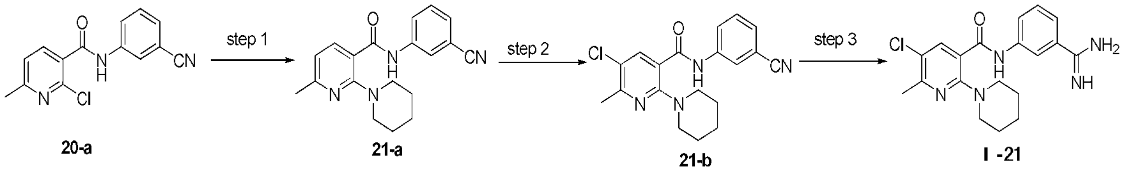


20-a 21-a 21-b I -21

Step 1: Following the process of Step 4 of Example 1, 2-chloro-N-(3-cyanophenyl)-6-methylnicotinamide (**20-a**) (100 mg, 0.37 mmol) and piperidine (72 μL, 0.74 mmol) were used as starting materials to obtain 95 mg of N-(3-cyanophenyl)-6-methyl-2-(piperidin-1-yl)nicotinamide (**21-a**) as a white solid in a yield of 80.51%. $^1H$ NMR (400 MHz, $CDCl_3$) δ: 12.34(s, 1H), 8.38(d, $J$ = 7.9 Hz, 1H), 8.21~8.19(m, 1H), 7.91~7.86(m, 1H), 7.47(t, $J$ = 7.9 Hz, 1H), 7.43~7.36(m, 1H), 7.07(d, $J$ = 7.9 Hz, 1H), 3.18~3.14(m, 4H), 2.54(s, 3H), 1.85~1.75(m, 4H), 1.72~1.65(m, 2H); MS(ESI) m/z $[M+H]^+$ = 321.0.

Step 2: Following the process of Step 5 of Example 1, N-(3-cyanophenyl)-6-methyl-2-(piperidin-1-yl)nicotinamide (**21-a**) (500 mg, 1.56 mmol) and 1,3-dichloro-5,5-dimethylhydantoin (277 mg, 1.4 mmol) were used as starting materials to obtain 319 mg of 5-chloro-N-(3-cyanophenyl)-6-methyl-2-(piperidin-1-yl)nicotinamide (**21-b**) as a yellow solid in a yield of 57.6%. $^1H$ NMR (400 MHz, DMSO-$d_6$) δ: 10.79(s, 1H), 8.20~8.19(m, 1H), 7.94~7.90(m, 1H), 7.79(s, 1H), 7.59~7.55(m, 2H), 3.70~3.53(m, 4H), 2.46(s, 3H), 1.54~1.49(m, 6H); MS(ESI) m/z $[M+H]^+$ = 356.0.

Step 3: Following the process of Example 12, 5-chloro-N-(3-cyanophenyl)-6-methyl-2-(piperidin-1-yl)nicotinamide (**21-b**) (300 mg, 0.85 mmol) was used as a starting material to obtain 80 mg of N-(3-amidinophenyl)-5-chloro-6-methyl-2-(piperidin-1-yl)nicotinamide (**I-21**) as a white solid in a yield of 25.5%. $^1H$ NMR (500 MHz, DMSO-$d_6$) δ: 10.87(s, 1H), 9.40(s, 3H), 8.25~8.22(m, 1H), 7.94(d, $J$ = 8.2 Hz, 1H), 7.77 (s, 1H), 7.60(t, $J$ = 8.0 Hz, 1H), 7.54~7.49(m, 1H), 3.31~3.27(m, 4H), 2.46(s, 3H), 1.54~1.52(m, 6H); MS(ESI) m/z $[M+H]^+$ = 373.0.

**Example 22:** Preparation of N-(3-amidinophenyl)-5-chloro-6-methyl-2-morpholinyl-nicotinamide (**I-22**)

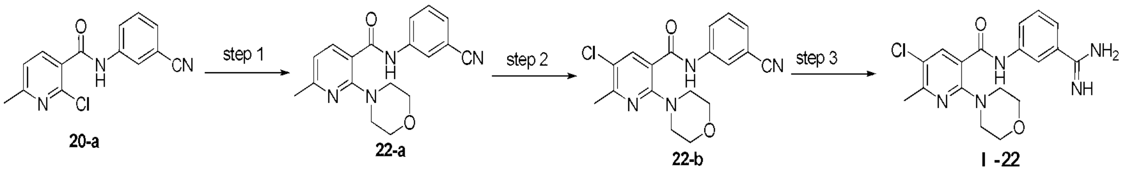


20-a 22-a 22-b I -22

Step 1: Following the process of Step 4 of Example 1, 2-chloro-N-(3-cyanophenyl)-6-methylnicotinamide (**20-a**) (100 mg, 0.37 mmol) and morpholine (65 μL, 0.74 mmol) were used as starting materials to obtain 61 mg of N-(3-cyanophenyl)-6-methyl-2-morpholinyl-nicotinamide (**22-a**) as a yellow solid in a yield of 51.3%. $^1H$ NMR (400 MHz, $CDCl_3$) δ: 11.59(s, 1H), 8.34(d, $J$ = 7.9 Hz, 1H), 8.25~8.24(m, 1H), 7.86~7.82(m, 1H), 7.48(t, $J$ = 7.9 Hz, 1H), 7.44~7.38(m, 1H), 7.09(d, $J$ = 7.9 Hz, 1H), 3.95~3.88(m, 4H), 3.29~3.22(m, 4H), 2.55(s,3H); MS(ESI) m/z $[M+H]^+$ = 323.0.

Step 2: Following the process of Step 5 of Example 1, N-(3-cyanophenyl)-6-methyl-2-morpholinyl-nicotinamide (**22-a**) (600 mg, 1.86 mmol) and 1,3-dichloro-5,5-dimethylhydantoin (330 mg, 1.68 mmol) were used as starting materials to obtain 477 mg of 5-chloro-N-(3-cyanophenyl)-6-methyl-2-morpholinyl-nicotinamide (**22-b**) as a yellow solid in a yield of 71.8%. $^1$H NMR (400 MHz, $CDCl_3$) δ: 11.53(s, 1H), 8.37(s, 1H), 8.23~8.18(m, 1H), 7.86~7.82(m, 1H), 7.49(t, *J* = 7.9 Hz, 1H), 7.45~7.42(m, 1H), 3.93~3.89(m, 4H), 3.25~3.21(m, 4H), 2.62(s, 3H); MS(ESI) m/z $[M+H]^+$ = 358.0.

Step 3: Following the process of Example 12, 5-chloro-N-(3-cyanophenyl)-6-methyl-2-morpholinyl-nicotinamide (**22-b**) (400 mg, 1.12 mmol) was used as a starting material to obtain 120 mg of N-(3-amidinophenyl)-5-chloro-6-methyl-2-morpholinyl-nicotinamide (**I-22**) as a white solid in a yield of 28.6%. $^1$H NMR (500 MHz, DMSO-$d_6$) δ: 10.90 (s, 1H), 9.41(s, 3H), 8.22(d, *J* = 2.2 Hz, 1H), 7.97(d, *J* = 8.0 Hz, 1H), 7.81(s, 1H), 7.60 (t, *J* = 8.0 Hz, 1H), 7.55~7.50(m, 1H), 3.66~3.61(m, 4H), 3.34~3.28(m, 4H), 2.48(s, 3H); MS(ESI) m/z $[M+H]^+$ = 375.0.

**Example 23:** Preparation of 2-(azepan-1-yl)-N-(3-amidinophenyl)-5-chloro-6-methylnicotinamide (**I-23**)

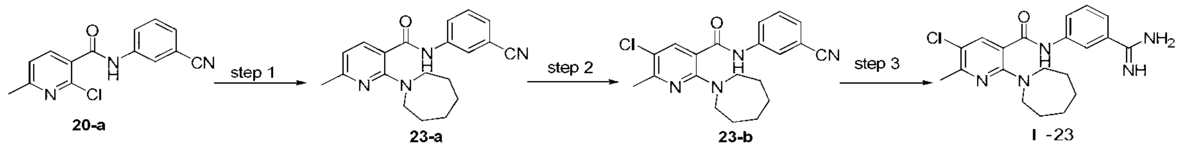


20-a 23-a 23-b I -23

Step 1: Following the process of Step 4 of Example 1, 2-chloro-N-(3-cyanophenyl)-6-methylnicotinamide (**20-a**) (100 mg, 0.37 mmol) and azepane (85 μL, 0.74 mmol) were used as starting materials to obtain 67 mg of 2-(azepan-1-yl)-N-(3-cyanophenyl)-6-methylnicotinamide (**23-a**) as a yellow solid in a yield of 54.5%. $^1$H NMR (400 MHz, $CDCl_3$) δ: 11.27 (s, 1H), 8.21 (d, *J* = 7.8 Hz, 1H), 8.13 (s, 1H), 7.83 (d, *J* = 8.2 Hz, 1H), 7.46 (t, *J* = 7.8 Hz, 1H), 7.43~7.37 (m, 1H), 6.94 (d, *J* = 7.8 Hz, 1H), 3.43~3.36 (m, 4H), 2.51 (s, 3H), 1.89~1.82 (m, 4H), 1.74~1.70 (m, 4H); MS(ESI) m/z $[M+H]^+$ = 335.1.

Step 2: Following the process of Step 5 of Example 1, 2-(azepan-1-yl)-N-(3-cyanophenyl)-6-methylnicotinamide (**23-a**) (450 mg, 1.35 mmol) and 1,3-dichloro-5,5-dimethylhydantoin (265 mg, 1.35 mmol) were used as starting materials to obtain 240 mg of 2-(azepan-1-yl)-5-chloro-N-(3-cyanophenyl)-6-methylnicotinamide (**23-b**) as a yellow solid in a yield of 48.4%. $^1$H NMR (400 MHz, $CDCl_3$) δ: 10.98(s, 1H), 8.22~8.17(m, 1H), 8.09~8.06(m, 1H), 7.84~7.81(m, 1H), 7.47(t, *J* = 7.8 Hz, 1H), 7.44~7.40(m, 1H), 3.42~3.35(m, 4H), 2.56(s, 3H), 1.86~1.84(m, 4H), 1.73~1.68(m, 4H); MS(ESI) m/z $[M+H]^+$ = 370.0.

Step 3: Following the process of Example 12, 2-(azepan-1-yl)-5-chloro-N-(3-cyanophenyl)-6-methylnicotinamide (**23-b**) (200 mg, 0.54 mmol) was used as a starting material to obtain 20 mg of 2-(azepan-1-yl)-N-(3-amidinophenyl)-5-chloro-6-methylnicotinamide (**I-23**) as a white solid in a yield of 9.6%. $^1$H NMR (500MHz, DMSO-$d_6$) δ: 10.85(s, 1H), 9.37(s, 3H), 8.16~8.15(m, 1H), 7.98(d, *J* = 7.8 Hz, 1H), 7.63 (s, 1H), 7.58(t, *J* = 8.0 Hz, 1H), 7.51~7.48(m, 1H), 3.51~3.46(m, 4H), 2.43(s, 3H), 1.75~1.70(m, 4H), 1.46~1.43(m, 4H); MS(ESI) m/z $[M+H]^+$ = 387.0.

**Example 24:** Preparation of 5-chloro-2-(4,4-difluoroazepan-1-yl)-N-(4-fluoro-3-(N'-hydroxyamidino)phenyl)-6-isop ropylnicotinamide (**I-24**)

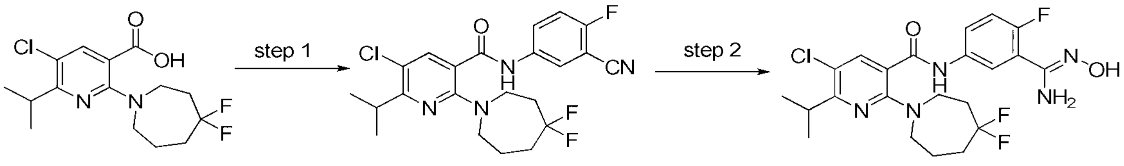


24-a 24-b I -24

Step 1: Following the process of Step 7 of Example 1, 5-chloro-2-(4,4-difluoroazepan-1-yl)-6-isopropylnicotinamide (**24-a**) (133 mg, 0.40 mmol) and 5-amino-2-fluorobenzonitrile (54 mg, 0.40 mmol) were used as starting materials to obtain 75 mg of 5-chloro-N-(3-cyano-4-fluorophenyl)-2-(4,4-difluoroazepan-1-yl)-6-isopropylnicotina mide (**24-b**) as a white solid in a yield of 41.7%. $^1$H NMR (400 MHz, $CDCl_3$) δ: 9.80(s, 1H), 8.09(s, 1H), 8.09~8.06(m, 1H), 7.78~7.73(m, 1H), 7.22(t, *J* = 8.8 Hz, 1H), 3.63~3.59(m, 2H), 3.54~3.47(m, 1H), 3.39(t, *J* = 5.8 Hz, 2H), 2.48~2.34(m, 2H), 2.23~2.09(m, 2H), 1.99~1.88(m, 2H), 1.26(s, 3H), 1.25(s, 3H) ; MS(ESI) m/z $[M+H]^+$ = 452.2.

Step 2: Following the process of Step 8 of Example 1, 5-chloro-N-(3-cyano-4-fluorophenyl)-2-(4,4-difluoroazepan-1-yl)-6-isopropylnicotina mide (**24-b**) (70 mg, 0.16 mmol) was used as a starting material to obtain 54 mg of 5-chloro-2-(4,4-difluoroazepan-1-yl)-N-(4-fluoro-3-(N'-hydroxyamidino)phenyl)-6-isop ropylnicotinamide (**I-24**) as a white solid in a yield of 72%. $^1$H NMR (500 MHz, DMSO-$d_6$) δ: 10.52(s, 1H), 9.64(s, 1H), 7.86~7.83(m, 1H), 7.72~7.68(m, 2H), 7.22(t, $J$ = 9.6 Hz, 1H), 5.79(s, 2H), 3.66~3.62(m, 2H), 3.43(t, $J$ = 6.0 Hz, 2H), 3.39~3.35(m, 1H), 2.35~2.27(m, 2H), 2.00~1.93(m, 2H), 1.88~1.83(m, 2H), 1.21(s, 3H), 1.19(s, 3H); MS (ESI) m/z $[M+H]^+$ = 485.2.

**Example 25:** Preparation of 6-chloro-3-(4,4-difluoroazepan-1-yl)-N-(4-fluoro-3-(N'-hydroxyamidino)phenyl)pyrida zine-4-carboxamide (**I-25**)

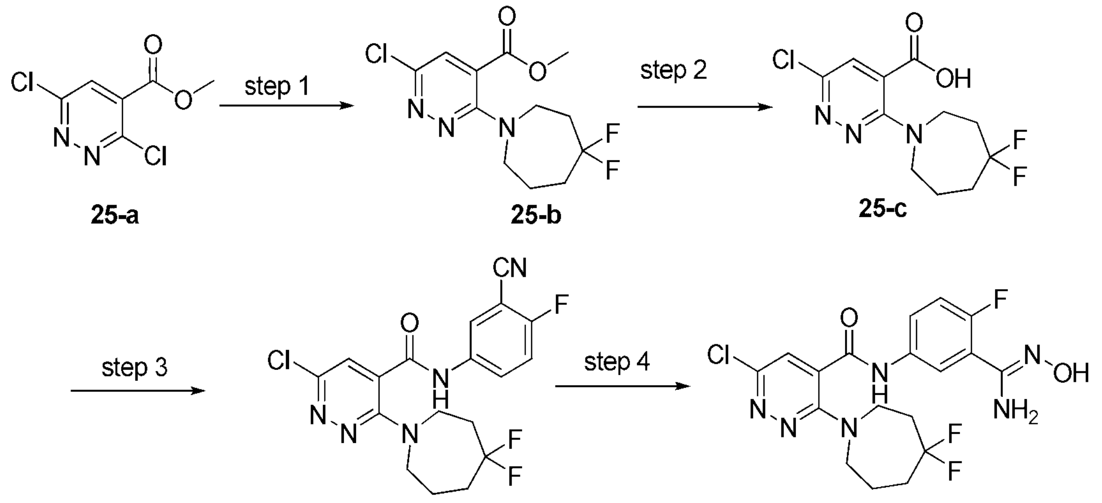


25-a 25-b 25-c

25-d I -25

Step 1: Following the process of Step 4 of Example 1, methyl 3,6-dichloropyridazine-4-carboxylate (**25-a**) (900 mg, 4.35 mmol) and 4,4-difluoroazepane hydrochloride (**1-c**) (896 mg, 5.22 mmol) were used as starting materials to obtain 560 mg of methyl 6-chloro-3-(4,4-difluoroazepan-1-yl)pyridazine-4-carboxylate (**25-b**) as a colorless liquid in a yield of 42.1%. $^1$H NMR (400 MHz, $CDCl_3$) δ: 7.49(s, 1H), 3.93(s, 3H), 3.85~3.78(m, 2H), 3.35-3.30 (m, 2H), 2.48-2.33 (m, 2H), 2.06-1.98 (m, 4H); MS (ESI) m/z $[M+H]^+$ = 306.9.

Step 2: Following the process of Step 6 of Example 1, methyl 6-chloro-3-(4,4-difluoroazepan-1-yl)pyridazine-4-carboxylate (**25-b**) (560 mg, 1.83 mmol) was used as a starting material to obtain 515 mg of 6-chloro-3-(4,4-difluoroazepan-1-yl)pyridazine-4-carboxylic acid (**25-c**) as a white solid in a yield of 96.4%. $^1$H NMR (400 MHz, DMSO-$d_6$) δ: 7.72 (s, 1H), 3.81~3.68 (m, 2H), 3.39 (t, $J$ = 5.8 Hz, 2H), 2.42~2.30 (m, 2H), 2.12~1.99 (m, 2H), 1.95~1.88 (m, 2H); MS(ESI) m/z $[M+H]^+$ = 293.0.

Step 3: Following the process of Step 7 of Example 1, 6-chloro-3-(4,4-difluoroazepan-1-yl)pyridazine-4-carboxylic acid (**25-c**) (200 mg, 0.686 mmol) and 5-amino-2-fluorobenzonitrile (93 mg, 0.686 mmol) were used as starting materials to obtain 177 mg of 6-chloro-N-(3-cyano-4-fluorophenyl)-3-(4,4-difluoroazepan-1-yl)pyridazine-4-carboxa mide (**25-d**) as a yellow solid in a yield of 63.0%. $^1$H NMR (400 MHz, DMSO-$d_6$) δ: 11.10 (s, 1H), 8.15 (dd, $J$ = 5.8, 2.8 Hz, 1H), 7.95~7.90 (m, 1H), 7.83~7.81 (m, 1H), 7.59 (t, $J$ = 9.2 Hz, 1H), 3.76~3.69 (m, 2H), 3.50 (t, $J$ = 6.0 Hz, 2H), 2.41~2.26 (m, 2H), 2.11~1.98 (m, 2H), 1.92~1.86 (m, 2H); MS(ESI) m/z $[M+H]^+$ = 410.9.

Step 4: Following the process of Step 8 of Example 1, 6-chloro-N-(3-cyano-4-fluorophenyl)-3-(4,4-difluoroazepan-1-yl)pyridazine-4-carboxa mide (**25-d**) (170 mg, 0.41 mmol) was used as a starting material to obtain 25 mg of 6-chloro-3-(4,4-difluoroazepan-1-yl)-N-(4-fluoro-3-(N'-hydroxyamidino)phenyl)pyrida zine-4-carboxamide (**I-25**) as a white solid in a yield of 13.6%. $^1$H NMR (500 MHz, DMSO-$d_6$) δ: 10.83(s, 1H), 9.67(s, 1H), 7.82(dd, $J$ = 6.4, 2.8 Hz, 1H), 7.78(s, 1H), 7.71~7.65(m, 1H), 7.26(t, $J$ = 9.6 Hz, 1H), 5.81(s, 2H), 3.76~3.71(m, 2H), 3.52(t, $J$ = 6.0 Hz, 2H), 2.39~2.28(m, 2H), 2.07~1.98(m, 2H), 1.91~1.86(m, 2H); MS(ESI) m/z $[M+H]^+$ = 444.1.

**Example 26:** Preparation of 2-(4,4-difluoroazepan-1-yl)-5-ethynyl-N-(4-fluoro-3-(N'-hydroxyamidino)phenyl)-6-m ethylnicotinamide (**I-26**)

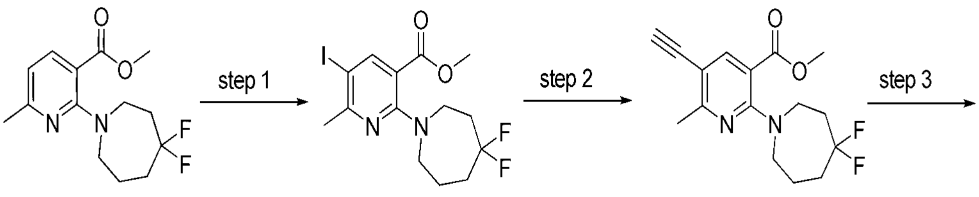


1-f 26-a 26-b

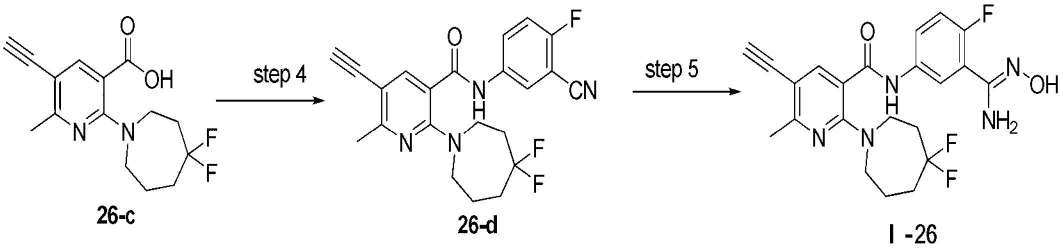


26-c 26-d I -26

Step 1: Methyl 2-(4,4-difluoroazepan-1-yl)-6-methylnicotinamide (**1-f**) (840 mg, 2.95 mmol) was dissolved in acetic acid (5 mL) and then *N*-iodosuccinimide (665 mg, 2.95 mmol) was added and reacted at room temperature. After the reaction was complete, the reaction mixture was poured into ice water, added with an appropriate amount of a sodium thiosulfate solution, adjusted to pH 7-8 with saturated sodium bicarbonate solution, and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using PE:EA = 30:1 (v/v) to obtain 1.2 g of methyl 5-iodo-2-(4,4-difluoroazepan-1-yl)-6-methylnicotinate (**26-a**) as a brown liquid in a yield of 99%. $^1$H NMR (400 MHz, $CDCl_3$) δ: 8.16 (s, 1H), 3.85 (s, 3H), 3.74-3.66 (m, 2H), 3.25 (t, *J* = 5.8 Hz, 2H), 2.58 (s, 3H), 2.42-2.27 (m, 2H), 1.99-1.90 (m, 4H); MS (ESI) m/z $[M+H]^+$ = 411.1.

Step 2: methyl 5-iodo-2-(4,4-difluoroazepan-1-yl)-6-methylnicotinate (**26-a**) (300 mg, 0.73 mmol), cuprous iodide (70 mg, 0.37 mmol), bis(triphenylphosphine)palladium dichloride (51.3 mg, 0.07 mmol), and trimethylethynylsilane (620 μL, 4.4 mmol) were added to triethylamine (4 mL) and reacted at 90°C under argon. After the reaction was complete, the triethylamine was evaporated, and potassium carbonate (202 mg, 1.46 mmol) and methanol (3 mL) were added and reacted at room temperature to remove the silicon protecting group. After the reaction was complete, the reaction solution was filtered, and the filtrate was retained, added with water, and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using PE:EA=50:1 (v/v) to obtain 143 mg of methyl 2-(4,4-difluoroazepan-1-yl)-5-ethynyl-6-methylnicotinate (**26-b**) as a brown liquid in a yield of 63.4%. $^1$H NMR (400 MHz, $CDCl_3$) δ: 7.97(s, 1H), 3.85(s, 3H), 3.78~3.71(m, 2H), 3.30~3.26(m, 2H), 3.24(s, 1H), 2.53(s, 3H), 2.41~2.30(m, 2H), 1.99~1.90(m, 4H); MS(ESI) m/z $[M+H]^+$ = 309.2.

Step 3: Following the process of Step 6 of Example 1, methyl 2-(4,4-difluoroazepan-1-yl)-5-ethynyl-6-methylnicotinate (**26-b**) (110 mg, 0.36 mmol) was used as a starting material to obtain 100 mg of 2-(4,4-difluoroazepan-1-yl)-5-ethynyl-6-methylnicotinic acid (**26-c**) as a white solid in a yield of 95.2%. $^1$H NMR (400 MHz, $CDCl_3$) δ: 8.28(s, 1H), 3.67~3.60(m, 2H), 3.35~3.32(m, 2H), 3.32(s, 1H), 2.60(s, 3H), 2.48~2.31(m, 2H), 2.14~2.0(m, 2H), 2.05~1.95 (m, 2H); MS(ESI) m/z $[M+H]^+$ = 295.2.

Step 4: Following the process of Step 7 of Example 1, 2-(4,4-difluoroazepan-1-yl)-5-ethynyl-6-methylnicotinic acid (**26-c**) (100 mg, 0.34 mmol) and 5-amino-2-fluorobenzonitrile (46 mg, 0.34 mmol) were used as starting materials to obtain 20 mg of N-(3-cyano-4-fluorophenyl)-2-(4,4-difluoroazepan-1-yl)-5-ethynyl-6-methylnicotinami de (**26-d**) as a white solid in a yield of 14.3%. $^1$H NMR (400 MHz, $CDCl_3$) δ: 8.87(s, 1H), 8.07~8.03(m, 1H), 8.00(s, 1H), 7.78~7.73(m, 1H), 7.22(t, *J* = 8.6 Hz, 1H), 3.71~3.64(m, 2H), 3.41(t, *J* = 5.8Hz, 2H), 3.32(s, 1H), 2.59(s, 3H), 2.44~2.34(m, 2H), 2.10~1.99(m, 2H), 1.97~1.83(m, 2H); MS(ESI) m/z $[M+H]^+$ = 413.2.

Step 5: Following the process of Step 8 of Example 1, N-(3-cyano-4-fluorophenyl)-2-(4,4-difluoroazepan-1-yl)-5-ethynyl-6-methylnicotinami de (**26-d**) (20 mg, 0.05 mmol) was used as a starting material to obtain 10 mg of 2-(4,4-difluoroazepan-1-yl)-5-ethynyl-N-(4-fluoro-3-(N'-hydroxyamidino)phenyl)-6-m ethylnicotinamide (**I-26**) as a white solid in a yield of 46.3%. 1H-NMR (500MHz, DMSO-$d_6$) δ: 10.51(s, 1H), 9.66(s, 1H), 7.85(dd, *J* = 6.4, 2.8 Hz, 1H), 7.74~7.69(m, 2H), 7.22(t, *J* = 9.6 Hz, 1H), 5.82(s, 2H), 4.31(s, 1H), 3.68~3.62(m, 2H), 3.44(t, *J* = 6.0 Hz, 2H), 2.48(s, 3H), 2.36~2.26(m, 2H), 2.00~1.92(m, 2H), 1.87~1.83(m, 2H); MS(ESI) m/z $[M+H]^+$ = 446.2.

**Example 27**: Preparation of 2-(4,4-difluoroazepan-1-yl)-N-(4-fluoro-3-(N'-hydroxyamidino)phenyl)-6-methyl-5-(tri fluoromethyl)nicotinamide (**I-27**)

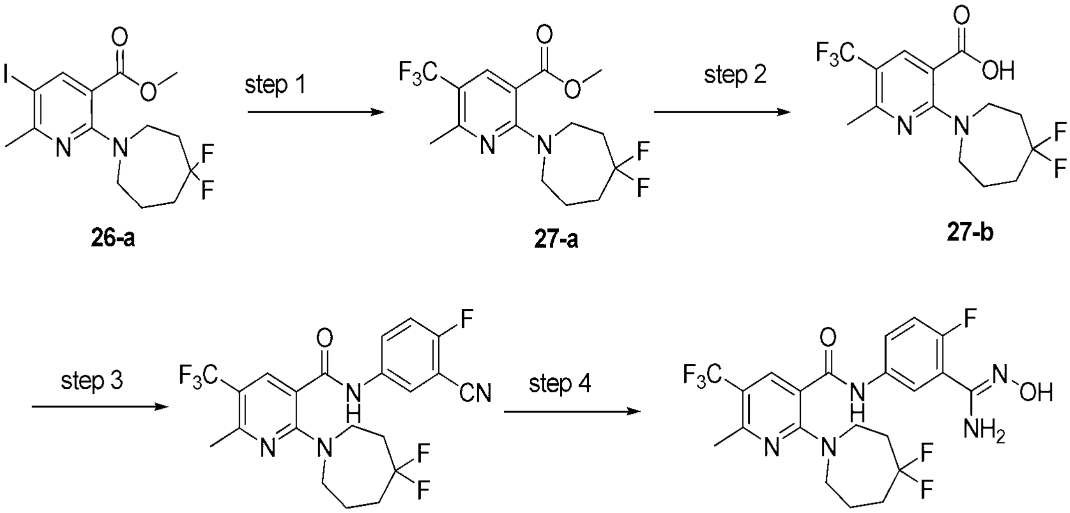


26-a 27-a 27-b 27-c I -27

Step 1: Under the protection of argon, methyl 5-iodo-2-(4,4-difluoroazepan-1-yl)-6-methylnicotinate (**26-a**) (400 mg, 0.98 mmol) obtained in Example 26 was dissolved in N,N-dimethylformamide (8 mL), added with cuprous iodide (371 mg, 1.95 mmol) and methyl fluorosulfonyldifluoroacetate (1.5 mL, 11.7 mmol) and reacted at 100°C. After the reaction was complete, the reaction mixture was filtered and the filtrate was retained, added with water (40 mL), and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using PE:EA=30:1 to 15:1 (v/v) to obtain 330 mg of methyl 2-(4,4-difluoroazepan-1-yl)-6-methyl-5-(trifluoromethyl)nicotinate (**27-a**) as a colorless liquid in a yield of 96.1%. $^1$H NMR (400 MHz, $CDCl_3$) δ: 8.10(s, 1H), 3.87(s, 3H), 3.80'''3.77(m, 2H), 3.32~3.28(m, 2H), 2.54(s, 3H), 2.41~2.30(m, 2H), 2.00~1.93(m, 4H); MS(ESI) m/z $[M+H]^+$ = 353.1.

Step 2: Following the process of Step 6 of Example 1, methyl 2-(4,4-difluoroazepan-1-yl)-6-methyl-5-(trifluoromethyl)nicotinate (**27-a**) (330 mg, 0.94 mmol) was used as a starting material to obtain 296 mg of 2-(4,4-difluoroazepan-1-yl)-6-methyl-5-(trifluoromethyl)nicotinic acid (**27-b**) as a white solid in a yield of 93.7%. $^1$H NMR (400 MHz, $CDCl_3$) δ: 8.30(s, 1H), 3.83~3.77(m, 2H), 3.40~3.35(m, 2H), 2.58(s, 3H), 2.44~2.32(m, 2H), 2.03~1.96(m, 4H); MS(ESI) m/z $[M+H]^+$ = 339.2.

Step 3: Following the process of Step 7 of Example 1, 2-(4,4-difluoroazepan-1-yl)-6-methyl-5-(trifluoromethyl) nicotinic acid (**27-b**) (296 mg, 0.88 mmol) and 5-amino-2-fluorobenzonitrile (119 mg, 0.875 mmol) were used as starting materials to obtain 250 mg of N-(3-cyano-4-fluorophenyl)-2-(4,4-difluoroazepan-1-yl)-6-methyl-5-(trifluoromethyl)n icotinamide **(27-c)** as a white solid in a yield of 62.6%. $^1$H NMR (400 MHz, DMSO-$d_6$) δ: 10.81(s, 1H), 8.18(dd, J = 5.8, 2.8 Hz, 1H), 7.99~7.90(m, 2H), 7.55(t, J = 9.2 Hz, 1H), 3.74~3.66(m, 2H), 3.45 (t, J = 5.8 Hz, 2H), 2.50(s, 3H), 2.38~2.28(m, 2H), 2.02~1.93(m, 2H), 1.90~1.85(m, 2H); MS(ESI) m/z $[M+H]^+$ = 456.9.

Step 4: Following the process of Step 8 of Example 1, N-(3-cyano-4-fluorophenyl)-2-(4,4-difluoroazepan-1-yl)-6-methyl-5-(trifluoromethyl)n icotinamide **(27-c)** (100 mg, 0.22 mmol) was used as a starting material to obtain 20 mg of 2-(4,4-difluoroazepan-1-yl)-N-(4-fluoro-3-(N'-hydroxyamidino)phenyl)-6-methyl-5-(tri fluoromethyl)nicotinamide (**I-27**) as a white solid in a yield of 18.7%. $^1$H NMR (500 MHz, DMSO-$d_6$) δ: 10.57(s, 1H), 9.64(s, 1H), 7.88(s, 1H), 7.86~7.81(m, 1H), 7.74~7.69(m, 1H), 7.22(t, J = 9.6Hz, 1H), 5.80(s, 2H), 3.72~3.69(m, 2H), 3.48(t, J = 6.0 Hz, 2H), 2.50(s, 3H), 2.37~2.29(m, 2H), 2.02~1.94(m, 2H), 1.90~1.85(m, 2H); MS(ESI) m/z $[M+H]^+$ = 489.8.

**Example 28:** Preparation of 5-cyano-2-(4,4-difluoroazepan-1-yl)-N-(4-fluoro-3-(N'-hydroxyamidino)phenyl)-6-met hylnicotinamide (**I-28**)

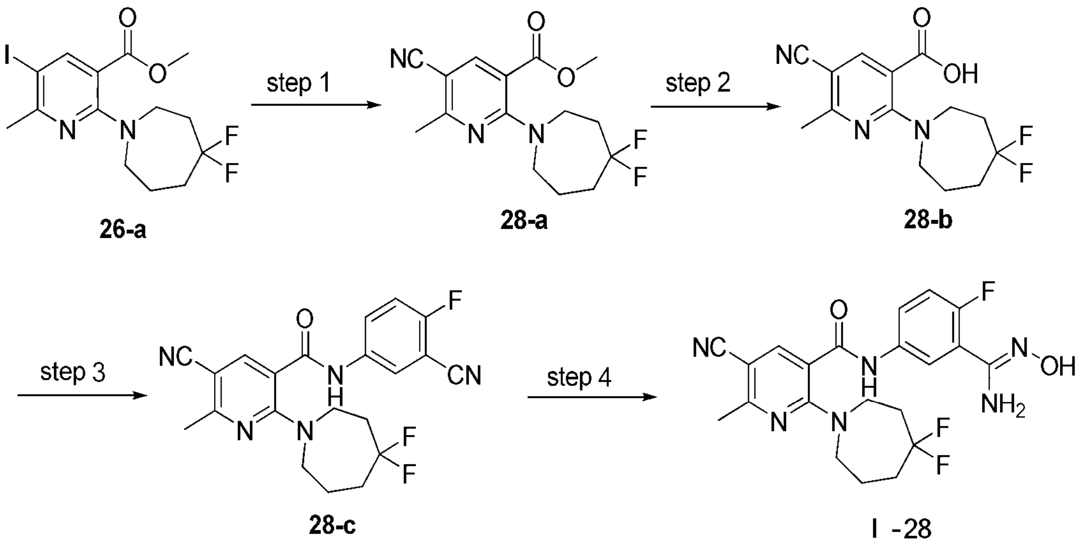


Step 1: Under **argon** atmosphere, methyl 5-iodo-2-(4,4-difluoroazepan-1-yl)-6-methylnicotinate **(26-a)** (500 mg, 1.22 mmol) obtained in Example 26 was dissolved in N,N-dimethylacetamide (10 mL), added with sodium carbonate (258 mg, 2.44 mmol) and potassium ferrocyanide trihydrate (984 mg, 3.66 mmol) and reacted at 100°C. After the reaction was complete, the reaction system was added with water (40 mL), and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using PE:EA = 20:1 (v/v) to obtain 300 mg of methyl 5-cyano-2-(4,4-difluoroazepan-1-yl)-6-methylnicotinate **(28-a)** as a colorless liquid in a yield of 79.6%. $^1$H NMR (400 MHz, $CDCl_3$) δ: 8.08 (s, 1H), 3.87 (s, 3H), 3.80-3.76 (m, 2H), 3.31 (t, J = 5.6 Hz, 2H), 2.59 (s, 3H), 2.41-2.26 (m, 2H), 2.01-1.91 (m, 4H); MS (ESI) m/z $[M+H]^+$ = 310.2.

Step 2: Following the process of Step 6 of Example 1, methyl 5-cyano-2-(4,4-difluoroazepan-1-yl)-6-methylnicotinate **(28-a)** (390 mg, 1.26 mmol) was used as a starting material to obtain 360 mg of 5-cyano-2-(4,4-difluoroazepan-1-yl)-6-methylnicotinic acid **(28-b)** as a white solid in a yield of 96.7%. $^1$H NMR (400 MHz, $CDCl_3$) δ: 8.27(s, 1H), 3.86~3.79(m, 2H), 3.39(t, J = 5.8 Hz, 2H), 2.62(s, 3H), 2.43~2.29(m, 2H), 2.04~1.94(m, 4H); MS(ESI) m/z $[M+H]^+$ = 296.1.

Step 3: Following the process of Step 7 of Example 1, 5-cyano-2-(4,4-difluoroazepan-1-yl)-6-methylnicotinic acid **(28-b)** (390 mg, 1.32 mmol) and 5-amino-2-fluorobenzonitrile (180 mg, 1.32 mmol) were used as starting materials to obtain 380 mg of 5-cyano-N-(3-cyano-4-fluorophenyl)-2-(4,4-difluoroazepan-1-yl)-6-methylnicotinamid e **(28-c)** as a white solid in a yield of 69.6%. $^1$H NMR (400 MHz, DMSO-$d_6$) δ: 10.81(s, 1H), 8.18(dd, J = 5.8, 2.8 Hz, 1H), 8.13(s, 1H), 7.98~7.89(m, 1H), 7.56(t, J = 9.0 Hz, 1H), 3.75~3.68(m, 2H), 3.47(t, J = 5.8 Hz, 2H), 2.54(s, 3H), 2.38~2.27(m, 2H), 2.03~1.95(m, 2H), 1.93~1.85(m, 2H); MS(ESI) m/z $[M+H]^+$ = 413.9.

Step 4: Following the process of Step 8 of Example 1, 5-cyano-N-(3-cyano-4-fluorophenyl)-2-(4,4-difluoroazepan-1-yl)-6-methylnicotinamid e **(28-c)** (100 mg, 0.24 mmol) was used as a starting material to obtain 47 mg of 5-cyano-2-(4,4-difluoroazepan-1-yl)-N-(4-fluoro-3-(N'-hydroxyamidino)phenyl)-6-met hylnicotinamide (**I-28**) as a white solid in a yield of 43.5%. $^1$H NMR (400 MHz, DMSO-$d_6$) δ: 10.56(s, 1H), 9.65(s, 1H), 8.07(s, 1H), 7.83(dd, J = 6.6, 2.8 Hz, 1H), 7.73~7.65(m, 1H), 7.26~7.20(m, 1H), 5.82(s, 2H), 3.75~3.68(m,2H), 3.48(t, J = 6.0 Hz, 2H), 2.53(s, 3H), 2.34~2.30(m, 2H), 2.02~1.94 (m, 2H), 1.90~1.83(m, 2H); MS(ESI) m/z $[M+H]^+$ = 447.2.

**Example 29:** Preparation of 2-(4,4-difluoroazepan-1-yl)-N-(4-fluoro-3-(N'-hydroxyamidino)phenyl)-6-methylnicoti namide **(1-29)**

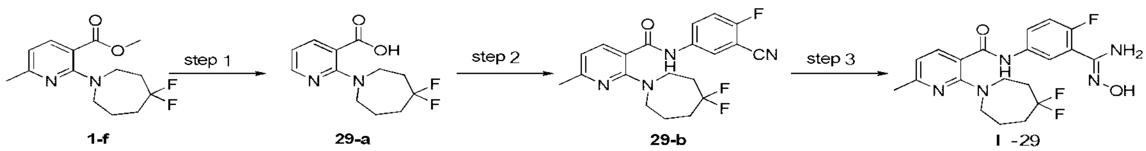


Step 1: Following the process of Step 6 of Example 1, methyl 2-(4,4-difluoroazepan-1-yl)-6-methylnicotinate **(1-f)** (400 mg, 1.41 mmol) was used as a starting material to obtain 309 mg of 2-(4,4-difluoroazepan-1-yl)-6-methylnicotinic acid **(29-a)** as a white solid in a yield of 81.3%. $^1$H NMR (400 MHz, $CDCl_3$) δ: 8.35(d, J = 7.9 Hz, 1H), 7.12(d, J = 7.9 Hz, 1H),

3.46~3.39(m, 2H), 3.31 (t, J = 5.8 Hz, 2H), 2.56(s, 3H), 2.51~2.40(m, 2H), 2.35~2.20(m, 2H), 2.06~1.99(m, 2H); MS(ESI) m/z $[M+H]^+$ = 270.9.

Step 2: Following the process of Step 7 of Example 1, 2-(4,4-difluoroazepan-1-yl)-6-methylnicotinic acid **(29-a)** (400 mg, 1.48 mmol) and 5-amino-2-fluorobenzonitrile (202 mg, 1.48 mmol) were used as starting materials to obtain 160 mg of N-(3-cyano-4-fluorophenyl)-2-(4,4-difluoroazepan-1-yl)-6-methylnicotinamide **(29-b)** as a white solid in a yield of 27.8%. $^1$H NMR (400 MHz, $CDCl_3$) δ: 10.40(s, 1H), 8.17~8.10(m, 2H), 7.78~7.69(m, 1H), 7.21(t, J = 8.8 Hz, 1H), 6.93(d, J = 7.8 Hz, 1H), 3.61~3.54(m, 2H), 3.37(t, J = 5.8 Hz, 2H), 2.50(s, 3H), 2.47~2.35(m, 2H), 2.26~2.12(m, 2H), 1.95~1.89(m, 2H); MS(ESI) m/z $[M+H]^+$ = 388.9.

Step 3: Following the process of Step 8 of Example 1, N-(3-cyano-4-fluorophenyl)-2-(4,4-difluoroazepan-1-yl)-6-methylnicotinamide **(29-b)** (70 mg, 0.18 mmol) was used as a starting material to obtain 27 mg of 2-(4,4-difluoroazepan-1-yl)-N-(4-fluoro-3-(N'-hydroxyamidino)phenyl)-6-methylnicoti namide (**I-29**) as a white solid in a yield of 35.5%. $^1$H NMR (600 MHz, $CDCl_3$) δ: 9.85(s, 1H), 8.02(d, J = 7.8 Hz, 1H), 7.88~7.79(m,2H), 7.12 (t, J = 10.0 Hz,1H), 6.83 (d, J = 7.8 Hz,1H), 5.45(s, 2H), 3.62~3.59(m, 2H), 3.41 (t, J = 5.8 Hz, 2H), 2.48(s, 3H), 2.43~2.35(m, 2H), 2.21~2.07(m, 2H), 1.97~1.84(m, 2H); MS(ESI) m/z $[M+H]^+$ = 421.9.

**Example 30:** Preparation of 2-(4,4-difluoroazepan-1-yl)-N-(4-fluoro-3-(N'-hydroxyamidino)phenyl)-6-(trifluoromet hyl) nicotinamide **(1-30)**

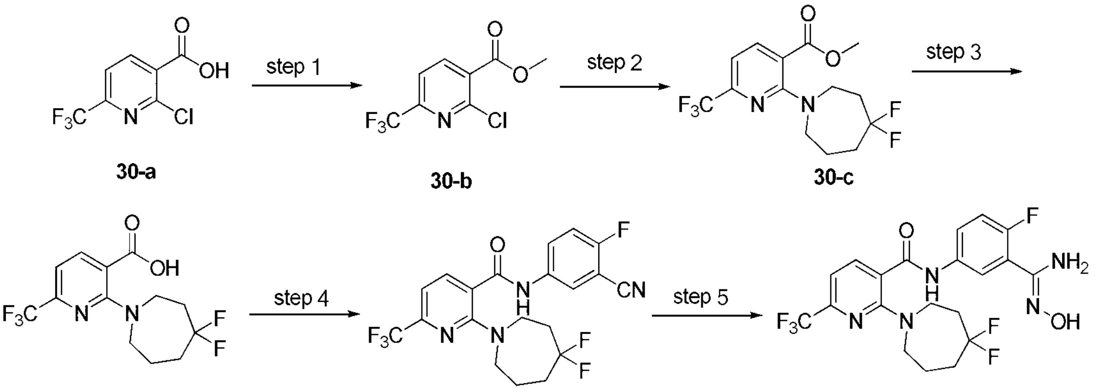


30-a 30-b 30-c

30-d 30-e I -30

Step 1: Under the protection of nitrogen, 2-chloro-6-(trifluoromethyl)nicotinic acid (**30-a**) (1 g, 4.4 mmol) was dissolved in dichloromethane (10 mL), added with a catalytic amount of N,N-dimethylformamide at 0°C, followed by dropwise addition of oxalyl chloride (750 μL, 8.9 mmol). The reaction was allowed to proceed at room temperature until the starting material completely converted to an acid chloride. Methanol (1.8 mL, 44.3 mmol) was then added and the reaction continued until the acid chloride was completely converted to an ester. After the reaction was complete, the mixture was concentrated, adjusted to pH 8-9 by adding saturated sodium bicarbonate solution, and extracted three times with ethyl acetate. The combined organic layer was washed once with saturated brine, dried over anhydrous sodium sulfate, and concentrated in vacuum to remove the organic solvent to obtain a crude product. The crude product was purified by silica gel column (200-300 mesh) chromatography using PE:EA= 10:1 (v/v) to obtain 831 mg of methyl 2-chloro-6-(trifluoromethyl)nicotinate **(30-b)** as a colorless liquid in a yield of 78.2%. $^1$H NMR (400 MHz, $CDCl_3$) δ: 8.33 (d, J = 7.8 Hz, 1H), 7.70 (d, J = 7.8 Hz, 1H), 4.00 (s, 3H); MS (ESI) m/z $[M+H]^+$ = 240.9.

Step 2: Following the process of Step 4 of Example 1, methyl 2-chloro-6-(trifluoromethyl)nicotinate **(30-b)** (825 mg, 3.4 mmol) and 4,4-difluoroazepane hydrochloride **(1-c)** (768 mg, 4.5 mmol) were used as starting materials to obtain 1.007 g of methyl 2-(4,4-difluoroazepan-1-yl)-6-(trifluoromethyl)nicotinate **(30-c)** as a colorless liquid in a yield of 86.4%. $^1$H NMR (400 MHz, $CDCl_3$) δ: 8.00(d, J = 7.8 Hz, 1H), 6.99(d, J = 7.8 Hz, 1H), 3.91(s, 3H), 3.78~3.71(m, 2H), 3.34~3.26(m, 2H), 2.44~2.30(m, 2H), 2.05~1.90(m, 4H); MS(ESI) m/z $[M+H]^+$ = 338.9.

Step 3: Following the process of Step 6 of Example 1, methyl 2-(4,4-difluoroazepan-1-yl)-6-(trifluoromethyl) nicotinate **(30-c)** (1 g, 3.0 mmol) was used as a starting material to obtain 867 mg of 2-(4,4-difluoroazepan-1-yl)-6-(trifluoromethyl)nicotinic acid **(30-d)** as a white solid in a yield of 90.4%. $^1$H NMR (600 MHz, $CDCl_3$) δ: 8.26(d, J = 7.8 Hz, 1H), 7.10(d, J = 7.8 Hz, 1H), 3.78~3.73(m, 2H), 3.40~3.37(m, 2H), 2.45~2.35(m, 2H), 2.08~1.96(m, 4H); MS(ESI) m/z $[M+H]^+$ = 324.9.

Step 4: Following the process of Step 7 of Example 1, 2-(4,4-difluoroazepan-1-yl)-6-(trifluoromethyl)nicotinic acid **(30-d)** (1 g, 3.1 mmol) and 5-amino-2-fluorobenzonitrile (420 mg, 3.1 mmol) were used as starting materials to obtain

80 mg of N-(3-cyano-4-fluorophenyl)-2-(4,4-difluoroazepan-1-yl)-6-(trifluoromethyl)nicotinami de **(30-e)** as a white solid in a yield of 5.9%. $^1$H NMR (400 MHz, DMSO-$d_6$) δ: 10.93(s, 1H), 8.20(dd, J = 5.8, 2.8 Hz, 1H), 8.00~7.90(m, 2H), 7.56 (t, J = 9.0 Hz, 1H), 7.20(d, J = 7.6 Hz, 1H), 3.68~3.61(m, 2H), 3.46(t, J = 5.9 Hz, 2H), 2.38~2.23(m, 2H), 2.04~1.92(m, 2H), 1.89~1.83(m, 2H); MS(ESI) m/z [M+H]$^+$= 442.8.

Step 5: Following the process of Step 8 of Example 1, N-(3-cyano-4-fluorophenyl)-2-(4,4-difluoroazepan-1-yl)-6-(trifluoromethyl)nicotinami de **(30-e)** (60 mg, 0.14 mmol) was used as a starting material to obtain 20 mg of 2-(4,4-difluoroazepan-1-yl)-N-(4-fluoro-3-(N'-hydroxyamidino)phenyl)-6-(trifluoromet hyl)nicotinamide (**I-30**) as a white solid in a yield of 31.0%. $^1$H NMR (500 MHz, Methanol-$d_4$) δ: 7.90 (d, J = 7.6 Hz, 1H), 7.84-7.75 (m, 2H), 7.18 (t, J = 9.5 H z, 1H), 7.11(d, J = 7.6 Hz, 1H), 3.78~3.72(m, 2H), 3.54(t, J = 5.8 Hz, 2H), 2.38~2.29(m, 2H), 2.04~1.91(m, 4H); MS(ESI) m/z [M+H]$^+$ = 476.1.

**Example 31:** Preparation of 2-chloro-6-(4,4-difluoroazepan-1-yl)-N-(1,1-dioxo-2,3-dihydrobenzisothiazol-6-yl)-3-tr ifluoromethylbenzamide (**I-31**)

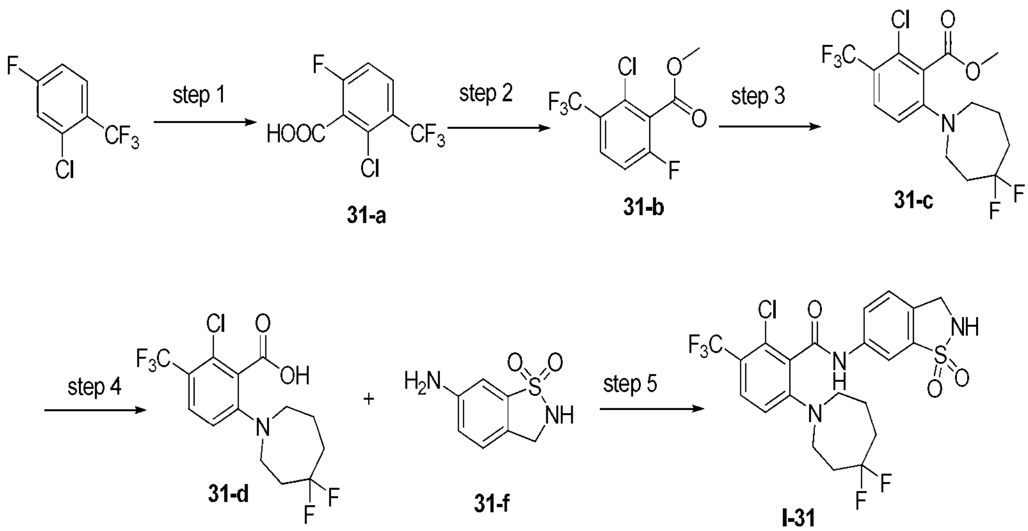


31-a 31-b 31-c 31-d 31-f I-31

Step 1: Under the protection of nitrogen, 2,2,6,6-tetramethylpiperidine (19 g, 135.9 mmol) was dissolved in anhydrous tetrahydrofuran (200 mL), n-BuLi (2.4 M, 57 mL) was added dropwise slowly thereto in an ice bath and reacted for 1 h. The reaction mixture was then cooled to -78°C and slowly added dropwise with 2-chloro-4-fluorobenzotrifluoride (18 g, 91 mmol). After 3 h of reaction, excess dry ice was added and the reaction was continued for 2 h. The mixture was naturally warmed to room temperature, quenched with saturated ammonium chloride, adjusted to approximately pH 2 with diluted hydrochloric acid, and extracted with EA and water. The organic layer was washed with saturated brine, dried over anhydrous sodium sulfate, and then rotatory evaporated to dryness to obtain a crude product. The crude product was then purified by silica gel (200-300 mesh) column chromatography using dichloromethane:methanol = 40-20:1 (v/v) to obtain 10 g of 2-chloro-6-fluoro-3-trifluoromethylbenzoic acid **(31-a)** in a yield of 47%. $^1$H NMR (400 MHz, DMSO-$d_6$) δ 14.60 (s, 1H), 8.03 (dd, J = 8.8, 5.8 Hz, 1H), 7.59 (t, J = 8.6 Hz, 1H); MS (ESI) m/z [M+H]$^+$ = 240.9.

Step 2: Under the protection of nitrogen, 2-Chloro-6-fluoro-3-trifluoromethylbenzoic acid **(31-a)** (8.7 g, 36 mmol) was dissolved in anhydrous dichloromethane in an ice bath, added with a catalytic amount of DMF, and slowly added dropwise with oxalyl chloride (2M, 22 mL). After the addition, the reaction mixture was warmed to room temperature until the starting material was completely converted to an acid chloride. The reaction mixture was rotatory evaporated to dryness, added with methanol (50 mL) and anhydrous triethylamine (11 g, 108 mmol), and reacted at room temperature until complete conversion was achieved. The reaction mixture was rotatory evaporated to dryness to obtain a crude product. The crude product was then purified by silica gel (200-300 mesh) column chromatography using PE/EA =20-10:1 (v/v) to obtain 6.5 g of methyl 2-chloro-6-fluoro-3-trifluoromethylbenzoate (**31-b**) in a yield of 70%. $^1$H NMR (400 MHz, $CDCl_3$) δ 7.77 (dd, J = 8.8, 5.6 Hz, 1H), 7.17 (t, J = 8.4 Hz, 1H), 4.00 (s, 3H); MS (ESI) m/z [M+H]$^+$ = 257.2.

Step 3: Methyl 2-chloro-6-fluoro-3-trifluoromethylbenzoate **(31-b)** (0.5 g, 1.94 mmol) and 4,4-difluoroazepane hydrochloride **(1-c)** (0.49 g, 2.91 mmol) were dissolved in dimethyl sulfoxide, and DIPEA (0.7 g, 5.8 mmol) was added thereto. The reaction mixture was heated to 90°C for overnight, and the resultant was extracted with EA and water. The organic layer was washed with saturated brine, dried over anhydrous sodium sulfate, and rotatory evaporated to dryness to obtain a crude product. The crude product was purified by column chromatography using PE:EA=10:1 (v/v) to obtain 0.2

g of methyl 2-chloro-6-(4,4-difluoroazepin-1-yl)-3-trifluoromethylbenzoate (**31-c**) in a yield of 28%. $^1$H NMR (400 MHz, $CDCl_3$) δ 7.57(d, J = 9.0 Hz, 1H), 6.88(d, J = 9.0 Hz, 1H), 3.95(s, 3H), 3.36(ddd, J = 15.1, 8.9, 4.2 Hz, 4H), 2.29~2.09(m, 4H), 1.91(ddd, J = 12.0, 6.1, 2.5 Hz, 2H); MS(ESI) m/z $[M+H]^+$ = 372.0.

Step 4: Following the process of Step 6 of Example 1, methyl 2-chloro-6-(4,4-difluoroazepan-1-yl)-3-trifluoromethylbenzoate **(31-c)** (690 mg, 1.8 mmol) was used as a starting material to obtain 200 mg of 2-chloro-6-(4,4-difluoroazepan-1-yl)-3-trifluoromethylbenzoic acid **(31-d)** in a yield of 30%. $^1$H NMR (400 MHz, DMSO-$d_6$) δ: 12.05(s, 1H), 7.65(d, J = 9.0 Hz, 1H), 7.06(d, J = 9.0 Hz, 1H), 3.53~3.40(m, 4H), 2.23(dd, J = 15.2, 5.0 Hz, 2H), 2.16~2.06(m, 2H), 1.86(dd, J = 11.8, 6.0 Hz, 2H); MS(ESI) m/z $[M+H]^+$ = 355.9.

Step 5: 2-Chloro-6-(4,4-difluoroazepan-1-yl)-3-trifluoromethylbenzoic acid **(31-d)** (100 mg, 0.28 mmol) was dissolved in anhydrous DCM (5 mL). The reaction solution was placed in an ice bath and a catalytic amount of DMF was added dropwise thereto. Oxalyl chloride (2 M, 0.2 mL) was added under nitrogen and the reaction was allowed to proceed at room temperature. After the starting material was converted completely, excess oxalyl chloride was evaporated to obtain an acid chloride, which was then dissolved in dichloromethane and 6-amino-2,3-dihydrobenzisothiazole-1,1-dioxide **(31-f)** (77 mg, 0.42 mmol) was added at 0°C. The mixture was placed in an ice bath and anhydrous pyridine (3 mL) was added dropwise. After the reaction was complete, ethyl acetate and water were added to separate an extracted organic layer. The organic layer was washed with saturated brine, dried over anhydrous sodium sulfate, and rotatory evaporated to dryness to obtain a crude product. The crude product was purified by silica gel (200-300 mesh) column chromatography using DCM/MeOH = 20-10:1 (v/v) to obtain 54 mg of 2-chloro-6-(4,4-difluoroazepan-1-yl)-N-(1,1-dioxo-2,3-dihydrobenzisothiazol-6-yl)-3-tr ifluoromethylbenzamide (**I-31**) in a yield of 37%. $^1$H NMR (400MHz, DMSO-$d_6$)δ: 11.16(s, 1H), 8.22(s, 1H), 7.91(s, 1H), 7.79(d, J = 8.4 Hz, 1H), 7.74(d, J = 9.0 Hz, 1H), 7.57(d, J = 8.1 Hz, 1H), 7.15(d, J = 9.7 Hz, 1H), 4.38(d, J = 4.5 Hz, 2H), 3.45(m, 4H), 2.18(m, 2H), 2.02(m, 2H), 1.79 (m, 2H); MS(ESI) m/z $[M+H]^+$ =524.0.

**Example 32:** Preparation of 2-chloro-6-(4,4-difluoroazepan-1-yl)-N-(4-fluoro-3-(N'-hydroxyamidino)phenyl)-3-(trif luoromethyl)benzamide (**I-32**)

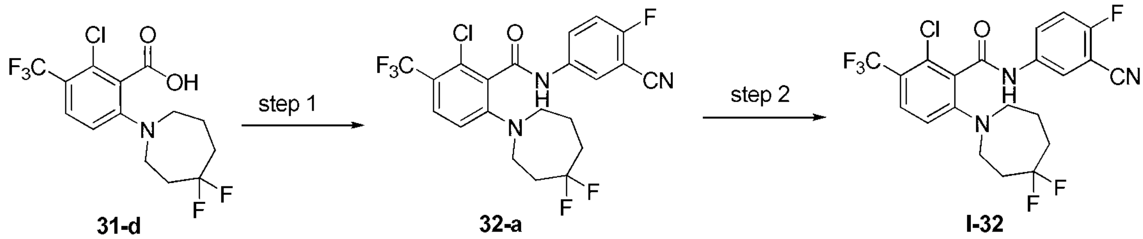


31-d 32-a I-32

Step 1: Following the process of Step 7 of Example 1, 2-chloro-6-(4,4-difluoroazepan-1-yl)-3-trifluoromethylbenzoic acid **(31-d)** (188 mg, 0.53 mmol) and 5-amino-2-fluorobenzonitrile (83 mg, 0.61 mmol) were used as starting materials to obtain 63 mg of 2-chloro-6-(4,4-difluoroazepan-1-yl)-3-trifluoromethylbenzoic acid **(32-a)** in a yield of 25%. $^1$H NMR (400 MHz, $CDCl_3$) δ: 8.02 (dd, J = 5.2, 2.6 Hz, 1H), 7.80 (dd, J = 5.8, 3.2 Hz, 1H), 7.73 (s, 1H), 7.62 (d, J = 8.8Hz, 1H), 6.97 (t, J = 8.2Hz, 1H), 3.43 (t, J = 5.4Hz, 4H), 2.30~2.00 (m, 4H), 1.89 (d, J = 5.4Hz, 2H); MS(ESI) m/z $[M+H]^+$= 476.1.

Step 2: Following the process of Step 8 of Example 1, 2-chloro-N-(3-cyano-4-fluorophenyl)-6-(4,4-difluoroazepan-1-yl)-3-(trifluoromethyl)b enzamide was used as a starting material to obtain 34 mg of 2-chloro-6-(4,4-difluoroazepan-1-yl)-N-(4-fluoro-3-(N'-hydroxyamidino)phenyl)-3-(trif luoromethyl)benzamide (**I-32**) in a yield of 51%. $^1$H NMR (400MHz, DMSO-$d_6$) δ: 10.82(s, 1H), 9.67(s, 1H), 7.85(dd, J = 6.4, 2.8 Hz, 1H), 7.69(dd, J = 11.8, 6.6 Hz, 2H), 7.29~7.18 (m, 1H), 7.12(d, J = 9.0 Hz, 1H), 5.85(s, 2H), 3.46(dd, J = 15.0, 8.4 Hz, 4H), 2.18(s, 2H), 2.03(s, 2H), 1.80(d, J = 5.8 Hz, 2H); MS(ESI) m/z $[M+H]^+$= 509.1.

**Example 33:** Preparation of 5-chloro-2-(4,4-difluoroazepan-1-yl)-N-(4-fluoro-3-(N'-hydroxyamidino)phenyl)-4-(trif luoromethyl)benzamide (**I-33**)

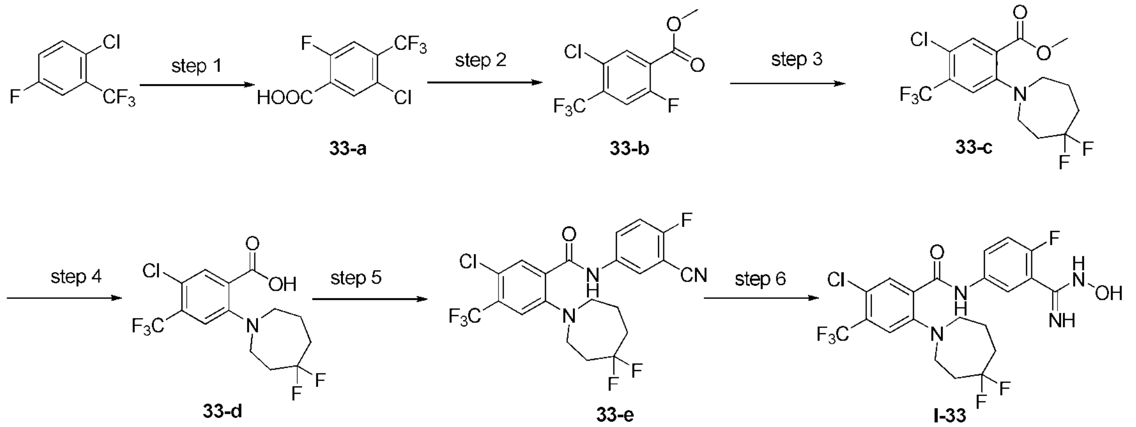

33-a 33-b 33-c 33-d 33-e I-33

Step 1: Following the process of Step 1 of Example 31, 2-chloro-5-fluorobenzotrifluoride (15 g, 75 mmol) was used as a starting material to obtain 10 g of 5-chloro-2-fluoro-4-(trifluoromethyl)benzoic acid **(33-a)** in a yield of 55%. $^1$H NMR (400 MHz, DMSO) δ: 14.35 (s, 1H), 7.88 (dd, *J* = 9.0, 5.0 Hz, 1H), 7.76 (t, *J* = 8.8 Hz, 1H); MS (ESI) m/z [M-H]-=240.8.

Step 2: Following the process of Step 2 of Example 31, 5-chloro-2-fluoro-4-(trifluoromethyl)benzoic acid **(33-a)** was used as a starting material to obtain 1.7 g of methyl 5-chloro-2-fluoro-4-(trifluoromethyl)benzoate (**33-b**) in a yield of 32%. $^1$H NMR (400 MHz, $CDCl_3$) δ: 8.09 (d, J = 6.0 Hz, 1H), 7.53 (d, J = 10.0 Hz, 1H), 4.00 (s, 3H); MS (ESI) m/z $[M+H]^+$ = 257.3.

Step 3: Methyl 5-chloro-2-fluoro-4-(trifluoromethyl)benzoate **(33-b)** (1.7 g, 6.9 mmol) and 4,4-difluoroazepane hydrochloride (1-c) (1.7 g, 10.3 mmol) were dissolved in dimethyl sulfoxide. DIPEA (2.6 g, 20.7 mmol) was added and the reaction system was heated to 90°C until the main starting material was reacted completely. The resultant was extracted with EA and water. The organic layer was washed with saturated brine and dried over anhydrous sodium sulfate, and the resultant was purified by silica gel (200-300 mesh) column chromatography using PE:EA = 20-10:1 (v/v) to obtain 0.5 g of methyl 5-chloro-2-(4,4-difluoroazepine-1-yl)-4-(trifluoromethyl)benzoate **(33-c)** in a yield of 21%. $^1$H NMR (400 MHz, $CDCl_3$) δ 7.70 (s, 1H), 7.28 (s, 1H), 3.94 (s, 3H), 3.43~3.38 (m, 2H), 3.36~3.32 (m, 2H), 2.37~2.25 (m, 2H), 2.17 (m, 2H), 1.95 (dt, J = 11.8, 6.0Hz, 2H); MS(ESI) m/z $[M+H]^+$= 372.1.

Step 4: Following the process of Step 6 of Example 1, methyl 5-chloro-2-(4,4-difluoroazepan-1-yl)-4-(trifluoromethyl)benzoate **(33-c)** (0.5 g, 1.4 mmol) was used as a starting material to obtain 0.5 g of 5-chloro-2-(4,4-difluoroazepine-1-yl)-4-(trifluoromethyl)benzoic acid **(33-d)** in a yield of 99%. $^1$H NMR (400 MHz, DMSO-$d_6$) δ: 13.78(s, 1H), 7.71(s, 1H), 7.35(s, 1H), 3.40 (d, *J* = 5.2 Hz, 2H), 3.35~3.33(m, 2H), 2.38~2.21(m, 2H), 2.12(m, 2H), 1.88~1.79(m, 2H); MS(ESI) m/z $[M+H]^+$= 355.8.

Step 5: Following the process of Step 7 of Example 1, 5-chloro-2-(4,4-difluoroazepine-1-yl)-4-(trifluoromethyl) benzoic acid **(33-d)** (0.2 g, 0.54 mmol) and 5-amino-2-fluorobenzonitrile (74 mg, 0.54 mmol) were used as starting materials to obtain 124 mg of 5-chloro-N-(3-cyano-4-fluorophenyl)-2-(4,4-difluoroazepan-1-yl)-4-(trifluoromethyl)b enzamide **(33-e)** in a yield of 48%. $^1$H NMR (400 MHz, DMSO-$d_6$) δ: 11.84 (s, 1H), 8.36 (s, 1H), 8.23~8.10 (m, 1H), 7.74 (d, *J* = 4.4 Hz, 1H), 7.59 (s, 1H), 3.38~3.29 (m, 2H), 3.24 (d, *J* = 4.4 Hz, 2H), 2.48 (d, *J* = 5.4 Hz, 2H), 2.40~2.24 (m, 2H), 1.97 (s, 2H). MS(ESI) m/z $[M+H]^+$=476.2.

Step 6: Following the process of Step 8 of Example 1, 5-chloro-N-(3-cyano-4-fluorophenyl)-2-(4,4-difluoroazepan-1-yl)-4-(trifluoromethyl)b enzamide **(33-e)** (124 mg, 0.26 mmol) was used as a starting material to obtain 46 mg of 5-chloro-2-(4,4-difluoroazepan-1-yl)-N-(4-fluoro-3-(N'-hydroxyamidino)phenyl)-4-(trif luoromethyl)benzamide (**I-33**) in a yield of 35%. $^1$H NMR (400 MHz, DMSO-$d_6$) δ: 10.66 (s, 1H), 9.6(s, 1H), 7.87(dd, *J* = 6.2, 2.4 Hz, 1H), 7.81~7.69(m, 1H), 7.66(s, 1H), 7.33(s, 1H), 7.26(t, *J* = 9.6 Hz, 1H), 5.87(s, 2H), 3.39(d, *J* = 8.0 Hz, 4H), 2.24(s, 2H), 2.12~2.01(m, 2H), 1.81 (s, 2H); MS(ESI) m/z $[M+H]^+$ = 509.2.

**Example 34:** Preparation of 5-chloro-2-(4,4-difluoroazepan-1-yl)-N-(4-fluoro-3-(sulfamoylamino)phenyl)-6-methyl nicotinamide (**I-34**)

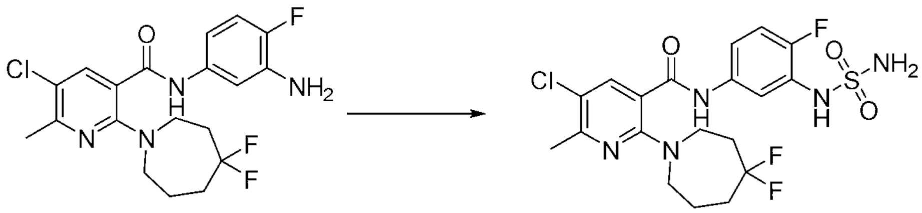

19-b

I-34

N-(3-amino-4-fluorophenyl)-5-chloro-2-(4,4-difluoroazepan-1-yl)-6-methylnicotinami de **(19-b)** (139 mg, 0.34 mmol) prepared in step 2 of Example 19 was dissolved in anhydrous tetrahydrofuran (20 mL). Triethylamine (0.18 mL, 1.35 mmol) was then added and the mixture was cooled to approximately 0°C. Chlorosulfonamide (77.8 mg, 0.67 mmol) was then added and the mixture was allowed to react under reflux until the starting material was completely consumed. The reaction mixture was washed with 1N hydrochloric acid, and extracted with ethyl acetate to obtain an ethyl acetate phase. The organic layer was washed once with saturated brine, dried over anhydrous sodium sulfate, and concentrated in vacuum to remove the organic solvent to obtain a crude product. The crude product was then purified by silica gel (200-300 mesh) column chromatography using dichloromethane:methanol = 40-4:1 (v/v) to obtain 45 mg of 5-chloro-2-(4,4-difluoroazepan-1-yl)-N-(4-fluoro-3-(sulfamoylamino)phenyl)-6-methyl nicotinamide (**I-34**) in a yield of 27%. $^1H$ NMR (400MHz, DMSO-$d_6$) δ: 10.47(s, 1H), 9.09(s, 1H), 7.76(dd, *J* =7.2, 2.4 Hz, 1H), 7.69(s, 1H), 7.48~7.52(q, 1H), 7.19 (t, *J* = 9.2 Hz, 1H), 7.05(s, 2H), 3.60(sq, 2H), 3.41(t, *J* = 6.0 Hz, 2H), 2.44(s, 3H), 2.27~2.35(sq, 2H), 1.95~2.00(sq, 2H), 1.84(sq, 2H); MS(ESI) m/z $[M+H]^+$ = 492.1, 493.9(3:1).

**Example 35:** Preparation of 5-chloro-2-(4,4-difluoroazepan-1-yl)-6-methyl-N-(3-(sulfamoylamino)phenyl)nicotina mide (**I-35**)

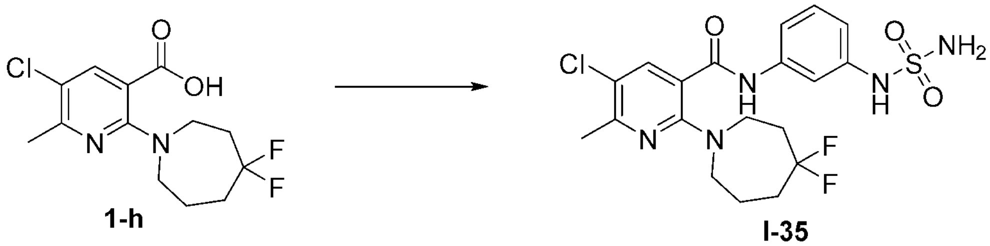

1-h

I-35

5-chloro-2-(4,4-difluoroazepan-1-yl)-6-methylnicotinic acid **(1-h)** (150 mg, 0.5 mmol) prepared in step 6 of Example 1 was dissolved in anhydrous toluene (10 mL), added with thionyl chloride (2.0 mL) and reacted under reflux until the starting material completely converted an acid chloride. The reaction mixture was concentrated to remove thionyl chloride and toluene to obtain a crude acid chloride. The crude acid chloride was then dissolved in anhydrous dichloromethane (10 mL), cooled to 0°C, slowly added with N-(3-aminophenyl)sulfamoylamide (93.0 mg, 0.5 mmol) and pyridine (2 mL) and reacted at room temperature until the reaction was complete. The reaction mixture was washed with 1N hydrochloric acid, and extracted with dichloromethane to obtain a dichloromethane phase. The organic layer was washed once with saturated brine, dried over anhydrous sodium sulfate, and concentrated in vacuum to remove the organic solvent to obtain a crude product. The crude product was then purified by silica gel (200-300 mesh) column chromatography using dichloromethane:methanol =40-10:1 (v/v) to obtain 30 mg of 5-chloro-2-(4,4-difluoroazepan-1-yl)-6-methyl-N-(3-(sulfamoylamino)phenyl)nicotina mide (**I-35**) as a solid in a yield of 12.8%. $^1H$ NMR (400 MHz, DMSO-$d_6$) δ: 10.39(s, 1H),9.51(s, 1H), 7.66(s, 1H), 7.46(s, 1H), 7.29(d, *J* = 7.6 Hz, 1H), 7.20(t, *J* = 8.0 Hz, 1H), 7.03(s, 2H), 6.95(d, *J* = 7.6 Hz, 1H), 3.59(s, 2H), 3.40(t, *J* = 6.4 Hz, 2H), 2.42(s, 3H), 2.31(brs, 2H), 1.96(brs, 2H), 1.81(brs, 2H); MS(ESI) m/z $[M+H]^+$ = 473.7, 475.7(3:1).

**Example 36:** Preparation of 5-chloro-2-(4,4-difluoroazepan-1-yl)-N-(4-fluoro-3-(N'-hydroxyamidino)phenyl)-6-(trif luoromethyl)nicotinamide (I-36)

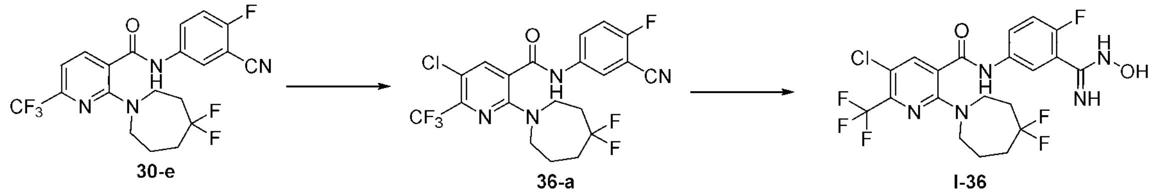

30-e 36-a I-36

Step 1: Following the process of Step 5 of Example 1, N-(3-cyano-4-fluorophenyl)-2-(4,4-difluoroazepan-1-yl)-6-(trifluoromethyl)nicotinami de **(30-e)** (152 mg, 0.32 mmol) was used as a starting material to obtain 120 mg of 5-chloro-N-(3-cyano-4-fluorophenyl)-2-(4,4-difluoroazepan-1-yl)-6-(trifluoromethyl)ni cotinamide **(36-a)** in a yield of 74%. $^1$H NMR (400 MHz, DMSO-$d_6$) δ: 10.98(s, 1H), 8.18(dd, J = 5.8, 2.6Hz, 1H), 8.15(s, 1H), 7.98~7.92(m, 1H), 7.58(t, J = 9.2 Hz, 1H), 3.65(s, 2H), 3.45(t, J = 5.6 Hz, 2H), 2.31(s, 2H), 2.00(s, 2H), 1.87 (d, J = 5.4 Hz, 2H); MS(ESI) m/z $[M+H]^+$ = 477.2.

Step 2: Following the process of Step 8 of Example 1, 5-chloro-N-(3-cyano-4-fluorophenyl)-2-(4,4-difluoroazepan-1-yl)-6-(trifluoromethyl)ni cotinamide **(36-a)** (120 mg, 0.25 mmol) was used as a starting material to obtain 54 mg of 5-chloro-2-(4,4-difluoroazepan-1-yl)-N-(4-fluoro-3-(N'-hydroxyamidino)Phenyl)-6-(trif luoromethyl)nicotinamide (**I-36**) in a yield of 42%. $^1$H NMR (400 MHz, DMSO-$d_6$) δ: 10.72(s, 1H), 9.68(s, 1H), 8.10(s, 1H), 7.84(dd, *J* = 6.4, 2.8 Hz, 1H), 7.75~7.67(m, 1H), 7.30~7.20(m, 1H), 5.84 (s, 2H), 3.65(s, 2H), 3.47(t, *J* = 5.8 Hz, 2H), 2.31(s, 2H), 2.00(d, *J* = 7.2 Hz, 2H), 1.87 (d, *J* = 5.8 Hz, 2H); MS(ESI) m/z $[M+H]^+$ = 510.2.

**Example 37:** Preparation of 5-chloro-N-(3-(N'-cyanoamidino)-4-fluorophenyl)-2-(4,4-difluoroazepan-1-yl)-6-triflu oromethylnicotinamide (**I-37**)

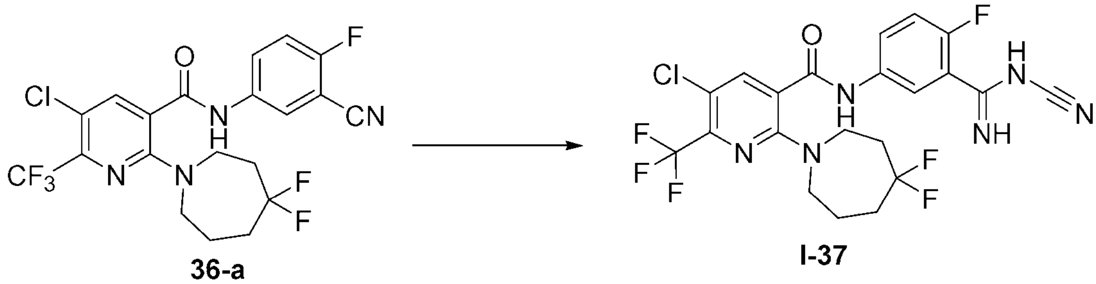

36-a I-37

Following the process of Example 3, 5-chloro-N-(3-cyano-4-fluorophenyl)-2-(4,4-difluoroazepan-1-yl)-6-(trifluoromethyl)ni cotinamide **(36-a)** (135 mg, 0.28 mmol) obtained in step 1 of Example 36 was used as a starting material to obtain 15 mg of 5-chloro-N-(3-(N'-cyanoamidino)-4-fluorophenyl)-2-(4,4-difluoroazepan-1-yl)-6-triflu oromethylnicotinamide (**I-37**) in a yield of 10.2%. $^1$H NMR (400 MHz, DMSO-$d_6$) 6: 10.86(brs, 1H), 9.04(brs, 1H), 8.85(brs, 1H), 8.11(s, 1H), 7.83~7.96(brs, 2H), 7.42(s, 1H), 3.65(brs, 2H), 3.46(brs, 2H), 2.28~2.34(t, 2H), 1.96~2.04(m, 2H), 1.86~1.90(m, 2H); MS(ESI) m/z $[M+H]^+$ = 519.1, 521.1(3:1).

**Example 38:** Preparation of 5-chloro-2-(4,4-difluoroazepan-1-yl)-6-(difluoromethyl)-N-(4-fluoro-3-(N'-hydroxyami dino)phenyl)nicotinamide (**I-38**)

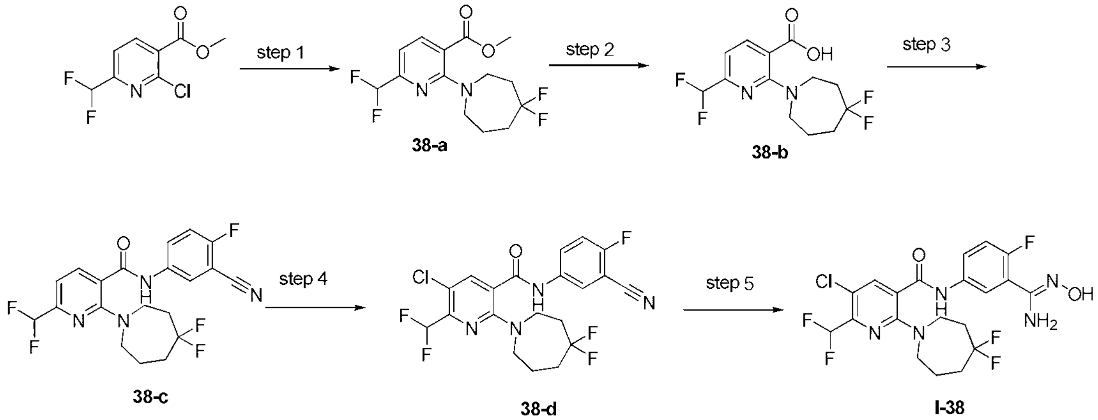


38-a 38-b 38-c 38-d I-38

Step 1: Following the process of Step 4 of Example 1, methyl 2-chloro-6-difluoromethylnicotinate (368 mg, 16.6 mmol) and **1-c** (428.0 mg, 24.9 mmol) were used as starting materials to obtain 320 mg of methyl 2-(4,4-difluoroazepan-1-yl)-6-difluoromethylnicotinate **(38-a)** in a yield of 60%. $^1$H NMR (400 MHz, DMSO-$d_6$) δ: 8.01(d, *J* = 7.6 Hz, 1H), 6.99(d, *J* = 7.6 Hz, 1H), 6.79(t, 1H, CHF2), 3.83(s, 3H), 3.62(sq, 2H), 3.28(t, *J* = 6.0 Hz, 2H), 2.27~2.37(tq, 2H), 1.87~2.01(dq, 4H); MS(ESI) m/z $[M+H]^+$ = 321.1.

Step 2: Following the process of Step 6 of Example 1, methyl 2-(4,4-difluorocycloheptane-1-yl)-6-difluoromethylnicotinate **(38-a)** (320 mg, 1.0 mmol) was used as a starting material to obtain 276 mg of 2-(4,4-difluoroazepan-1-yl)-6-(difluoromethyl)nicotinic acid **(38-b)** in a yield of 90%. The product was directly used in the next step. MS (ESI) m/z $[M+H]^+$ = 307.1.

Step 3: Following the process of Step 7 of Example 1, 2-(4,4-difluoroazepan-1-yl)-6-(difluoromethyl)nicotinic acid **(38-b)** (120 mg, 0.39 mmol) was used as a starting material to obtain 160 mg of N-(3-cyano-4-fluorophenyl)-2-(4,4-difluorocycloheptane-1-yl)-6-difluoromethylnicoti namide **(38-c)** in a yield of 96.3%. $^1$H NMR (400 MHz, DMSO-$d_6$) δ: 10.86(s, 1H), 8.19~8.21(q, *J* = 2.8 Hz, 1H), 7.91~7.97(q, 1H), 7.90(d, *J* = 7.6 Hz, 1H), 7.56(t, *J* = 9.2 Hz, 1H), 7.03(d, *J* = 7.6 Hz, 1H), 6.89(t, 1H, $CHF_2$), 3.64(sq, 2H), 4.33(t, *J* = 6.0 Hz, 2H), 2.33(t, 2H), 1.92~2.03(m, 2H), 1.85(m, 2H); MS(ESI) m/z $[M+H]^+$ = 425.1.

Step 4: Following the process of Step 5 of Example 1, N-(3-cyano-4-fluorophenyl)-2-(4,4-difluoroazacycloheptan-1-yl)-6-difluoromethylnico tinamide **(38-c)** (100 mg, 0.24 mmol) was used as a starting material to obtain 100 mg of 5-chloro-N-(3-cyano-4-fluorophenyl)-2-(4,4-difluoroazacycloheptan-1-yl)-6-difluorom ethylnicotinamide **(38-d)** in a yield of 92.5%. $^1$H NMR (400 MHz, DMSO-$d_6$) δ: 10.92(s, 1H), 8.17~8.19(q, 1H), 8.03(s, 1H), 7.93~7.97(m, 1H), 7.58(t, *J* = 9.2 Hz, 1H), 7.06(t, 1H, CHF2), 3.64~3.66(sq, 2H), 3.44(t, *J* = 6.0 Hz, 2H), 2.28~2.37(t, *J* = 6.0 Hz, 2H), 1.98~2.02(q, 2H), 1.85~1.88(q, 2H); MS(ESI) m/z $[M+H]^+$ = 459.1, 461.0 (3:1).

Step 5: 5-Chloro-N-(3-cyano-4-fluorophenyl)-2-(4,4-difluorocycloheptane-1-yl)-6-difluoromet hylnicotinamide **(38-d)** (70.0 mg, 0.15 mmol) and a 40% aqueous hydroxylamine solution (10 eq) were added to dimethyl sulfoxide and reacted overnight at room temperature. After the reaction was complete, the reaction system was added with water, extracted with ethyl acetate, and concentrated to obtain a crude product. The crude product was purified by silica gel (200-300 mesh) column chromatography using DCM:MeOH = 40:1 (v/v) to obtain 70 mg of 5-chloro-2-(4,4-difluoroazepan-1-yl)-6-difluoromethyl-N-(4-fluoro-3-(N'-hydroxyamidi no)phenyl)nicotinamide (**I-38**) in a yield of 93.3%. $^1$H NMR (400 MHz, DMSO-$d_6$) δ: 10.65(s, 1H), 9.65(s, 1H), 7.96(s, 1H), 7.82~7.85(q, 1H), 7.68~7.72(m, 1H), 7.24(t, *J* = 9.6 Hz,1H), 7.05(t, 1H, CHF2), 5.81(s, 2H), 3.65(sq, 2H), 3.46(t, 2H), 2.30~2.33(m, 2H), 1.98~2.03(m, 2H), 1.84~1.87(m, 2H); MS(ESI) m/z $[M+H]^+$ = 492.1, 494.1(3:1).

**Example 39:** Preparation of N-(3-amidino-4-fluorophenyl)-5-chloro-2-(4,4-difluorocycloheptan-1-yl)-6-difluorome thylnicotinamide (**I-39**)

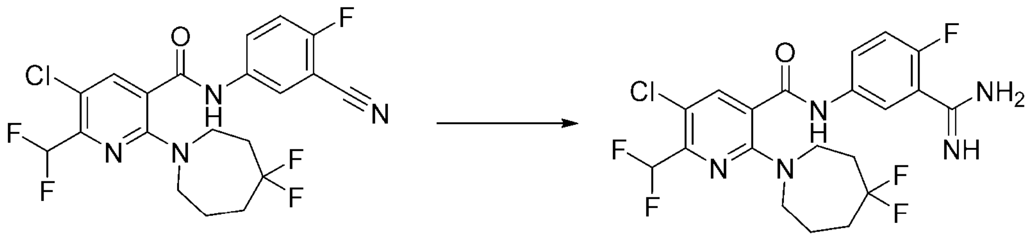

38-d

I-39

Following the process of Example 12, 5-chloro-N-(3-cyano-4-fluorophenyl)-2-(4,4-difluorocycloheptan-1-yl)-6-difluorometh ylnicotinamide **(38-d)** (560 mg, 1.22 mmol) was used as a starting material to obtain 260.0 mg of N-(3-amidino-4-fluorophenyl)-5-chloro-2-(4,4-difluorocycloheptan-1-yl)-6-difluorome thylnicotinamide (**I-39**) in a yield of 44.7%. $^1$H NMR (400 MHz, DMSO-$d_6$) δ: 10.94(s, 1H), 9.38(brs, 3H), 8.01~03(sq, 1H), 7.92(s, 1H), 7.88~7.92(sq, 1H),7.52(t, J = 9.6 Hz, 1H), 7.07(t, 1H, CHF2), 3.66(q, 2H), 3.46(t, 2H), 2.33(t, 2H), 2.02(m, 2H), 1.87(m, 2H); MS(ESI) m/z [M+H]$^+$ = 475.8, 477.8(3:1).

**Example 40:** Preparation and resolution of 5-chloro-2-(4,4-difluoro-3-methylpiperidin-1-yl)-N-(4-fluoro-3-(N'-hydroxyamidino)ph enyl)-6-(trifluoromethyl)nicotinamide (**I-40**) and chiral Isomers (**I-40-P1**) and (**I-40-P2**)

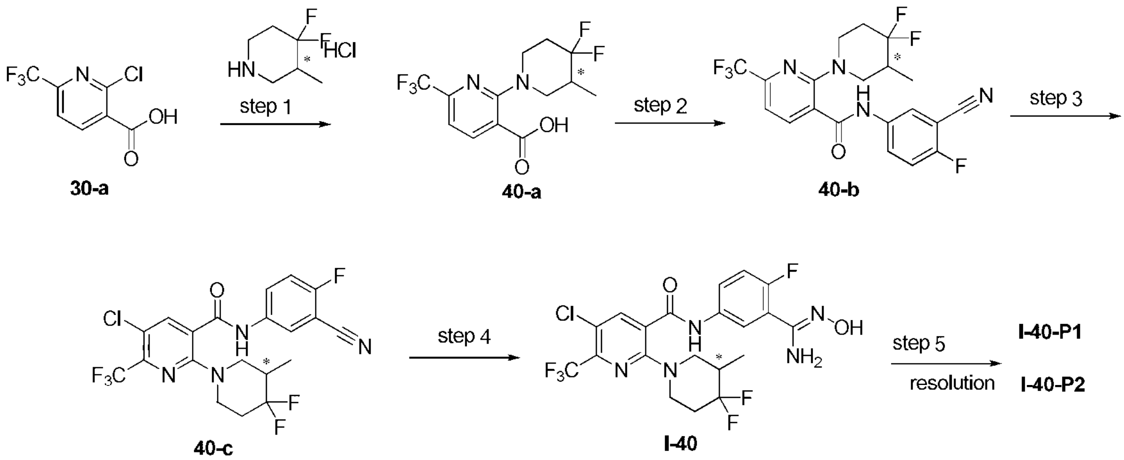


30-a 40-a 40-b

40-c I-40 I-40-P1 I-40-P2

Step 1: 2-Chloro-6-trifluoromethylnicotinic acid **(30-a)** (450.0 mg, 2.0 mmol), racemic 4,4-difluoro-3-methylpiperidine hydrochloride (446.0 mg, 2.6 mmol), and potassium carbonate (830.0 mg, 6.0 mmol) were added to N,N-dimethylformamide (20.0 mmol), and reacted at 90°C until the main starting material disappeared. The mixture was cooled to room temperature, added with water, and extracted with ethyl acetate. The organic layer was dried and concentrated to obtain 600.0 mg of crude 2-(4,4-difluoro-3-methylpiperidin-1-yl)-6-(trifluoromethyl)nicotinic acid **(40-a),** which was used directly in the next step. MS (ESI) m/z [M+H]$^+$ = 325.1.

Step 2: Following the process of Step 7 of Example 1, 2-(4,4-difluoro-3-methylpiperidin-1-yl)-6-(trifluoromethyl) nicotinic acid **(40-a)** (500.0 mg, 0.93 mmol) was used as a starting material to obtain 220.0 mg of N-(3-cyano-4-fluorophenyl)-2-(4,4-difluoro-3-methylpiperidin-1-yl)-6-methylnicotina mide **(40-b)** in a yield of 21.9%. $^1$H NMR (400 MHz, DMSO-$d_6$)δ: 10.74(brs, 1H), 9.67(brs, 1H), 8.14(s, 1H), 7.85~7.87(sm, 1H), 7.68~7.72(sm, 1H), 7.27(t, *J* = 10.0 Hz, 1H), 5.83(brs, 2H), 3.90(d, *J* = 13.6 Hz,1H), 3.75(d,J = 13.6 Hz, 1H), 3.22(t, *J* = 10.8 Hz, 1H), 3.02(t, *J* = 10.8 Hz, 1H), 2.12~2.18(m, 2H), 1.98~2.01(m, 1H), 0.88(sd, 3H); MS(ESI) m/z [M+H]$^+$ = 443.2.

Step 3: Following the process of Step 5 of Example 1, N-(3-cyano-4-fluorophenyl)-2-(4,4-difluoro-3-methylpiperidin-1-yl)-6-methylnicotina mide **(40-b)** (200.0 mg, 4.5 mmol) was used as a starting material to obtain 120.0 mg of 5-chloro-N-(3-cyano-4-fluorophenyl)-2-(4,4-difluoro-3-methylpiperidin-1-yl)-6-(trifluo romethyl)nicotinamide **(40-c)** in a yield 55.7%. $^1$H NMR (400 MHz, DMSO-$d_6$) δ: 11.02 (s, 1H), 8.20 (m, 1H), 8.19 (s, 1H), 7.93-7.97 (m, 1H), 7.59 (t, J = 9.2 Hz, 1H), 3.82~3.87(d, J = 14.0 Hz, 1H), 3.75(d, J = 14.0 Hz, 1H), 3.22(td, J = 13.6, 3.6 Hz, 1H), 3.03(dd, J = 13.6, 3.6 Hz, 1H), 2.11~2.18(m, 2H), 1.85~2.02(m, 2H), 0.90(s, 1.5H), 0.89(s, 1.5H); MS(ESI) m/z [M+H]$^+$ = 477.2, 479.1 (3:1).

Step 4: Following the process of Step 5 of Example 38, 5-chloro-N-(3-cyano-4-fluorophenyl)-2-(4,4-difluoro-3-methylpiperidin-1-yl)-6-(trifluo romethyl)nicotinamide **(40-c)** (100.0 mg, 0.21 mmol) was used as a starting material to

obtain 100.0 mg of 5-chloro-2-(4,4-difluoro-3-methylpiperidin-1-yl)-N-(4-fluoro-3-(N'-hydroxyamidino)ph enyl)-6-(trifluoromethyl)nicotinamide (**I-40**) in a yield of 92%. $^1$H NMR (400 MHz, DMSO-$d_6$) δ: 10.74 (brs, 1H), 9.67 (brs, 1H), 8.14 (s, 1H), 7.85~7.87(sm, 1H), 7.68~7.72(sm, 1H), 7.27(t, J = 10.0 Hz, 1H), 5.83(brs, 2H), 3.90(d, J =13.6 Hz, 1H), 3.75(d, J = 13.6 Hz, 1H), 3.22(t, J = 10.8 Hz, 1H), 3.02(t, J = 10.8 Hz, 1H), 2.12~2.18(m, 2H), 1.98~2.01(m, 1H), 0.88(sd, 3H); MS(ESI) m/z $[M+H]^+$ = 510.2, 512.1 (3:1).

Step 5: Chiral resolution of 5-chloro-2-(4,4-difluoro-3-methylpiperidin-1-yl)-N-(4-fluoro-3-(N'-hydroxyamidino) ph enyl)-6-(trifluoromethyl)nicotinamide (**I-40**)

**I-40** has the following chiral isomers:

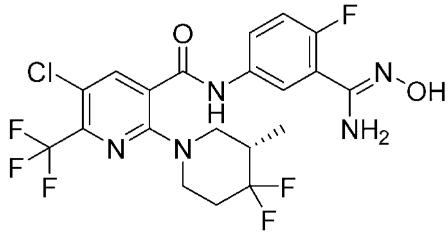
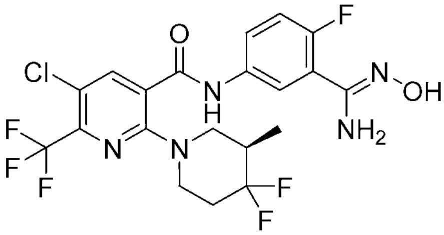
.

**Preparation conditions:** [Instrument: Berger SFC Multigram II (Mettler-Toledo International Inc.); Column: Superchiral R-OJ (Chiralway Biotech Co., Ltd.), 2.1 cm ID * 25 cm Length, 5 um; Column temperature: 40°C; Mobile phase: $CO_2$/EtOH/DEA = 85/15/0.05 (v/v/v); Detection wavelength: 214 nm; Flow rate: 30 ml/min].

**Analytical conditions:** [Instrument: Berger analytical SFC (Mettler-Toledo International Inc.); Column: Superchiral R-OJ (Chiralway Biotech Co., Ltd.), 0.46 cm ID * 15 cm Length, 5 μm; Column temperature: 40°C; Mobile phase: $CO_2$/EtOH/DEA = 85/15/0.05 (v/v/v); Detection wavelength: 214 nm; Flow rate: 3.0 ml/min].

**Specific rotation test conditions:** [Instrument: SGW-1 (Shanghai INESA Physico-Optical Instrument Co., Ltd.); Temperature: 20°C; Wavelength: 589.4 nm; Solvent: Methanol].

Compound **I-40** was resolved by the chiral preparative separation column to obtain **I-40-P1** (peak elution time 1.755 min, ee value 98%) and **I-40-P2** (peak elution time 2.042 min, ee value 98%).

**Example 41:** Preparation and resolution of 5-chloro-2-(4,4-difluoro-3-methylpiperidin-1-yl)-N-(4-fluoro-3-(N'-hydroxyamidino)ph enyl)-6-(methyl)nicotinamide (**I-41**) and Chiral Isomers (**I-41-P1**) and (**I-41-P2**)

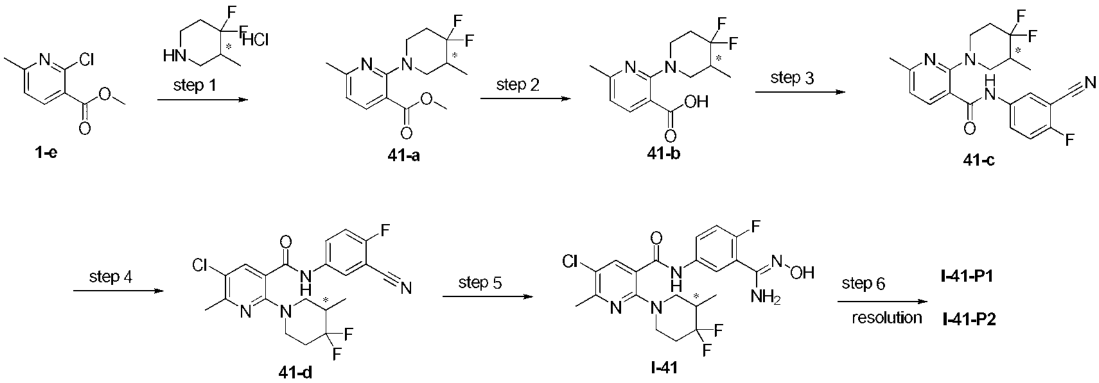

1-e
41-a
41-b
41-c
41-d
I-41
I-41-P1
I-41-P2

Step 1: Following the process of Step 4 of Example 1, **1-e** (441.0 mg, 2.0 mmol) and racemic 4,4-difluoro-3-methylpiperidine hydrochloride (375.7 mg, 2.2 mmol) were used as starting materials to obtain 300.0 mg of methyl 2-(4,4-difluoro-3-methylpiperidin-1-yl)-6-methylnicotinate **(41-a)** in a yield of 35%. $^1$H NMR (400 MHz, DMSO-$d_6$) δ: 7.89(d, J = 7.6 Hz, 1H), 6.75(d, J = 7.6 Hz, 1H), 3.80(s, 3H), 3.62~3.72(m, 2H), 3.08(t, J = 11.2 Hz, 1H), 2.87(t, J = 11.2 Hz, 1H), 2.37(s, 3H); MS(ESI) m/z $[M+H]^+$ = 285.2.

Step 2: Following the process of Step 6 of Example 1, methyl 2-(4,4-difluoro-3-methylpiperidin-1-yl)-6-methylnicotinate **(41-a)** (280.0 mg, 1.0 mmol) was used as a starting material to obtain 250.0 mg of 2-(4,4-difluoro-3-methylpiperidin-1-yl)-6-methylnicotinic acid **(41-b)** in a yield 92.5%. MS (ESI) m/z $[M+H]^+$ = 271.2.

Step 3: Following the process of Step 7 of Example 1, methyl 2-(4,4-difluoro-3-methylpiperidin-1-yl)-6-methylnicotinate **(41-b)** (250.0 mg, 0.93 mmol) and 5-amino-2-fluorobenzonitrile (150 mg, 1.1 mmol) were used as starting materials to obtain 150.0 mg of N-(3-cyano-4-fluorophenyl)-2-(4,4-difluoro-3-methylpiperidin-1-yl)-6-methylnicotina mide

**(41-c)** in a yield of 20.8%. $^1H$ NMR (400 MHz, DMSO-*$d_6$*) δ: 10.70(s, 1H), 8.27(s, 1H), 7.99(s, 1H), 7.74(d, J = 7.6 Hz, 1H), 7.56(t, J = 8.8 Hz, 1H), 6.86(d, J = 7.6 Hz, 1H), 3.65~3.74(m, 2H), 3.10(t, J = 10.4 Hz, 1H), 2.87(t, J = 10.4 Hz, 1H), 2.41(s, 3H), 2.09~2.18(m, 2H), 1.88~2.01(m, 2H), 0.91(s, 1.5H), 0.90 (s, 1.5H); MS (ESI) m/z $[M+H]^+$ = 389.2.

Step 4: N-(3-cyano-4-fluorophenyl)-2-(4,4-difluoro-3-methylpiperidin-1-yl)-6-methylnicotina mide **(41-c)** (140.0 mg, 3.6 mmol) and N-chlorosuccinimide (62.3 mg, 4.7 mmol) as starting materials were reacted in acetonitrile to obtain 150.0 mg of 5-chloro-N-(3-cyano-4-fluorophenyl)-2-(4,4-difluoro-3-methylpiperidin-1-yl)-6-methyl nicotinamide **(41-d)** in a yield of 98.4%. $^1H$ NMR (400 MHz, DMSO-*$d_6$*) δ: 10.78(brs, 1H), 8.21(sq, J = 6.0, 2.8 Hz, 1H), 7.96(sm, J = 6.0, 2.8 Hz, 1H), 7.85(s, 1H), 7.57(t, J = 9.2 Hz, 1H), 3.65~4.75(m, 2H), 3.09~3.16(td, J = 11.6, 2.4 Hz, 1H), 2.89(t, J = 10.8 Hz, 1H), 2.47(s, 3H), 2.05~2.15(m, 2H), 1.95~2.02(m, 1H), δ 1.84-1.91 (m, 1H), 0.91 (s, 1.5H), 0.89 (s, 1.5H); MS (ESI) m/z $[M+H]^+$ = 423.2, 425.2 (3:1).

Step 5: Following the process of Step 5 of Example 38, 5-chloro-N-(3-cyano-4-fluorophenyl)-2-(4,4-difluoro-3-methylpiperidin-1-yl)-6-methyl nicotinamide **(41-d)** (125.0 mg, 0.3 mmol) was used as a starting material to obtain 120.0 mg of 5-chloro-2-(4,4-difluoro-3-methylpiperidin-1-yl)-N-(4-fluoro-3-(N'-hydroxyamidino)ph enyl)-6-methylnicotinamide (**I-41**) in a yield of 86%. $^1H$ NMR (400 MHz, DMSO-*$d_6$*) δ:10.54(brs, 1H), 9.66(s, 1H), 7.88(dd, J = 6.4, 2.8 Hz, 1H), 7.81(s, 1H), 7.69~7.73(m, 1H), 7.25(t, J = 9.6 Hz, 1H), 5.82(brs, 2H), 3.77(d, J = 13.6 Hz, 1H), 3.68(dd, J = 13.6 Hz, 1H), 3.12(t, J = 10.0 Hz, 1H), 2.89(dd, J = 10.0 Hz, 1H), 2.48(s, 3H), 2.07-2.20 (m, 2H), 1.94-2.03 (m, 1H), 0.90 (sd, 3H); MS (ESI) m/z $[M+H]^+$ = 456.2, 438.2 (3:1).

Step 6: Chiral resolution of 5-chloro-2-(4,4-difluoro-3-methylpiperidin-1-yl)-N-(4-fluoro-3-(N'-hydroxyamidino) ph enyl)-6-(methyl)nicotinamide (**I-41**)

1-41 has the following chiral isomers:

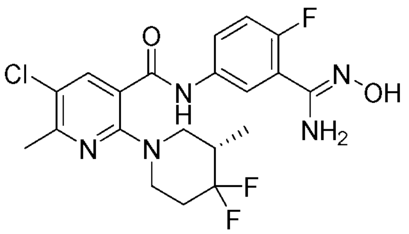
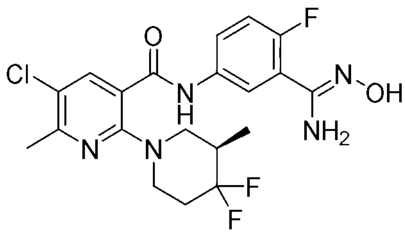

**Preparation conditions:** [Instrument: Berger SFC Multigram II (Mettler-Toledo International Inc.); Column: Superchiral R-OJ (Chiralway Biotech Co., Ltd.), 2.1 cm ID * 25 cm Length, 5 μm; Column temperature: 40°C; Mobile phase: $CO_2$/EtOH/DEA = 80/20/0.05 (v/v/v); Detection wavelength: 214 nm; Flow rate: 30 ml/min].

**Analytical conditions:** [Instrument: Berger analytical SFC (Mettler-Toledo International Inc.); Column: Superchiral R-OJ (Chiralway Biotech Co., Ltd.), 0.46 cm ID * 15 cm Length, 5 μm; Column temperature: 40°C; Mobile phase: $CO_2$/EtOH/DEA = 80/20/0.05 (v/v/v); Detection wavelength: 214 nm; Flow rate: 3.0 ml/min].

**Specific rotation test conditions:** [Instrument: SGW-1 (Shanghai INESA Physico-Optical Instrument Co., Ltd.); Temperature: 20°C; Wavelength: 589.4 nm; Solvent: Methanol].

Compound I-41 was resolved by the chiral preparative separation column described above to obtain **I-41-P1** (peak elution time 1.650 min, ee 98%) and **I-41-P2** (peak elution time 1.911min, ee value 98%).

**Example 42:** Preparation of 5-chloro-2-(4,4-difluoroazepan-1-yl)-N-(4-fluoro-3-(N'-hydroxyamidino)phenyl)-6-deu teromethylnicotinamide (**I-42**)

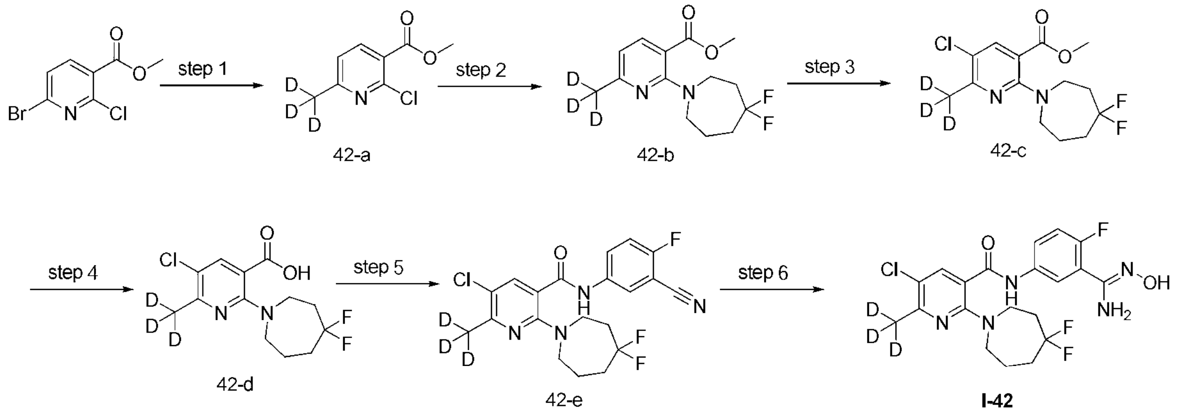


Step 1: Under a nitrogen atmosphere, at approximately 0°C, a solution of deuteromethylmagnesium iodide ($CD_3MgI$) (5.2 mL, 1.0 mol/L) was slowly added dropwise to a solution of methyl 6-bromo-2-chloronicotinate (1.0 g, 5.0 mmol) and 1,3-bis(diphenylphosphinopropane)nickel dichloride ($Ni(dppp)Cl_2$) (100 mg, 0.16 mmol) in anhydrous THF (30 mL) under stirring. After the addition was complete, the mixture was allowed to warm naturally to room temperature and react overnight. The reaction mixture was slowly added with saturated brine, and extracted with ethyl acetate to obtain an organic phase, which was dried over anhydrous sodium sulfate and concentrated to obtain a crude product. The crude product was purified by silica gel (200-300 mesh) column chromatography using petroleum ether:dichloromethane (1:1, v/v) to obtain 330.0 mg of methyl 6-deuterated methyl-2-chloronicotinate **(42-a)** in a yield of 43.8%. $^1H$ NMR (400 MHz, DMSO-$d_6$) δ: 8.15 (d, J = 7.6 Hz, 1H), 7.42 (d, J = 7.6 Hz, 1H), 2.86 (s, 3H); MS (ESI) m/z $[M+H]^+$ = 189.1, 191.1 (3:1).

Step 2: Following the process of Step 4 of Example 1, methyl 6-deuterated methyl-2-chloronicotinate **(42-a)** (250 mg, 1.35 mmol) was used as a starting material to obtain 200.0 mg of methyl 2-(4,4-difluoroazepan-1-yl)-6-(deuterated methyl)nicotinate **(42-b)** in a yield of 43.5%. $^1H$ NMR (400 MHz, DMSO-$d_6$) δ: 7.76(d, *J* = 8.0 Hz, 1H), 6.61(d, *J* = 8.0 Hz, 1H), 3.78(s, 3H), 3.58~3.61(sq, 2H), 3.26(t, *J* = 5.6 Hz, 2H), 2.28~2.38(tq, 2H), 1.92~2.01(m, 2H), 1.84~1.90(m, 2H); MS(ESI) m/z $[M+H]^+$ = 288.1.

Step 3: Following the process of Step 5 of Example 1, methyl 2-(4,4-difluoroazepan-1-yl)-6-(deuteromethyl) nicotinate **(42-b)** (450.0 mg, 1.57 mmol) was used as a starting material to obtain 300 mg of methyl 5-chloro-2-(4,4-difluoroazepan-1-yl)-6-(deuteromethyl)nicotinate **(42-c)** in a yield of 59.6%. $^1H$ NMR (400 MHz, DMSO-$d_6$) δ: 7.83(s,1H), 3.80(s, 3H), 3.58(brs, 2H), 3.23(brs, 2H), 2.31(brs, 2H), 1.98(brs, 2H), 1.87(brs, 2H); MS(ESI) m/z $[M+H]^+$ = 322.2, 324.2(3:1).

Step 4: Following the process of Step 6 of Example 1, methyl 5-chloro-2-(4,4-difluoroazepan-1-yl)-6-(deuteromethyl)nicotinate **(42-c)** (280.0 mg, 0.87 mmol) was used as a starting material to obtain 268.0 mg of 5-chloro-2-(4,4-difluoroazepan-1-yl)-6-(deuteromethyl)nicotinic acid **(42-d),** which was directly used in the next step, in a yield of 100%.

Step 5: Following the process of Step 7 of Example 1, 5-chloro-2-(4,4-difluoroazepan-1-yl)-6-(duteromethyl) nicotinic acid **(42-d)** (268.0 mg, 0.87 mmol) and 5-amino-2-fluorobenzonitrile (142.0 mg, 1.04 mmol) were used as starting materials to obtain 191.0 mg of 5-chloro-N-(3-cyano-4-fluorophenyl)-2-(4,4-difluoroazepan-1-yl)-6-(duteromethyl)nic otinamide **(42-e)** in a yield of 51.5%. $^1H$ NMR (400 MHz, DMSO-$d_6$) δ: 10.76(brs, 1H), 8.17(dd, J = 5.6, 2.8 Hz, 1H), 7.84(td, J = 7.6, 1.2 Hz, 1H), 7.78(s, 1H), 7.54(t, J = 7.6 Hz, 1H), 3.61(sq, 2H), 3.38(t, J = 5.6 Hz, 2H), 2.24~2.36(tm, 2H), 1.91~2.01(m, 2H); MS(ESI) m/z $[M+H]^+$ = 426.2.

Step 6: Following the process of Step 5 of Example 38, 5-chloro-N-(3-cyano-4-fluorophenyl)-2-(4,4-difluoroazepan-1-yl)-6-(duteomethyl)nico tinamide **(42-e)** (145 mg, 0.34 mmol) was used as a starting material to obtain 120.0 mg of 5-chloro-2-(4,4-difluoroazepan-1-yl)-N-(4-fluoro-3-(N'-hydroxyamidino)phenyl)-6-(du teomethyl)nicotinamide (**I-42**) in a yield of 76.9%. $^1H$ NMR (400 MHz, DMSO-$d_6$) δ: 10.50(brs, 1H), 9.64(brs, 1H), 7.84(dd, J = 6.4, 2.4 Hz, 1H), 7.72(s, 1H), 7.70(dd, 1H), 7.22(t, J = 9.6 Hz, 1H), 5.80(brs, 2H), 3.61(sq, 2H), 3.41(t, J = 5.6 Hz, 2H), 2.26~2.35(tm, 2H); MS(ESI) m/z $[M+H]^+$ = 459.2.

**Example 43:** Preparation of 5-chloro-2-(4,4-difluoroazepan-1-yl)-6-(dimethylamino)-N-(4-fluoro-3-(N'-hydroxyami dino)phenyl)nicotinamide (**I-43**)

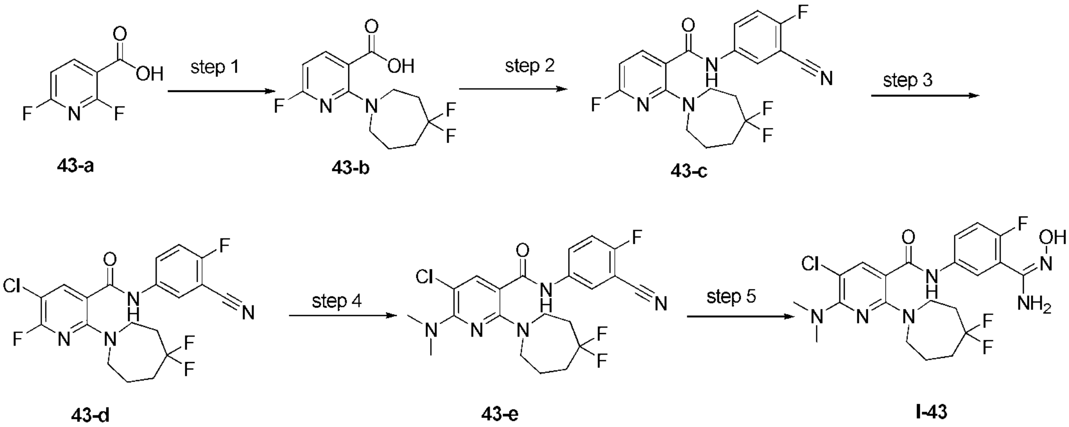


43-a 43-b 43-c 43-d 43-e I-43

Step 1: 2,6-difluoronicotinic acid **(43-a)** (1.0 g, 6.29 mmol) and 4,4-difluoroazepane hydrochloride (1-c) (1.29 g, 7.5 mmol) were dissolved in N,N-dimethylformamide, and potassium carbonate (2.61 g, 18.9 mmol) was added. The reaction mixture was heated to 100°C and allowed to react overnight. After the reaction was complete, the mixture was diluted with 10 mL of water and adjusted to pH 2-3 with 6N HCl to precipitate a solid, which was filtered to obtain 1.2 g of 2-(4,4-difluoroazepane)-6-fluoronicotinic acid **(43-b)** in a yield of 70%. $^1$H NMR (400 MHz, DMSO-$d_6$) δ: 12.86 (s, 1H), 8.08-7.99 (m, 1H), 6.37 (m, 1H), 3.56 (m, 2H), 3.33-3.28 (m, 2H), 2.29 (m, 2H), 1.98 (m, 2H), 1.88 (m, 2H); MS (ESI) m/z [M-H]- = 273.1.

Step 2: 2-(4,4-difluoroazepane)-6-fluoronicotinic acid **(43-b)** (500 mg, 1.82 mmol) was dissolved in dichloromethane (20 mL), and a catalytic amount of DMF was added thereto. The reaction mixture was placed in an ice bath and slowly added dropwise with a solution of a 2M oxalyl chloride in dichloromethane (1.82 mL, 3.64 mmol). The mixture was allowed to react at room temperature for 2 h, concentrated under reduced pressure, added with 5-amino-2-fluorobenzonitrile (298 mg, 2.18 mmol) in an ice bath, and then slowly added dropwise with anhydrous pyridine (6 mL) and allowed to react at room temperature for 1 h. After the reaction was complete, the reaction mixture was quenched with ice water and extracted with ethyl acetate. The organic layer was washed with saturated sodium bisulfite and saturated brine, dried over anhydrous sodium sulfate, and concentrated under reduced pressure. The resultant was then purified by column chromatography to obtain 370 mg of N-(3-cyano-4-fluorophenyl)-2-(4,4-difluoroazepane)-6-fluoronicotinamide **(43-c)** as a white solid in a yield of 52%. $^1$H NMR (400MHz, DMSO-$d_6$) δ: 10.75(s, 1H), 8.21~8.16(m, 1H), 7.92(q, J = 8.2 Hz, 2H), 7.58~7.51(m, 1H), 6.47~6.42(m, 1H), 3.58(d, J = 6.0 Hz, 2H), 3.40(t, J = 5.8 Hz, 2H), 2.30(s, 2H), 1.95(d, J = 14.0 Hz, 2H), 1.85(d, J = 6.4 Hz, 2H); MS(ESI) m/z [M+H]$^+$ = 393.1.

Step 3: N-(3-cyano-4-fluorophenyl)-2-(4,4-difluoroazepane)-6-fluoronicotinamide (**43-c**) (150 mg, 0.52 mmol) and N-chlorosuccinimide (17.2 mg, 0.52 mmol) were dissolved in N,N-dimethylacetamide (3 mL) and reacted at 100°C for 1 h. After the reaction was complete, the reaction mixture was added with water (10 mL) and extracted three times with ethyl acetate. The combined organic layer waswashed once with saturated brine, dried over anhydrous sodium sulfate, and concentrated in vacuum to remove the organic solvent to obtain a crude product. The crude product was purified by silica gel (200-300 mesh) column chromatography using PE:EA=20:1 (v/v) to obtain 120 mg of 5-chloro-N-(3-cyano-4-fluorophenyl)-2-(4,4-difluoroazepane)-6-fluoronicotinamide (**43-d**) as a yellowish solid in a yield of 74%. $^1$H NMR (400MHz, DMSO-$d_6$) δ: 10.82(s, 1H), 8.17(dd, J = 5.8, 2.6 Hz, 1H), 8.11(d, J = 9.2 Hz, 1H), 7.97~7.89(m, 1H), 7.56(t, J = 9.2 Hz, 1H), 3.57(s, 2H), 3.39(t, J = 5.9 Hz, 2H), 2.31(s, 2H), 1.96(d, J = 17.5 Hz, 2H), 1.86(d, J = 7.1 Hz, 2H); MS(ESI) m/z [M+H]$^+$ = 427.1.

Step 4: 5-Chloro-N-(3-cyano-4-fluorophenyl)-2-(4,4-difluoroazepane)-6-fluoronicotinamide (**43-d**) (27 mg, 0.063 mmol), $K_2CO_3$ (44 mg, 0.315 mmol), and dimethylamine hydrochloride (16 mg, 0.19 mmol) were added to a microwave tube and microwaved at 100°C for 1 h. After the reaction was complete as monitored by TLC, the mixture was diluted with water and extracted with ethyl acetate. The organic layer was washed with water and saturated brine respectively, dried over anhydrous sodium sulfate, and concentrated under reduced pressure to obtain a crude product. The crude product was purified by column chromatography using PE:EA (10:1 v/v) to obtain 9 mg of 5-chloro-N-(3-cyano-4-fluorophenyl)-2-(4,4-difluoroazepane)-6-(dimethylamino)nicot inamide **(43-e)** as a white solid in a yield of 31%. $^1$H NMR (400 MHz, DMSO-$d_6$) δ: 10.53(s, 1H), 8.19~8.14(m, 1H), 7.92(m, 1H), 7.67(s, 1H), 7.52(t, J = 9.2 Hz, 1H), 3.59(m, 2H), 3.37(m, 2H), 3.00(s, 6H), 2.3 (s, 2H), 2.01~1.92 (m, 2H), 1.83(m, 2H); MS(ESI) m/z [M+H]$^+$ = 452.1.

Step 5: Following the process of Step 5 of Example 38, 5-chloro-N-(3-cyano-4-fluorophenyl)-2-(4,4-difluoroazepane)-6-(dimethylamino)nicot inamide **(43-e)** (9 mg, 0.02 mmol) and an aqueous hydroxylamine solution (50% wt, 15 μL, 0.2 mmol) were used to obtain 3 mg of 5-chloro-2-(4,4-difluoroazepane)-N-(4-fluoro-3-(N'-hydroxyamidino)phenyl)-6-(dimet hylamino)nicotinamide as a white solid in a yield of 31%. $^1$H NMR (400 MHz, $CDCl_3$) δ: 9.62(s, 1H), 8.04(s, 1H), 7.86(s, 1H), 7.73(dd, J = 6.3, 2.8 Hz, 1H), 7.12(dd, J = 11.0, 9.0 Hz, 1H), 5.30(s, 1H), 5.16(s, 2H), 3.58~3.51(m, 2H),

3.39(t, J = 5.7 Hz, 2H), 3.13(s, 6H), 2.44~2.33(m, 2H), 2.22~2.11(m,2H), 1.94~1.89(m, 2H); MS(ESI) m/z $[M+H]^+$ = 485.2.

**Example 44:** Preparation of 5-chloro-2-(4,4-difluoroazepan-1-yl)-N-(4-fluoro-3-(N'-hydroxyamidino)phenyl)-6-(flu oromethyl)nicotinamide **(1-44)**

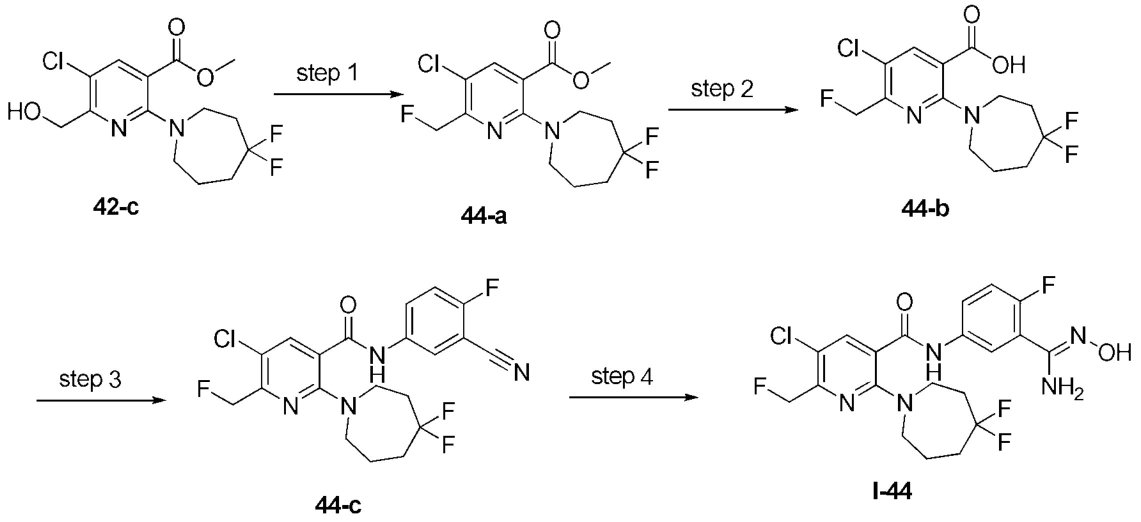


42-c 44-a 44-b 44-c I-44

Step 1: methyl 5-chloro-2-(4,4-difluoroazepan-1-yl)-6-hydroxymethylnicotinate **(42-c)** (268.0 mg, 0.8 mmol) was dissolved in dichloromethane (10.0 mL), cooled to 0°C, slowly added with diethylaminosulfur trifluoride (DAST) (155.0 mg, 0.96 mmol) dropwise, and naturally warmed to room temperature for reaction. After **42-c** was completely consumed, the reaction mixture was poured into ice water and extracted three times with dichloromethane. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using PE:EA = 10:1-4:1 (v/v) to obtain 200.0 mg of methyl 5-chloro-2-(4,4-difluoroazepan-1-yl)-6-(fluoromethyl)nicotinate **(44-a)** in a yield of 74.0%. $^1$H NMR (400 MHz, DMSO-$d_6$) δ: 7.96(s, 1H), 5.49(s, 1H), 5.37(s, 1H), 3.82(s, 3H), 3.62(sm, J = 7.2 Hz,2H), 3.24(t, J = 5.2 Hz, 2H), 2.72~2.36(td, 2H), 1.95~1.99(m, 2H), 1.84~1.92(m, 2H); MS(ESI) m/z $[M+H]^+$ = 337.2, 339.2 (3:1).

Step 2: Following the process of Step 6 of Example 1, methyl 5-chloro-2-(4,4-difluoroazepan-1-yl)-6-(fluoromethyl)nicotinate **(44-a)** (200.0 mg, 0.6 mmol) was used as a starting material to obtain 192 mg of 5-chloro-2-(4,4-difluoroazepan-1-yl)-6-fluoromethylnicotinic acid **(44-b),** which was directly used in the next step, in a yield of 100%.

Step 3: Following the process of Step 7 of Example 1, 5-chloro-2-(4,4-difluoroazepan-1-yl)-6-fluoromethylnicotinic acid **(44-b)** (192 mg, 0.6 mmol) was used as a starting material to obtain 140.0 mg of 5-chloro-N-(3-cyano-4-fluorophenyl)-2-(4,4-difluoroazepan-1-yl)-6-(fluoromethyl)nic otinamide **(44-c)** in a yield of 53.4%. $^1$H NMR (400 MHz, DMSO-$d_6$) δ: 10.87(s, 1H), 8.17~8.19(sq, J = 8.0Hz, 1H), 7.93~7.95(m, 1H), 7.92(s, 1H), 7.56(t, J = 9.2Hz, 1H), 5.50(s, 1H), 5.38(s, 1H), 3.62~3.64(sm, 2H), 3.417(d, J = 6.0 Hz, 2H), 2.26~2.37(tq, 2H), 1.94~2.03(m, 2H), 1.81~188(m, 2H); MS(ESI) m/z $[M+H]^+$ = 441.2, 443.2 (3:1). Step 4: Following the process of Step 5 of Example 38, 5-chloro-N-(3-cyano-4-fluorophenyl)-2-(4,4-difluoroazepan-1-yl)-6-(fluoromethyl)nic otinamide **(44-c)** (100.0 mg, 0.23 mmol) was used as a starting material to obtain 66.0 mg of 5-chloro-2-(4,4-difluoroazepan-1-yl)-N-(4-fluoro-3-(N'-hydroxyamidino)phenyl)-6-(flu oromethyl)nicotinamide (**I-44**) in a yield of 61.4%. $^1$H NMR (400 MHz, DMSO-$d_6$) δ: 10.62(brs, 1H), 9.68(brs, 1H), 7.87(s, 1H), 7.85(s, 1H), 7.71(s, 1H), 7.24(s, 1H), 5.86(brs, 2H), 5.50(s, 1H), 5.38(s, 1H), 3.64(s, 2H), 3.44(s, 2H), 2.30(s, 2H), 1.97(s, 2H), 1.85(s, 2H), MS(ESI) m/z $[M+H]^+$ = 474.2.

**Example 45:** Preparation of 5-chloro-2-(4,4-difluoroazepan-1-yl)-6-ethyl-N-(4-fluoro-3-(N'-hydroxyamidino)pheny l)nicotinamide **(1-45)**

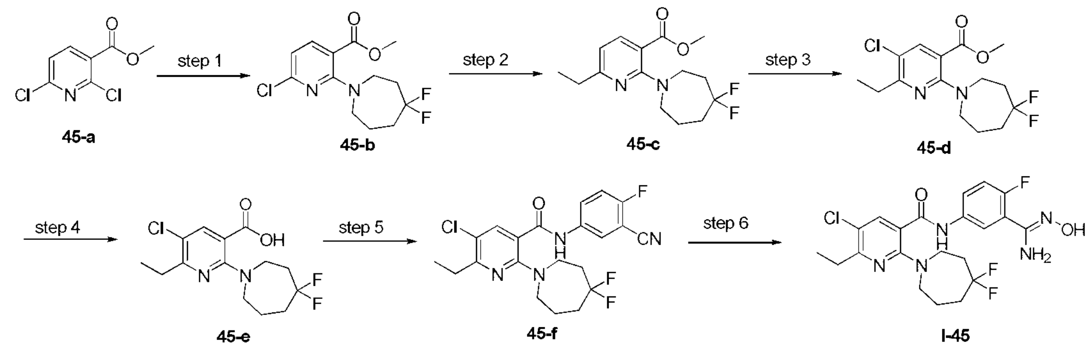


Step 1: 2,6-dichloronicotinic acid **(45-a)** (1.0 g, 4.85 mmol) and 4,4-difluoroazepane hydrochloride **(1-c)** (1.0 g, 5.82 mmol) were dissolved in N,N-dimethylformamide (20 mL), added with potassium carbonate (2.0 g, 14.56 mmol) and allowed to react at room temperature overnight. After the reaction was complete, the reaction mixture was diluted with 10 mL of water, adjusted to pH 2-3 with 6N HCl, and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using PE:EA = 20-10:1 (v/v) to obtain 440.0 mg of 2-(4,4-difluoroazepane)-6-chloronicotinic acid **(45-b)** in a yield of 29.7%. MS (ESI) m/z $[M+H]^+$ = 305.2, 307.2 (3:1).

Step 2: Under $N_2$ atmosphere, a solution of ethylmagnesium bromide (1.6 mL, 2.0 mol/L) was slowly added dropwise to a solution of 2-(4,4-difluoroazepane)-6-chloronicotinic acid **(45-b)** (500.0 mg, 1.6 mmol) and 1,3-bis(diphenylphosphino)propane nickel dichloride ($Ni(dppp)Cl_2$) (28.4 mg, 0.05 mmol) in anhydrous THF (30 mL) under stirring at room temperature. After the addition was complete, the mixture was naturally warmed to room temperature and allowed to react overnight. The starting material was not completely consumed. The reaction mixture was slowly added with saturated brine, and extracted with ethyl acetate to obtain an organic phase, which was dried over anhydrous sodium sulfate, and concentrated to obtain a crude product. The crude product was purified by silica gel (200-300 mesh) column chromatography using petroleum ether:ethyl acetate (20-10:1, v/v) to obtain 140.0 mg of methyl 2-(4,4-difluoroazepan-1-yl)-6-ethylnicotinate **(45-c)** with a conversion rate of 60% and an adjusted yield of 47.8%. $^1H$ NMR (400 MHz, $CDCl_3$) δ: 7.81(d, J = 8.0 Hz, 1H), 6.51(d, J = 8.0 Hz, 1H), 3.85(s, 3H), 3.74(st, J = 8.8 Hz, 2H), 3.29(t, J = 5.6 Hz, 2H), 2.67(q, J = 7.6 Hz, 2H), 2.40(tt, J =9.2, 4.4 Hz, 2H), 1.98~2.01(q, 2H), 1.93~1.96(d, J = 8.8 Hz, 2H), 1.25(t, J = 7.6 Hz, 3H); MS(ESI) m/z $[M+H]^+$ = 299.2.

Step 3: Methyl 2-(4,4-difluoroazepan-1-yl)-6-ethylnicotinate **(45-c)** (140 mg, 0.47 mmol) and N-chlorosuccinimide (81.4 mg, 0.61 mmol) were dissolved in acetonitrile (3 mL) and reacted under reflux. After the reaction was complete, the reaction mixture was added with water (10 mL) and extracted three times with ethyl acetate. The combined organic layer was washed once with saturated brine, dried over anhydrous sodium sulfate, and concentrated in vacuum to remove the organic solvent to obtain a crude product. The crude product was then purified by silica gel (200-300 mesh) column chromatography using a PE:EA =30:1 (v/v) to obtain 80.0 mg of methyl 5-chloro-2-(4,4-difluoroazepan-1-yl)-6-ethylnicotinate **(45-d)** as an oil in a yield of 51.2%. $^1H$ NMR (600MHz, $CDCl_3$) δ: 7.84 (s,1H), 3.86 (s,3H), 3.72~3.74 (q,2H), 3.26(t, J = 6.0 Hz, 2H), 2.82 (q, J = 7.8Hz, 2H), 2.33~2.40 (tq, 2H), 1.95~1.98 (q, 2H), 1.92~1.94 (q, 2H), 1.25(t, J =7.8 Hz, 3H); MS(ESI) m/z $[M+H]^+$ = 333.2, 335.2(3:1).

Step 4: Following the process of Step 6 of Example 1, methyl 5-chloro-2-(4,4-difluoroazepan-1-yl)-6-ethylnicotinate **(45-d)** (57.0 mg, 0.17 mmol) was used as a starting material to obtain 54 mg of 5-chloro-2-(4,4-difluoroazepan-1-yl)-6-ethylnicotinic acid **(45-e),** which was directly used in the next step, in a yield of 100%. MS (ESI) m/z $[M+H]^+$ = 319.2, 321.2 (3:1). Step 5: Following the process of Step 7 of Example 1, 5-chloro-2-(4,4-difluoroazepan-1-yl)-6-ethylnicotinic acid **(45-e)** (54.0 mg, 0.17 mmol) and 5-amino-2-fluorobenzonitrile (28.0 mg, 0.20 mmol) were used as starting materials to obtain 42.0 mg of 5-chloro-N-(3-cyano-4-fluorophenyl)-2-(4,4-difluoroazepan-1-yl)-6-ethylnicotinamide (**45-f**) in a yield of 56%. $^1H$ NMR (400 MHz, DMSO-$d_6$) δ: 10.76 (s,1H), 8.18 (dd, *J* = 5.6, 2.4 Hz, 1H), 7.92~7.96 (m, 1H), 7.78 (s, 1H), 7.54 (t, *J* = 9.2 Hz, 1H), 3.62~3.64 (brs, 2H), 3.40 (t, *J* = 5.6 Hz, 2H), 2.78 (q, *J* = 7.6 Hz, 2H), 2.27~2.37 (tq, 2H), 1.93~2.01 (q, 2H), 1.85~1.91(q, 2H), 1.22(t, *J* = 7.6 Hz, 3H); MS (ESI) m/z $[M+H]^+$ = 426.2, 428.2 (3:1).

Step 6: Following the process of Step 5 of Example 38, 5-chloro-N-(3-cyano-4-fluorophenyl)-2-(4,4-difluoroazepan-1-yl)-6-ethylnicotinamide (**45-f**) (40 mg, 0.09 mmol) was used as a starting material to obtain 9.0 mg of 5-chloro-2-(4,4-difluoroazepan-1-yl)-N-(4-fluoro-3-(N'-hydroxyamidino)phenyl)-6-eth ylnicotinamide (**I-45**) in a yield of 21%. $^1H$ NMR (400 MHz, DMSO-$d_6$) δ: 10.50 (s,1H), 9.64 (s, 1H), 7.83 (dd, *J* = 9.2, 2.4 Hz, 1H), 7.71 (s, 1H), 7.70 (qt, *J* =8.8, 2.4 Hz, 1H), 7.20 (dd, *J* = 9.2, 8.8 Hz, 1H), 5.80 (brs, 2H), 3.62~3.64 (sm, 2H), 3.42 (t, *J* = 6.0 Hz, 2H), 2.77 (q, *J* = 7.6Hz, 2H), 2.27~2.36 (m, 2H), 1.95~2.01 (m, 2H), 1.82~1.87 (m, 2H), 1.22(t, *J* = 7.6 Hz, 3H); MS(ESI) m/z $[M+H]^+$ = 470.2, 472.2(3:1).

**Example 46:** Preparation of 5-chloro-2-(4,4-difluoroazepan-1-yl)-N-(4-fluoro-3-(N'-hydroxyamidino)phenyl)-6-(pyr rolidinyl)nicotinamide (**I-46**)

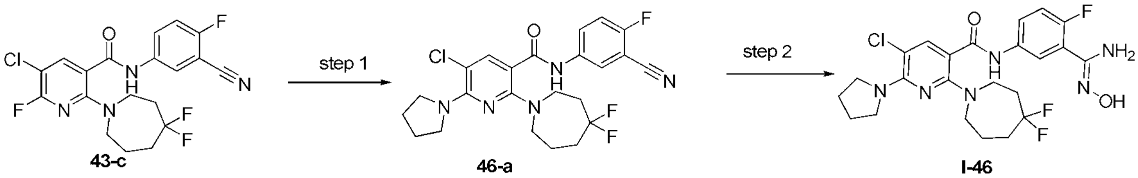


43-c 46-a I-46

Step 1: 5-chloro-N-(3-cyano-4-fluorophenyl)-2-(4,4-difluoroazepan-1-yl)-6-fluoronicotinamid e **(43-c)** (100 mg, 0.14 mmol), $K_2CO_3$ (32 mg, 0.14 mmol), and tetrahydropyrrole (20 μL, 0.14 mmol) were added to a sealed tube and reacted at 50°C. After the reaction was complete as monitored by TLC, the mixture was diluted with water and extracted with ethyl acetate. The organic layer was washed with water and saturated brine respectively, dried over anhydrous sodium sulfate, and concentrated under reduced pressure to obtain a crude product. The crude product was purified by silica gel (200-300 mesh) column chromatography using PE:EA=10:1 (v/v) to obtain 77 mg of 5-chloro-N-(3-cyano-4-fluorophenyl)-2-(4,4-difluoroazepane)-6-(pyrrolidinyl)nicotina mide **(46-a)** as a yellow solid in a yield of 55%. $^1$H NMR (400 MHz, DMSO-*d*$_6$) δ: 10.45 (s, 1H), 8.17 (brs, 1H), 7.93 (brs, 1H), 7.61 (s, 1H), 7.51 (t, *J* = 9.2 Hz, 1H), 3.64 (t, 4H), 3.56 (brs, 2H), 3.36 (m, 2H), 2.33 (m, 2H), 1.93 (m, 2H), 1.87 (t, 4H), 1.82 (s, 2H); MS(ESI) m/z $[M+H]^+$ =478.2.

Step 2: Following the process of Step 5 of Example 38, 5-chloro-N-(3-cyano-4-fluorophenyl)-2-(4,4-difluoroazepan-1-yl)-6-(pyrrolidinyl)nicoti namide **(46-a)** (77 mg, 0.16 mmol) and an aqueous hydroxylamine solution (50% wt, 99 μL, 1.6 mmol) were used to obtain 27 mg of 5-chloro-2-(4,4-difluoroazepan-1-yl)-N-(4-fluoro-3-(N'-hydroxyamidino) phenyl)-6-(pyr rolidinyl)nicotinamide (**I-46**) as a white solid in a yield of 33%. $^1$H NMR (400 MHz, DMSO-*d*$_6$) δ: 10.22 (s, 1H), 9.62 (s, 1H), 7.87~7.78 (m, 1H), 7.73~7.66 (m, 1H), 7.56 (s, 1H), 7.19 (t, *J* = 9.7 Hz, 1H), 5.79 (s, 2H), 3.64 (t, 4H), 3.61~3.57(m,2H), 3.37(m, 2H), 2.33(m, 2H), 1.94(m, 2H), 1.88(t, 4H), 1.84(m, 2H); MS(ESI) m/z $[M+H]^+$ = 511.2.

**Example 47:** Preparation of 4,5-dichloro-2-(4,4-difluoroazepan-1-yl)-N-(4-fluoro-3-(N'-hydroxyamidino)phenyl)be nzamide (**I-47**)

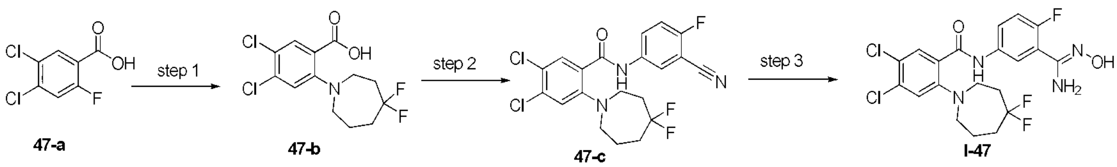


47-a 47-b 47-c I-47

Step 1: 4,5-dichloro-2-fluorobenzoic acid **(47-a)** (300 mg, 1.44 mmol) and 4,4-difluoroazepane hydrochloride **(1-c)** (271 mg, 1.58 mmol) were dissolved in dimethyl sulfoxide, and potassium carbonate (793 mg, 5.76 mmol) was added. The reaction system was heated to 140°C and stirred overnight. After the reaction was complete, the reaction system was diluted with 10 mL of water, adjusted to about pH 2-3 with 6N HCl, and extracted with ethyl acetate. The organic layer was washed with water and saturated brine respectively, dried over anhydrous sodium sulfate, and concentrated under reduced pressure to obtain a crude product. The crude product was purified by silica gel (200-300 mesh) column chromatography using PE:EA:AcOH = 100:10:1 (v/v/v) to obtain 154 mg of chloro-2-(4,4-difluoroazepane)-4-fluorobenzoic acid **(47-b)** in a yield of 37%; MS (ESI) m/z [M-H] = 322.0.

Step 2: Following the process of Step 7 of Example 1, 4,5-dichloro-2-(4,4-difluoroazepan-1-yl)benzoic acid **(47-b)** (154 mg, 0.48 mmol) and 5-amino-2-fluorobenzonitrile (72 mg, 0.57 mmol) was used to obtain 45 mg of 4,5-dichloro-N-(3-cyano-4-fluorophenyl)-2-(4,4-difluoroazepan-1-yl)benzamide **(47-c)** as a white solid in a yield of 21%. $^1$H NMR (400 MHz, DMSO-*d*$_6$) δ: 10.83 (s, 1H), 8.19 (dd, *J* = 5.8, 2.7 Hz, 1H), 8.00~7.94 (m, 1H), 7.64 (s, 1H), 7.60~7.53 (m, 1H), 7.26 (s, 1H), 3.57 (m, 2H), 3.29 (m, 2H), 2.23 (m, 2H), 2.13~1.98 (m, 2H), 1.78 (m, 2H); MS(ESI) m/z$[M+H]^+$ = 442.1.

Step 3: Following the process of Step 5 of Example 38, 4,5-dichloro-N-(3-cyano-4-fluorophenyl)-2-(4,4-difluoroazepan-1-yl)benzamide **(47-c)** (45 mg, 0.11 mmol) and an aqueous hydroxylamine solution (50 wt%, 90 μL, 2.2 mmol) were used to obtain 10 mg of 4,5-dichloro-2-(4,4-difluoroazepan-1-yl)-N-(4-fluoro-3-(N'-hydroxyamidino)phenyl)be nzamide **(I-47)** as a white solid in a yield of 21%. $^1$H NMR (400 MHz, DMSO-*d*$_6$) δ: 10.58 (s, 1H), 9.65 (s, 1H), 7.86 (dd, *J* = 6.6, 2.6 Hz, 1H), 7.70 (dd, *J* = 8.6, 4.2 Hz, 1H), 7.59 (s, 1H), 7.30~7.16 (m, 2H), 5.81 (s, 2H), 3.36(m, 2H), 3.30 (m, 2H), 2.22 (m, 2H), 2.13~2.00 (m, 2H), 1.78 (m, 2H) ; MS(ESI) m/z$[M+H]^+$ = 475.1.

**Example 48:** Preparation of 5-chloro-2-(4,4-difluoroazepan-1-yl)-N-(3-(N'-hydroxyamidino)phenyl)-4-trifluoromet hylbenzamide (**I-48**)

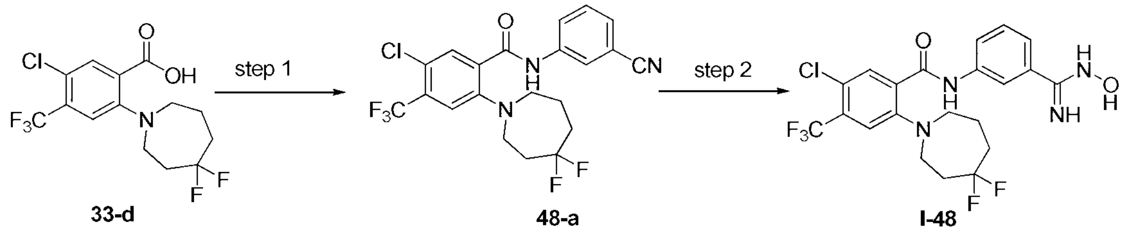


33-d 48-a I-48

Step 1: Following the process of Step 7 of Example 1, 5-chloro-2-(4,4-difluoroazepan-1-yl)-4-trifluoromethylbenzoic acid **(33-d)** (50 mg, 0.14 mmol) and 3-aminobenzonitrile (20 mg, 0.17 mmol) were used to obtain 30 mg of 5-chloro-N-(3-cyanophenyl)-2-(4,4-difluoroazepan-1-yl)-4-trifluoromethylbenzamide (**48-a**) as a white solid in a yield of 47%. $^1H$ NMR (400 MHz, DMSO-$d_6$) δ: 10.89 (s, 1H), 8.17 (s, 1H), 7.93 (m, 1H), 7.72 (s, 1H), 7.61 (d, J = 4.8 Hz, 2H), 7.36 (s, 1H), 3.43~3.35 (m, 4H), 2.24 (m, 2H), 2.14~1.97 (m, 2H), 1.81 (m, 2H); MS(ESI) m/z $[M+H]^+$ = 458.1.

Step 2: Following the process of Step 5 of Example 38, 5-chloro-N-(3-cyanophenyl)-2-(4,4-difluoroazepan-1-yl)-4-trifluorobenzamide **(48-a)** (30 mg, 0.07 mmol) and an aqueous hydroxylamine solution (50% wt, 80 μL, 1.4 mmol) were used to obtain 10 mg of 5-chloro-2-(4,4-difluoroazepan-1-yl)-N-(3-(N'-hydroxyamidino)phenyl)-4-trifluoromet hylbenzamide (**I-48**) as a white solid in a yield of 31%. $^1H$ NMR (400 MHz, DMSO-$d_6$) δ: 10.60 (s, 1H), 9.65 (s, 1H), 8.05 (d, J = 2.1 Hz, 1H), 7.67(d, J = 7.5 Hz, 1H), 7.64(s, 1H), 7.41~7.31(m, 3H), 5.76(s, 2H), 3.43~3.27(m, 4H), 2.23(m, 2H), 2.05(m, 2H), 1.80(m, 2H); MS(ESI) m/z $[M+H]^+$ = 491.1.

**Example 49:** Preparation of 5-chloro-2-(4,4-difluoroazepan-1-yl)-N-(3-(N'-hydroxyamidino)phenyl)-6-(trifluorome thyl) benzamide **(1-49)**

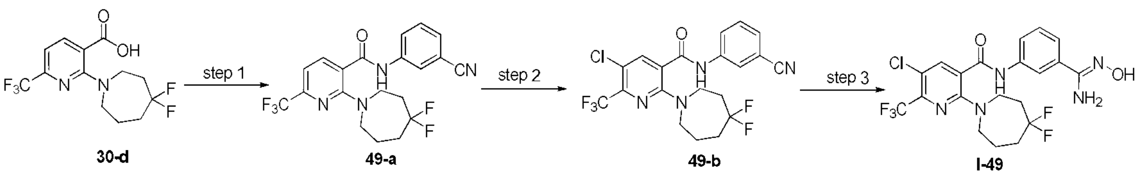


30-d 49-a 49-b I-49

Step 1: 2-(4,4-difluoroazepan-1-yl)-6-(trifluoromethyl)nicotinic acid **(30-d)** (90 mg, 0.28 mmol) was dissolved in dichloromethane (3 mL) and added with a catalytic amount of DMF. The reaction mixture was placed in an ice bath, slowly added dropwise with an oxalyl chloride solution (32 μL, 0.42 mmol) for reacting at room temperature for 2 h. After concentrated under reduced pressure, The reaction mixture was added with 3-aminobenzonitrile (36 mg, 0.33 mmol) in an ice bath, and then slowly added dropwise with anhydrous pyridine (2 mL) for reacting at room temperature for 1 h. After the reaction was complete, the reaction mixture was quenched with ice water and extracted with ethyl acetate. The organic layer was washed with saturated sodium bisulfite and saturated brine, dried over anhydrous sodium sulfate, concentrated under reduced pressure, and separated by column chromatography to obtain 70 mg of N-(3-cyanophenyl)-2-(4,4-difluoroazepan-1-yl)-6-(trifluoromethyl)nicotinamide **(49-a)** as a white solid in a yield of 59%. $^1H$ NMR (400MHz, DMSO-$d_6$) δ: 10.89(s, 1H), 8.17(s, 1H), 7.98(d, *J* = 7.6 Hz, 1H), 7.95~7.88(m, 1H), 7.60(d, *J* = 4.8 Hz, 2H), 7.24~7.19(m, 1H), 3.66(t, *J* = 5.4 Hz, 2H), 3.48(t, *J* = 5.6 Hz, 2H), 2.33(m, 2H), 1.96(m, 2H), 1.87(m, 2H); MS(ESI) m/z $[M+H]^+$ = 425.1.

Step 2: N-(3-cyanophenyl)-2-(4,4-difluoroazepan-1-yl)-6-(trifluoromethyl)nicotinamide **(49-a)** (70 mg, 0.16 mmol) and N-chlorosuccinimide (22 mg, 0.16 mmol) were dissolved in N,N-dimethylacetamide (1 mL) and reacted at 100°C. After the reaction was complete, the reaction mixture was added with water (10 mL) and extracted three times with ethyl acetate. The combined organic layer was washed once with saturated brine, dried over anhydrous sodium sulfate, and concentrated in vacuum to remove the organic solvent to obtain a crude product. The crude product was purified by silica gel (200-300 mesh) column chromatography using PE:EA = 20:1 (v/v) to obtain 67 mg of 5-chloro-N-(3-cyanophenyl)-2-(4,4-difluoroazepan-1-yl)-6-(trifluoromethyl)nicotinami de **(49-b)** as a white solid in a yield of 89%. $^1H$ NMR (400 MHz, DMSO-$d_6$) δ: 10.94 (s, 1H), 8.14 (s, 2H), 7.95-7.87 (m, 1H), 7.60 (d, *J* = 6.3 Hz, 2H), 3.64 (m, 2H), 3.46 (t, *J* = 5.9 Hz, 2H), 2.32 (m, 3H), 1.97 (m, 2H), 1.86 (m, 2H); MS (ESI) m/z $[M+H]^+$ = 459.1.

Step 3: Following the process of Step 5 of Example 38, 5-chloro-N-(3-cyanophenyl)-2-(4,4-difluoroazepan-1-yl)-6-(trifluoromethyl)nicotinami de **(49-b)** (667 mg, 0.15 mmol) was used as a starting material to obtain 15 mg of 5-chloro-2-(4,4-difluoroazepan-1-yl)-N-(3-(N'-hydroxyamidino)phenyl)-6-(trifluorome thyl)benzamide (**I-49**) as a white solid in a yield of 21%. $^1$H NMR (400 MHz, DMSO-$d_6$) δ: 10.65(s, 1H), 9.65(s, 1H), 8.06(s,1H), 8.01(s,1H), 7.67(d, J = 7.6 Hz, 1H), 7.447.33(m, 2H), 5.77(s, 2H), 3.65 m, 2H), 3.49(m, 2H), 2.31(m, 2H), 1.99(m, 2H), 1.86(m, 2H); MS(ESI) m/z $[M+H]^+$ = 492.1.

**Test Example 1: Biological Testing**

Blocking Activity of Compounds of the Invention on Sodium Channel 1.8 (Nav1.8) **Test Method:** The effects of compounds on Nav1.8 channel currents were detected by using whole-cell manual patch clamp technique.

**1. Preparation and Analysis of Test Compounds**

**Control:** Electrophysiology extracellular solution containing 0.5% DMSO

**Test Compounds:** a certain amount of compound was weighed and dissolved in dimethyl sulfoxide (DMSO) to prepare a 20 mM DMSO stock solution. On the day of testing, the 20 mM compound stock solution was serially diluted with extracellular solution to the final concentration to be tested. The DMSO content in the test solution did not exceed 0.5%., since DMSO at this this concentration had no effect on the measurement of Nav1.8 channel currents. For example, to prepare 100 nM and 1 μM compound solutions, the serial dilution method was as follows: first, 5 μL of the DMSO stock solution was pipetted into 10 mL of extracellular fluid and dissolved evenly to obtain a 10 μM compound solution; then 1 mL of the 10 μM compound was pipetted into 9 mL of extracellular fluid and dissolved evenly to obtain a 1 μM compound solution; then 1 mL of the 1 μM compound was pipetted into 9 mL of extracellular fluid and dissolved evenly to obtain a 100 nM compound solution. VX-150 was used as all positive controls in this experiment, with a 50% inhibitory concentration of 33.45 ± 0.86 nM, consistent with the results reported in the original literature. The negative control used in this experiment was extracellular fluid containing 0.5% DMSO; the change in channel current was ≤5% 10 minutes after administration.

**2. Cell Culture**

(1) Nav1.8 Cell Line: HEK293 (Flp-In T-Rex-293) cells stably expressing the human Nav1.8 sodium channel, encoding gene: NM_001293306.2.

(2) Culture and Passaging Conditions and Methods: The cell line was cultured in a 37°C, 5% $CO_2$ incubator. Nav1.8 stable cell line was cultured in DMEM (Gibco) high-glucose complete medium supplemented with 10% tetracycline-free fetal bovine serum (HyClone) and 100 μg/mL Hygromycin B. The day before the experiment, cells were digested and passaged when they grew to a density of about 90%. First, the culture medium was aspirated and the cells were washed with phosphate buffered saline (PBS) preheated at 37°C. After discarding the PBS, trypsin was added for digestion and the cells were transferred to a centrifuge tube for centrifugation at 800 rpm for 3 minutes. The supernatant was discarded, and the cells were resuspended in complete culture medium containing 1 μg/mL Doxcycline. The cells were passaged into 6-well plates. After induction culture for 20 hours, the cells were separated and passaged onto coverslips coated with polylysine and cultured for 1-2 hours before being used for electrophysiological recording experiments.

**3. Electrophysiological experiments**

(1) Nav1.8 sodium channel currents were recorded using the whole-cell voltage clamp technique at room temperature (23~25°C).

(2) Whole-cell voltage clamp recording experiments were performed using an Axon patch 700B patch clamp amplifier (Molecular Devices) with a Digidata 1440A digital-to-analog converter (Molecular Devices), and glass microelectrodes were pulled from glass electrode blanks (World Precision Instruments) using a puller (P97, Sutter). After perfusion with electrode fluid, the tip resistance was approximately 1.5-2.5 MΩ. The glass microelectrode was connected to the patch clamp amplifier by inserting it into the amplifier headstage. Clamp voltage and data recording were controlled and recorded by a computer using pClamp 10 software (Molecular Devices), with a sampling frequency of 20 kHz and a filter frequency of 2 kHz.

(3) Extracellular and intracellular solutions for electrophysiological experiments:

Extracellular solution formulation (mM): 140 NaCl, 3 KCl, 1 $CaCl_2$, 1 $MgCl_2$, 10 HEPES and 20 Glucose, adjusted to pH 7.3 with NaOH.
Intracellular solution formulation (mM): 140 CsF, 10 NaCl, 10 HEPES, 1.1 EGTA and 20 Glucose, adjusted to pH 7.3 with CsOH.

(4) Electrophysiological stimulation protocol: After obtaining whole-cell recordings, -60 mV clamp voltage was applied for 4-5 minutes to equilibrate the electrode solution and intracellular solution, and then electrophysiological recordings were started. Current stimulation and compound activity detection protocol: Cells were clamped at -60 mV, stimulated for 20 ms with a depolarizing voltage of +10 mV, followed by repolarization to -60 mV, with a stimulation frequency of 0.5 Hz. After confirming that the Nav1.8 sodium channel current was stable (approximately 1 minute), drug administration was initiated and continued until the cell current ceased to change (compound inhibition reached steady-state). At least three cells were tested for each compound concentration ($n \geq 3$). A single concentration of 100 nM VX-150 was administered as a positive control after testing all compounds.

**4. Data Analysis**

Data acquisition and analysis were performed using pClamp10 (Molecular Devices), GraphPad Prism 5 (GraphPad Software), and Excel (Microsoft). All data are presented as mean ± standard error (SEM). The effect of the compound on the current was calculated using the following equation:

Inhibition (%) = [1 - Current after drug administration ($I_{Drug}$) / Current before drug administration ($I_{Control}$)] × 100.

Dose-effect curves were fitted using the Hill equation: Y = Bottom + (Top - Bottom) / (1 + 10 ^ (X - Log$IC_{50}$)), where Bottom and Top represent the minimum and maximum inhibition values, respectively, X represents the logarithm of compound concentration, Y represents $I_{Drug}/I_{Control}$, and $IC_{50}$ represents the drug dose that produces half-maximal inhibition.
The results are shown in Tables 1, 2, 3, and 4 below.

Table 1: $IC_{50}$ Values (nM) of Compounds of the Invention on Nav1.8 Channel Activity

| Compounds | $IC_{50}$ (nM) |
|---|---|
| I-12 | 0.26±0.06 nM |
| VX-150 | 33.45±0.86 nM |
| VX548 | 0.34±0.04 nM |

Table 2: Percent blocking activity of the compounds of the present invention on Nav1.8 channels at 1 nM

| Compounds | inhibition Percent @1 nM | Compounds | inhibition Percent @1 nM |
|---|---|---|---|
| I-1 | 71% | I-32 | 50% |
| I-2 | 79% | I-33 | 86% |
| I-5 | 82% | I-36 | 50% |
| I-6 | 65% | I-38 | 33% |
| I-8 | 51% | I-39 | 51% |
| I-10 | 32% | I-40-P1 | 59% |
| I-11 | 90% | I-40-P2 | 63% |
| I-12 | 73% | I-41-P1 | 38% |
| I-13 | 85% | I-41-P2 | 35% |
| I-15 | 66% | I-42 | 65% |
| I-19 | 49% | I-43 | 55% |
| I-24 | 56% | I-44 | 32% |
| I-31 | 46% | I-45 | 94% |

Table 3: Percent Blocking Activity of Compounds of the Present Invention on Nav1.8 Channels at 10 nM

| **Compounds** | **inhibition Percent @10 nM** | **Compounds** | **inhibition Percent @10 nM** |
|---|---|---|---|
| I-7 | 83% | I-46 | 50% |
| I-26 | 76% | I-47 | 80% |
| I-27 | 83% | I-48 | 77% |
| I-29 | 57% | I-49 | 77% |
| I-37 | 62% | | |

Table 4: Percent Blocking Activity of Some Compounds of the Present Invention on Nav1.8 Channels at 30 nM

| **Compounds** | **inhibition Percent @30 nM** | **Compounds** | **inhibition Percent@30 nM** |
|---|---|---|---|
| I-17 | 99% | I-18 | 90% |
| I7-b | 33% | I-23 | 95% |

**Test Example 2: Pharmacokinetics**

The in vivo pharmacokinetics in rats were evaluated via a single intravenous injection or oral gavage administration in this test example.

1. Test Methods and Conditions: Male SD rats were fasted overnight and administered a single intravenous dose of 2 mg/kg or 1 mg/kg of the test compound (solvent for Intravenous administration was 5% DMSO/10% Solutol® HS 15/10% EtOH/75% saline, with a dosing volume of 5 mL/kg) and an oral gavage dose of 10 mg/kg or 5 mg/kg of the test compound (solvent for administration was 0.5% CMC-Na, with a dosing volume of 10 mL/kg), respectively.
2. Sampling Information: Blood samples (0.2 mL) were collected from the retroorbital venous plexus at the designated time points: 0.25, 0.5, 1.0, 2.0, 3.0, 4.0, 6.0, 8.0, and 24 hours before and after dosing (a 5 min sampling point was added for the intravenous administration group), respectively. The samples were placed in EDTA-K2 tubes and centrifuged at 11,000 rpm for 5 minutes. Plasma was separated and frozen at -20°C.
3. Assay Information: LC/MS/MS was used to determine the concentration of the parent drug in rat/mouse plasma following intravenous and oral gavage administration of the test compound. Pharmacokinetic parameters in rat/mouse plasma were calculated for both administration routes, and the bioavailability of each compound in rats was also calculated. The results are shown in Tables 5 and 6 below.

Table 5: Intravenous Pharmacokinetic Information of Representative Compounds

| Compounds | Species/Dose (mg/kg) | $AUC_{0-t}$ (ng*hr/mL) | $t_{1/2}$ (h) | CL (mL/min/kg) | Vss (L/kg) |
|---|---|---|---|---|---|
| **I-1** | **Rat, mg/kg** | **1290** | **0.723** | **29.6** | **0.638** |
| **I-3** | **Rat, mg/kg** | **567** | **0.325** | **29.7** | **0.442** |
| **I-24** | **Rat, mg/kg** | **1260** | **2.0** | **12.8** | **1.38** |
| **I-33** | **Rat, mg/kg** | **2270** | **2.67** | **7.44** | **1.16** |
| **I-36** | **Rat, mg/kg** | **4440** | **3.28** | **3.91** | **0.823** |
| VX-150 | Rat, mg/kg | 2510 | 1.08 | 13.9 | 1.09 |
| VX-548 | Rat, mg/kg | 804 | 0.67 | 20.7 | 0.92 |

Table 6: Oral Gavage Pharmacokinetic Information of Representative Compounds

| Compounds | Species/Dose (mg/kg) | Tmax (h) | AUC0-t (ng*hr/mL) | Cmax (ng/mL) | $t_{1/2}$ (h) | F% |
|---|---|---|---|---|---|---|
| **I-1** | **Rat, 10mg/kg** | **0.5** | **546** | **433** | **1.49** | **8.7%** |
| **I-3** | **Rat, 5mg/kg** | **6.0** | **1770** | **381** | **2.08** | **62.4%** |

(continued)

| Compounds | Species/Dose (mg/kg) | Tmax (h) | AUC0-t (ng*hr/mL) | Cmax (ng/mL) | $t_{1/2}$ (h) | F% |
|---|---|---|---|---|---|---|
| **I-24** | **Rat, 5mg/kg** | **2.0** | **1950** | **244** | **2.73** | **30.95%** |
| **I-33** | **Rat, 5mg/kg** | **6.0** | **8640** | **623** | **5.12** | **38.9%** |
| **I-36** | **Rat, 5mg/kg** | **4.3** | **4410** | **386** | **3.15** | **38.9%** |
| VX-150 | Rat, 10mg/kg | 2.0 | 3810 | 723 | 2.88 | 30.4% |
| VX-548 | Rat, 5 mg/kg | 0.5 | 518 | 157 | 2.68 | 14% |

Note: VX-150 represents the compound with the structure of

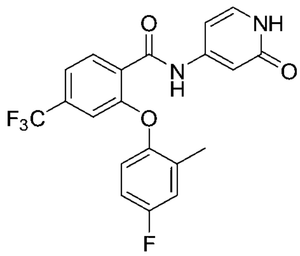

in WO2014120808. VX548 is a Phase III clinical trial drug targeting Nav1.8 with the structure of

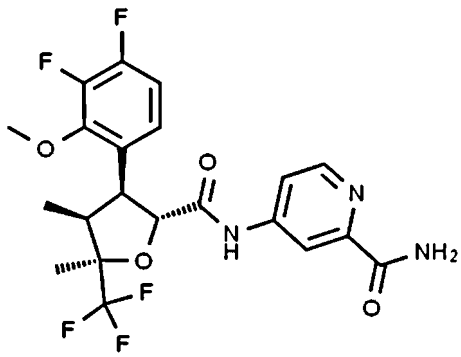

and CAS number 2649467-58-1.

The above examples are intended only to illustrate the technical solutions of the present invention and are not intended to limit the present invention. Although the present invention has been described in detail with reference to the aforementioned embodiments, those skilled in the art should understand that the technical solutions described in the aforementioned embodiments may be modified, or replaced some or all of the technical features therein with equivalents, without departing from the spirit and essence defined by the claims of the present invention; and these modifications or replacements are still within the scope defined by the claims of the present invention.

## Claims

1. A compound of Formula I, or a tautomer, stereoisomer, deuterated substance, or pharmaceutically acceptable salt thereof,

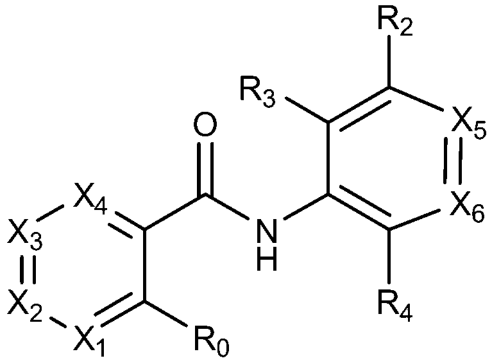

I

wherein:

$R_0$ is

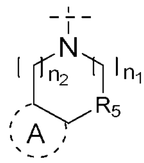 or 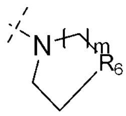 ,

Ring A is selected from substituted or unsubstituted C6-C10 aryl, and substituted or unsubstituted 5-10 membered heteroaryl, wherein the substituted means being substituted by 1, 2, 3 or 4 substituents selected from the group consisting of halogen, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkoxy;

$n_1$ is 0, 1, 2, 3 or 4; $n_2$ is 0 or 1; m is 0, 1, 2, 3 or 4;

$R_5$ is O, S, C=O, $CR_{5a}R_{5b}$ or $NR_{5c}$, wherein $R_{5a}$ and $R_{5b}$ are each independently selected from hydrogen and halogen; $R_{5c}$ is selected from hydrogen, substituted or unsubstituted C1-C4 alkyl, and substituted or unsubstituted C3-C6 cycloalkyl, wherein the substituted means being substituted by 1, 2, or 3 substituents selected from the group consisting of halogen, C1-C4 alkyl, halogenated C1-C4 alkyl, C1-C4 alkoxy, and halogenated C1-C4 alkoxy;

$R_6$ is O, S, C=O, $CR_{6a}R_{6b}$, or $NR_{6c}$, wherein $R_{6a}$ and $R_{6b}$ are each independently selected from hydrogen and halogen; or $CR_{6a}R_{6b}$ forms substituted or unsubstituted C3-C6 cycloalkyl, substituted or unsubstituted 3-8 membered heterocyclyl, [substituted or unsubstituted C3-C6 cycloalkyl] fused [substituted or unsubstituted phenyl]; [substituted or unsubstituted C3-C6 cycloalkyl] fused [substituted or unsubstituted 5-6 membered heteroaryl], [substituted or unsubstituted 3-8 membered heterocyclyl] fused [substituted or unsubstituted phenyl], [substituted or unsubstituted 3-8 membered heterocyclyl] fused [substituted or unsubstituted 5-6 membered heteroaryl], [substituted or unsubstituted cyclopentenyl] fused [substituted or unsubstituted phenyl], or [substituted or unsubstituted cyclopentenyl] fused [substituted or unsubstituted 5-6 membered heteroaryl]; $R_{6c}$ is selected from hydrogen, substituted or unsubstituted C1-C4 alkyl, and substituted or unsubstituted C3-C6 cycloalkyl, wherein the substituted means being substituted by 1, 2 or 3 substituents selected from the group consisting of halogen, C1-C4 alkyl, halogenated C1-C4 alkyl, C1-C4 alkoxy, and halogenated C1-C4 alkoxy;

$X_1$ is N or $CR_a$, $X_2$ is N or $CR_b$, $X_3$ is N or $CR_c$, $X_4$ is N or $CR_d$, and at most two N atoms are present in $X_1$, $X_2$, $X_3$, and $X_4$;

$R_a$, $R_b$, $R_c$, and $R_d$, at each occurrence, are independently selected from hydrogen, halogen, cyano, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkoxy; in particular, $R_a$ is hydrogen; $R_b$ is selected from $-CF_3$, methyl, cyclopropyl, isopropyl, and -Cl; $R_c$ is selected from -Cl, $-CF_3$, cyano, and ethynyl; $R_d$ is selected from hydrogen, -Cl, and -F;

one of $X_5$ and $X_6$ is $CR_1$, and the other is N or $CR_e$;

$R_e$, at each occurrence, is selected from hydrogen, halogen, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkoxy; preferably selected from -Cl, -F, Br, hydrogen, methoxy, and methyl;
$R_1$ is

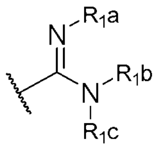 , 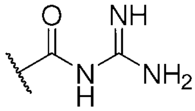 , 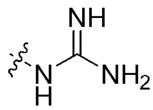 or 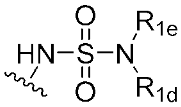 ;

or $R_1$ and $R_e$, together with the carbon to which they are attached, form

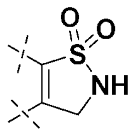 ;

wherein, $R_{1a}$ is selected from hydrogen, -CN, -OH, and C1-C6 alkoxy; $R_{1b}$, $R_{1c}$, $R_{1e}$, and $R_{1d}$ are each independently selected from hydrogen, C1-C6 alkyl, and C3-C6 cycloalkyl;
$R_2$, $R_3$, and $R_4$ are each independently selected from hydrogen, halogen, substituted or unsubstituted C1-C3 alkyl, substituted or unsubstituted C1-C3 alkoxy, and substituted or unsubstituted C3-C6 cycloalkyl, wherein the substituted means being substituted by 1, 2, or 3 substituents selected from the group consisting of C1-C4 alkyl, halogen, -CN, $-NO_2$ and $-NH_2$.

**2.** The compound or the tautomer, stereoisomer, deuterated substance, or pharmaceutically acceptable salt thereof according to claim 1, wherein
$R_0$ is

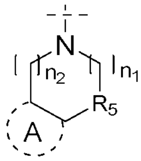 ,

and is selected from substituted or unsubstituted indolinyl, substituted or unsubstituted isoindolinyl, substituted or unsubstituted 1,2,3,4-tetrahydroquinolinyl, substituted or unsubstituted tetrahydroisoquinolinyl, substituted or unsubstituted 4,5,6,7-tetrahydrothieno[3,2-c]pyridinyl, and substituted or unsubstituted 4,5,6,7-tetrahydrothieno[2,3-c] pyridinyl, wherein the substituent for the substituted is selected from the group consisting of halogen, C1-C4 alkyl, C1-C4 alkoxy, phenyl, and halogenated C1-C4 alkyl.

**3.** The compound or the tautomer, stereoisomer, deuterated substance, or pharmaceutically acceptable salt thereof according to claim 1, wherein

$R_0$ is

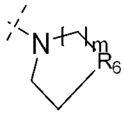 ,

and is selected from substituted or unsubstituted tetrahydropyrrolyl, substituted or unsubstituted piperidinyl, substituted or unsubstituted morpholinyl, substituted or unsubstituted thiomorpholinyl, substituted or unsubstituted piperazinyl, substituted or unsubstituted azepanyl, substituted or unsubstituted 1,4-oxazepanyl, substituted or unsubstituted azaspiro[2.5]octanyl, substituted or unsubstituted spiro[indene-1,4'-piperidinyl], substituted or unsubstituted 4,5-dihydrospiro[piperidin-4,7'-thieno[2,3-c]pyranyl], and substituted or unsubstituted 3H-spiro[isobenzofuran-1,4'-piperidinyl], wherein the substituent for the substituted is selected from the group consisting of halogen, C1-C4 alkyl, C1-C4 alkoxy, phenyl and halogenated C1-C4 alkyl;
preferably, $R_0$ is selected from the following groups:

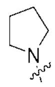 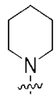 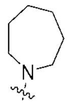 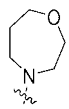 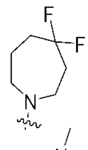 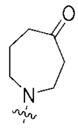 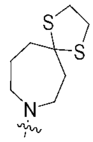

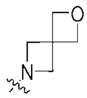 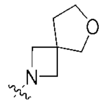 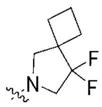 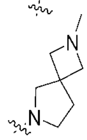 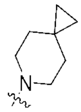 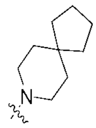

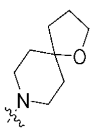 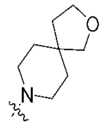 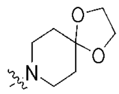 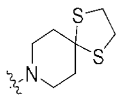 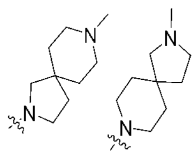 .

4. The compound or the tautomer, stereoisomer, deuterated substance, or pharmaceutically acceptable salt thereof according to claim 1, wherein the compound of formula I is selected from the compounds of following formula I-a1, formula I-a2, formula I-b1, formula I-b2, formula I-c1, formula I-c2, and formula I-d:

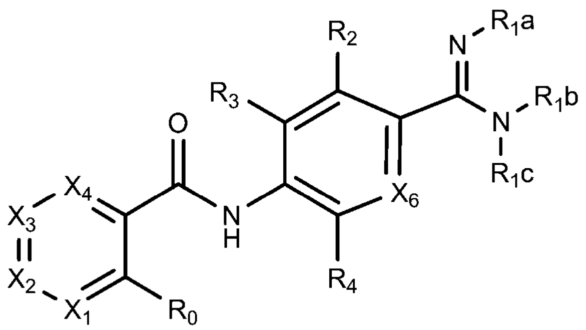

I-a1

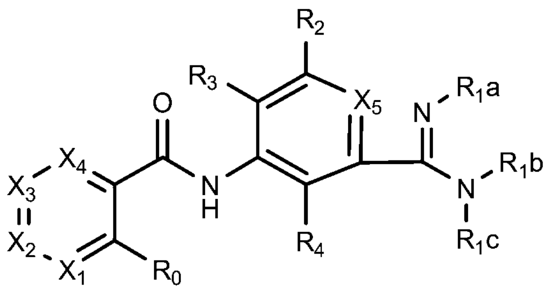

I-a2

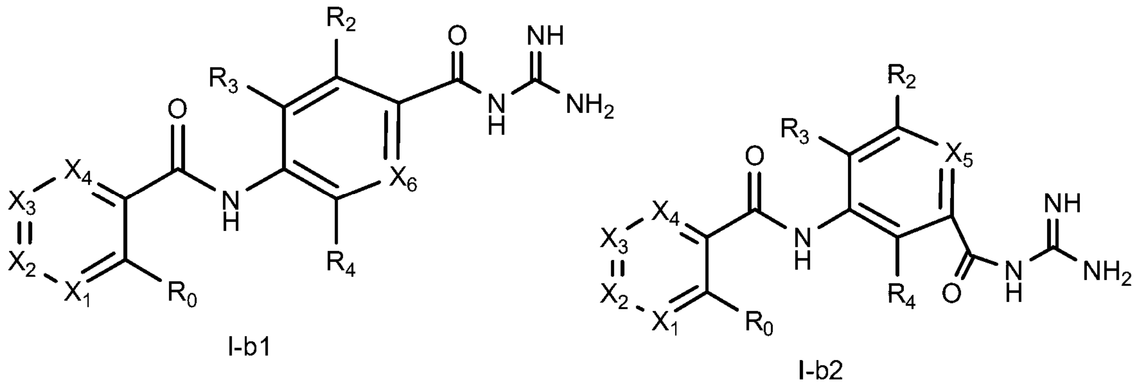

I-b1

I-b2

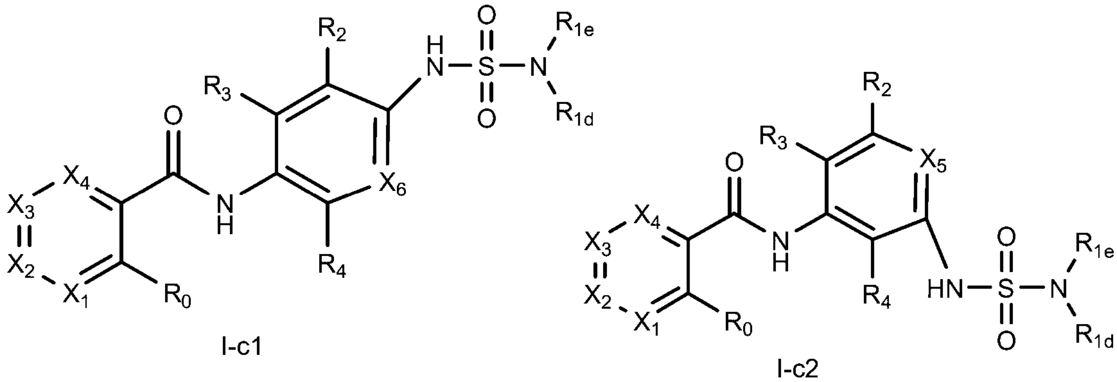

I-c1

I-c2

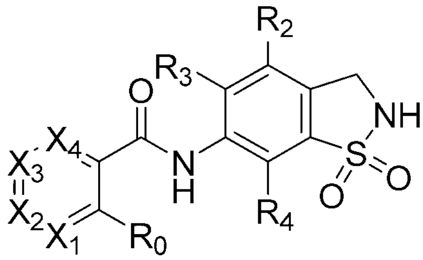

1-d

wherein, $X_5$ and $X_6$ are N or $CR_e$, and $X_1$, $X_2$, $X_3$, $X_4$, $R_0$, $R_{1a}$, $R_{1b}$, $R_{1c}$, $R_{1d}$, $R_{1e}$, $R_2$, $R_3$, $R_4$, and $R_e$ are defined as those in the corresponding claim.

**5.** The compound or the tautomer, stereoisomer, deuterated substance, or pharmaceutically acceptable salt thereof according to any one of claims 1 to 4, wherein the compound of Formula I is selected from the compounds of following Formula II:

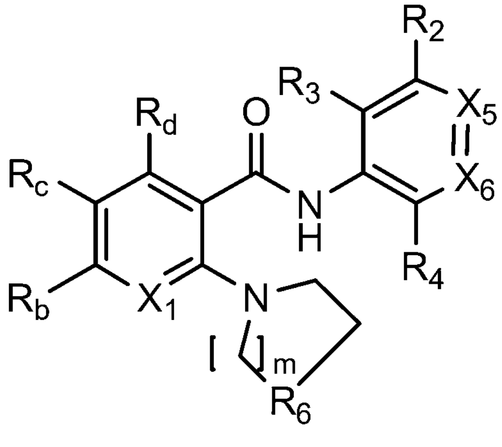

II

wherein, $X_1$ is C or N; m, $R_6$, $R_b$, $R_c$, $R_d$, $R_2$, $R_3$, $R_4$, $X_5$, and $X_6$ are defined as those in the corresponding claim; preferably, the compound of Formula II is selected from the compounds of following Formula II-a1, Formula II-a2, Formula II-b1, Formula II-b2, Formula II-c1, Formula II-c2, and Formula II-d:

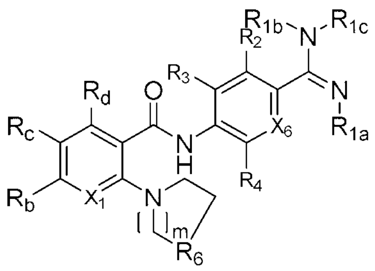

II-a1

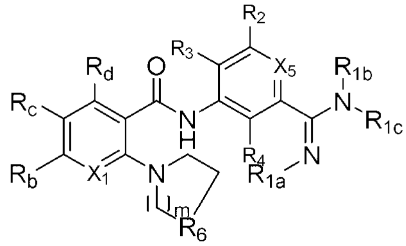

II-a2

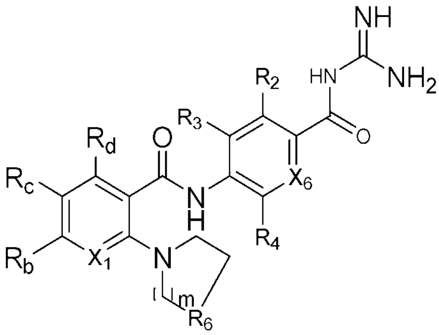

II-b1

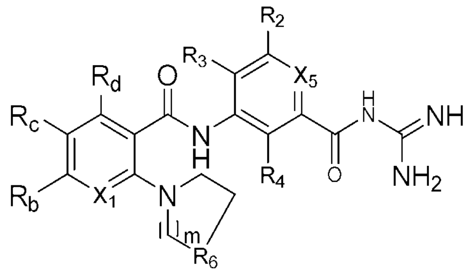

II-b2

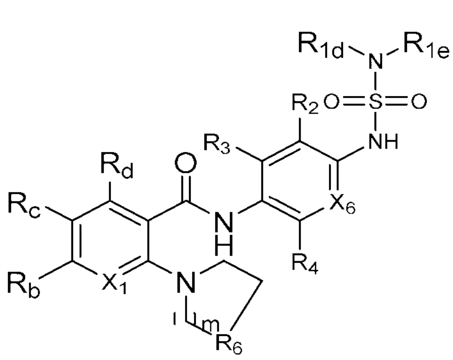

II-c1

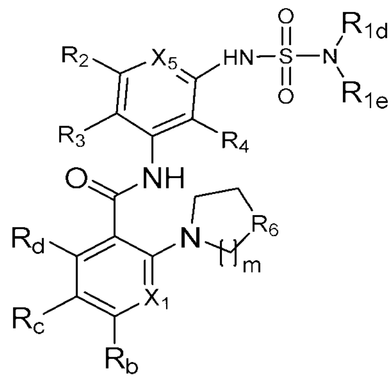

II-c2

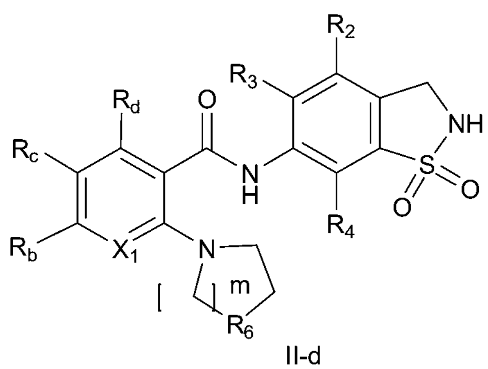

II-d

wherein, $X_1$, m, $R_6$, $R_b$, $R_c$, $R_d$, $R_2$, $R_3$, $R_4$, $X_5$, $X_6$, $R_{1a}$, $R_{1b}$, $R_{1c}$, $R_{1d}$, and $R_{1e}$ are defined as those in the corresponding claim;

Preferably, the compounds of Formulas II-a1 and II-a2 are selected from the compounds of following Formula II-a1-1 or Formula II-a2-1:

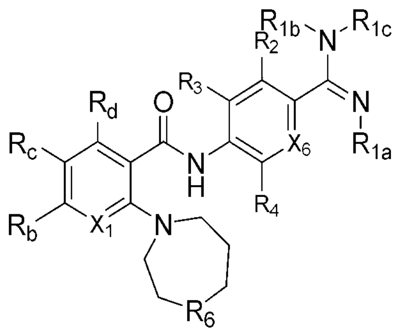

**II-a1-1**

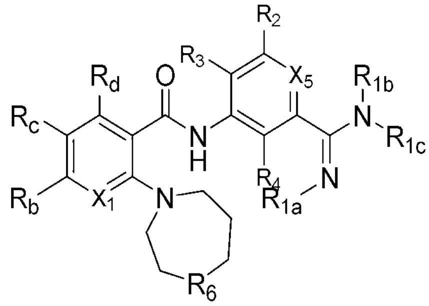

**II-a2-1**

wherein,

$R_{1a}$ is hydrogen, -CN, -OH, or C1-C6 alkoxy, preferably -OH;

$R_{1b}$ and $R_{1c}$ are each independently selected from hydrogen, and C1-C6 alkyl, preferably hydrogen;

$R_6$ is $-CH_2$, -C=O, or $-CF_2$;

$R_b$, $R_c$, and $R_d$ are each independently selected from hydrogen, halogen, cyano, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkoxy; preferably, $R_b$, $R_c$, and $R_d$ are each independently selected from hydrogen, chlorine, fluorine, C1-C4 alkyl, C2-C4 alkynyl, C3-C6 cycloalkyl, and trifluoromethyl;

$X_1$ is N or CH ;

$X_5$ and $X_6$ are N or $CR_e$, wherein $R_e$, at each occurrence, is selected from hydrogen, halogen, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkyloxy; preferably, Re, at each occurrence, is selected from hydrogen, fluorine, chlorine, bromine, methoxy, and methyl;

$R_2$, $R_3$, and $R_4$ are each independently selected from hydrogen, halogen, substituted or unsubstituted C1-C3 alkyl, substituted or unsubstituted C1-C3 alkoxy, and substituted or unsubstituted C3-C6 cycloalkyl, wherein the substituted means being substituted by 1, 2, or 3 substituents selected from the group consisting of methyl, -F, -CN, $-NO_2$, and $-NH_2$;

preferably, the compound of formula II-a2-1 is selected from the compounds of following formula II-a2-1-A:

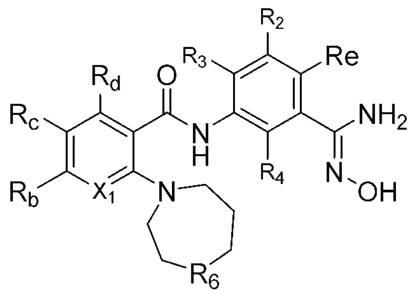

**II-a2-1-A**

$R_e$ is hydrogen, F, Cl, Br, C1-C6 alkyl, or C1-C6 alkoxy, preferably hydrogen, F, Cl, or Br, more preferably hydrogen or F, especially F;

$R_6$ is $-CH_2$, -C=O, or $-CF_2$;

$R_b$, $R_c$, and $R_d$ are each independently selected from hydrogen, halogen, cyano, C1-C6 alkyl, halogenated C1-C6 alkyl, C1-C6 alkoxy, halogenated C1-C6 alkoxy, C2-C6 alkenyl, halogenated C2-C6 alkenyl, C2-C6 alkenyloxy, halogenated C2-C6 alkenyloxy, C2-C6 alkynyl, halogenated C2-C6 alkynyl, C2-C6 alkynyloxy, halogenated C2-C6 alkynyloxy, C3-C6 cycloalkyl, and C3-C6 cycloalkyloxy; preferably, $R_b$, $R_c$, and $R_d$ are each independently selected from hydrogen, chlorine, fluorine, C1-C4 alkyl, C2-C4 alkynyl, C3-C6 cycloalkyl, and trifluoromethyl;

$X_1$ is N or CH;
$R_2$, $R_3$, and $R_4$ are defined as those in the corresponding claim.

**6.** The compound or the tautomer, stereoisomer, deuterated substance, or pharmaceutically acceptable salt thereof according to any one of claims 1 to 4, wherein the compound of formula I is selected from the following compounds:

| | |
|---|---|
| 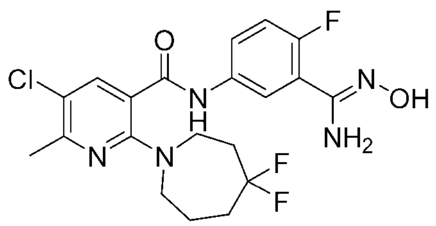<br>I-1 | 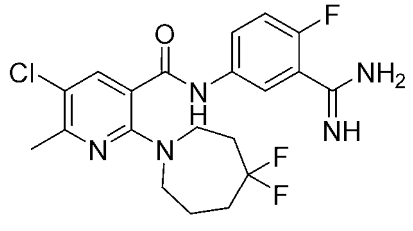<br>I-2 |
| 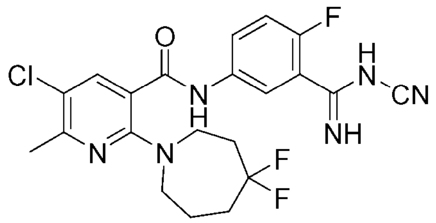<br>I-3 | 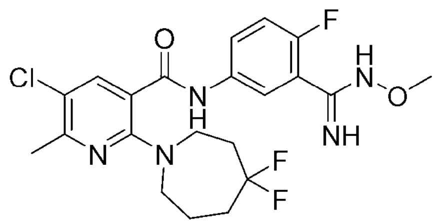<br>I-4 |
| 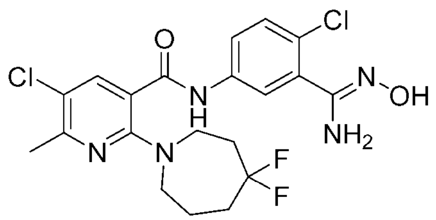<br>I-5 | 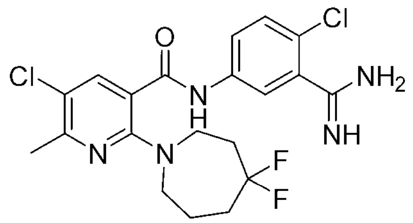<br>I-6 |
| 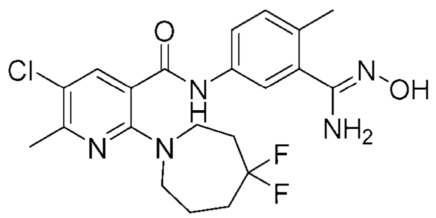<br>I-7 | 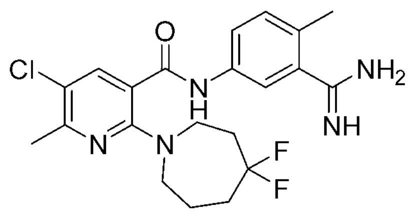<br>I-8 |
| 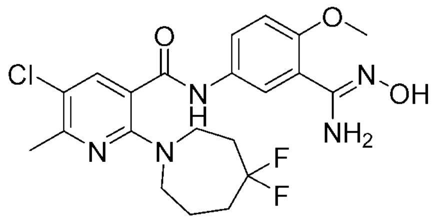<br>I-9 | 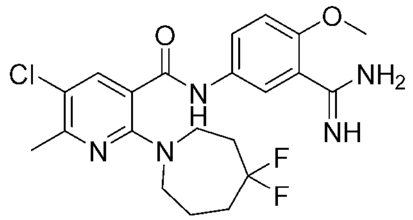<br>I-10 |
| 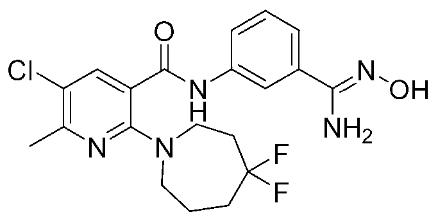<br>I-11 | 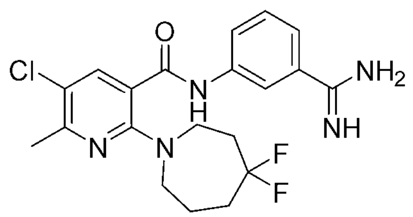<br>I-12 |

| | |
|---|---|
| 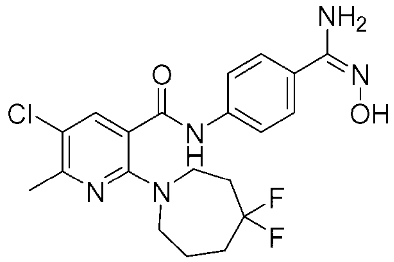<br>I-13 | 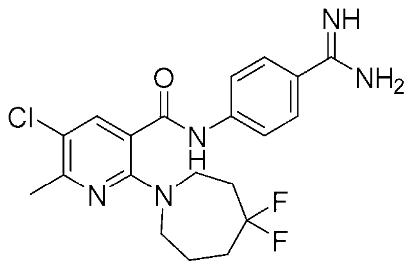<br>I-14 |
| 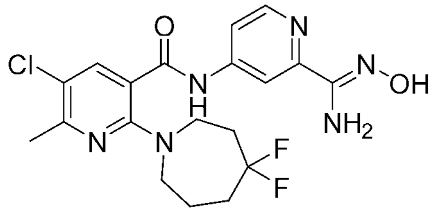<br>I-15 | 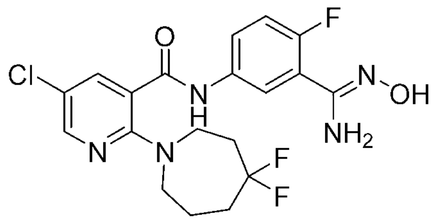<br>I-16 |
| 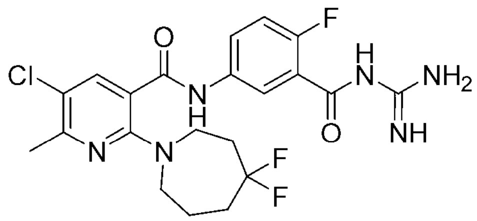<br>I-17 | 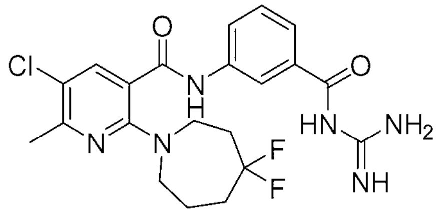<br>I-18 |
| 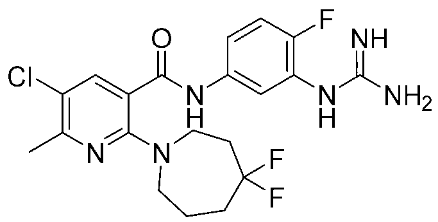<br>I-19 | 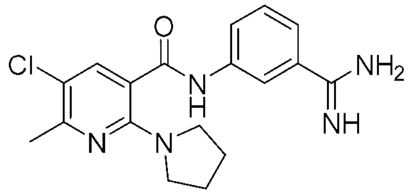<br>I-20 |
| 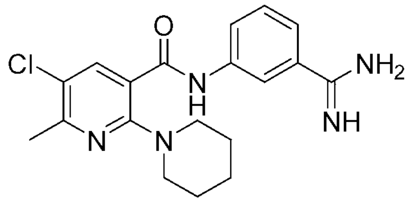<br>I-21 | 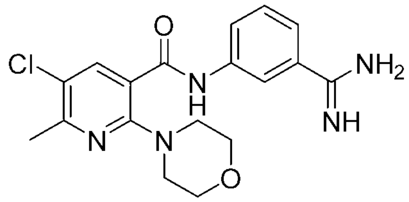<br>I-22 |
| 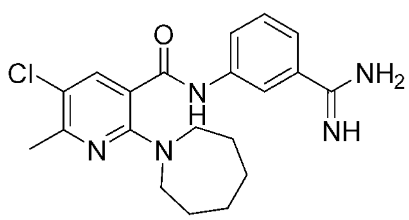<br>I-23 | 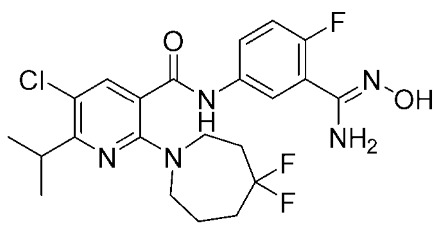<br>I-24 |

| | |
|---|---|
| 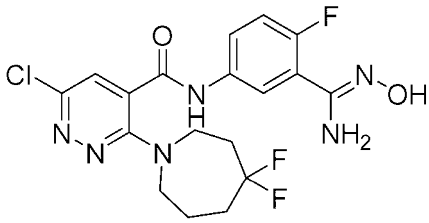 I-25 | 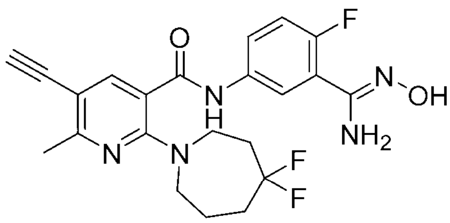 I-26 |
| 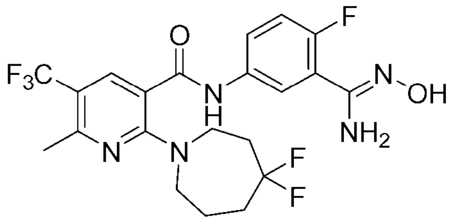 I-27 | 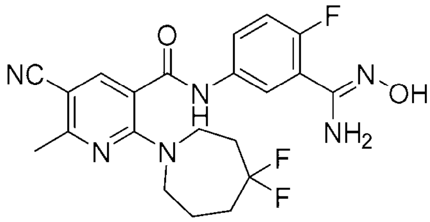 I-28 |
| 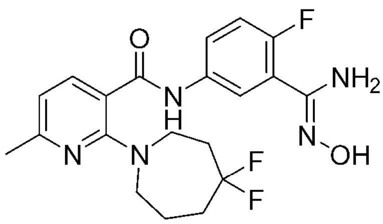 I-29 | 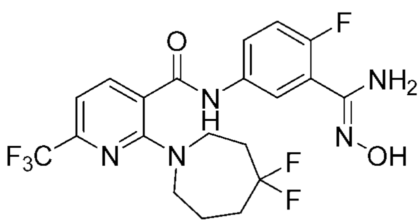 I-30 |
| 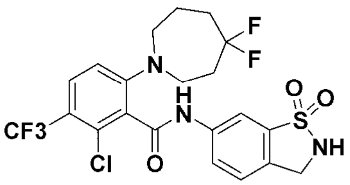 I-31 | 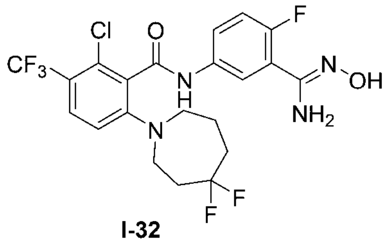 I-32 |
| 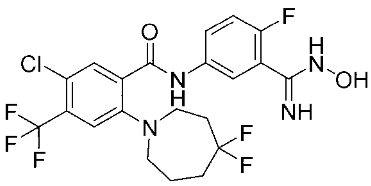 I-33 | 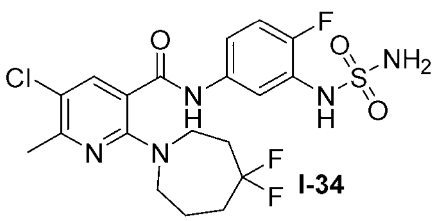 I-34 |
| 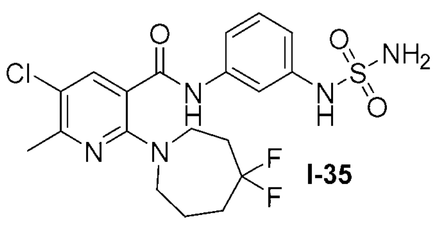 I-35 | 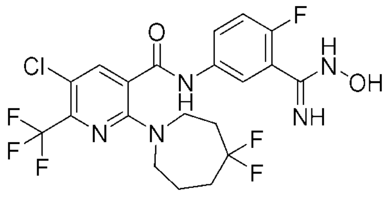 I-36 |

| | |
|---|---|
| 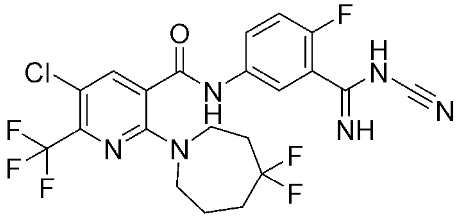 **I-37** | 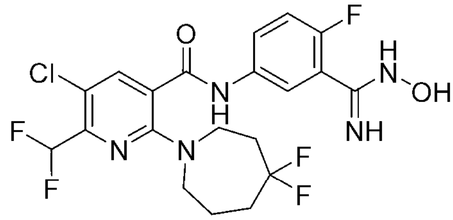 I-38 |
| 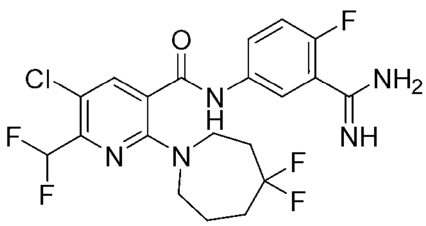 **I-39** | 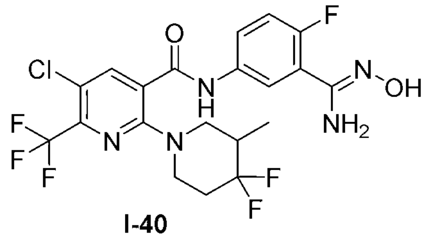 **I-40** |
| 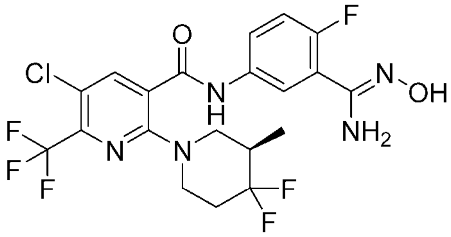 | 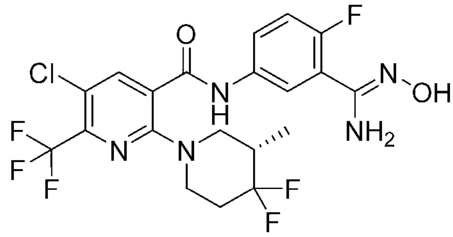 |
| 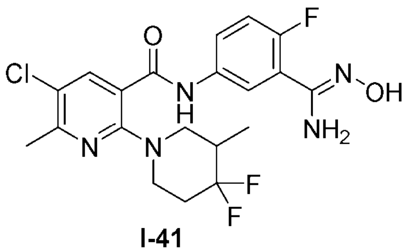 **I-41** | 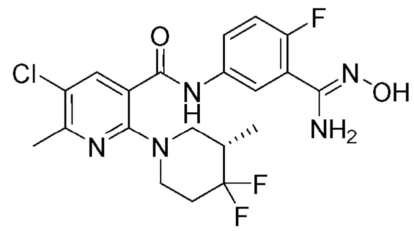 |
| 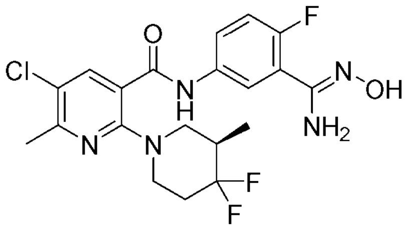 | 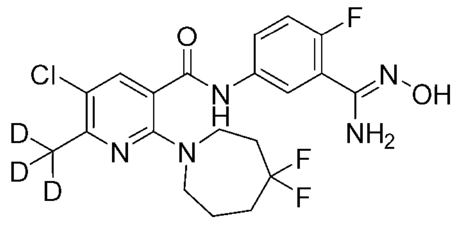 **I-42** |
| 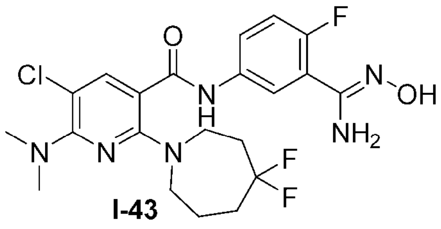 **I-43** | 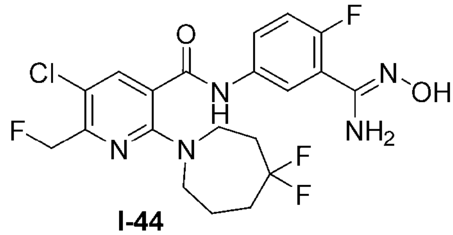 **I-44** |

| | |
|---|---|
| 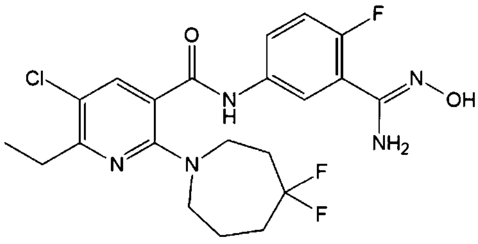 I-45 | 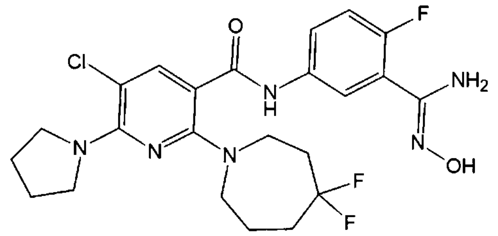 I-46 |
| 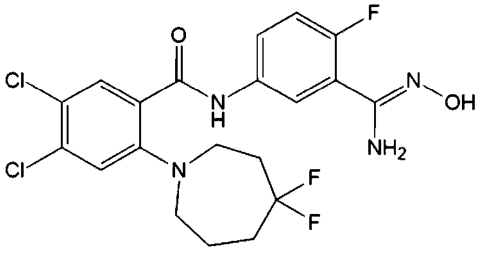 I-47 | 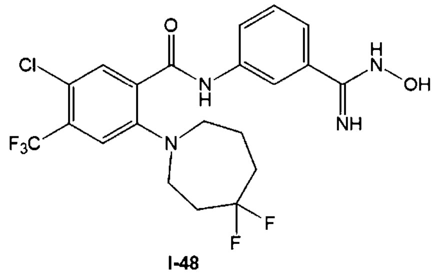 I-48 |
| 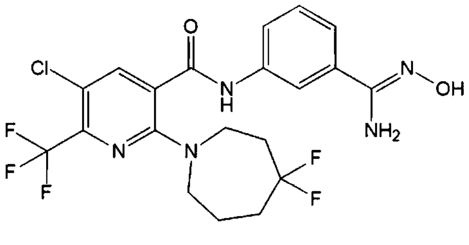 I-49 | |

7. A method for preparing the compound of any one of claims 1 to 6, which is achieved via the following reaction routes:

Route 1:

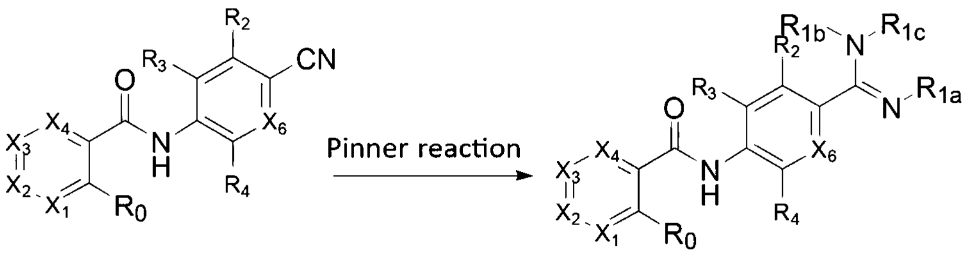


III-a1 I-a1

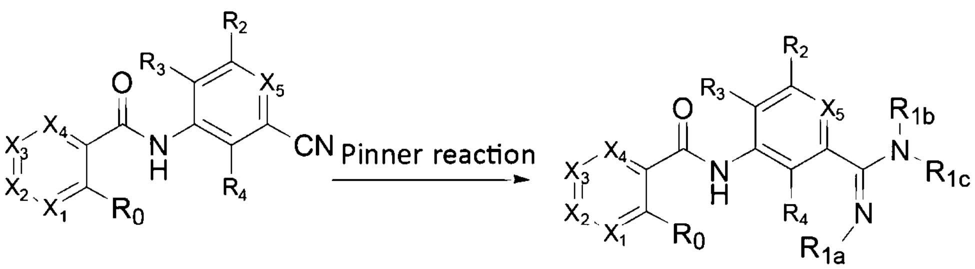


III-a2 I-a2

**III-a1** or **III-a2** undergoes a Pinner reaction to form a Pinner salt, which is then reacted with $NR_{1b}R_{1c}$ and $NR_{1a}$ to prepare **I-a1** or **I-a2;**
wherein, each of the substituents is defined as that in the corresponding claim;

Route 2:

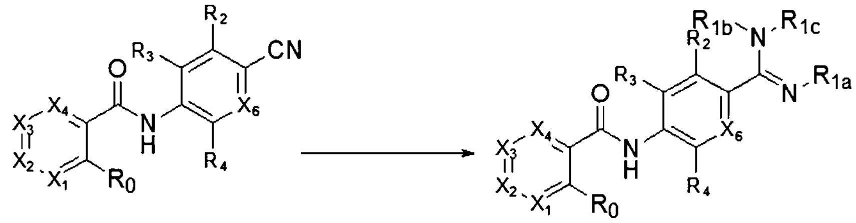

**III-a1** **I-a1** $R_{1a}$=OH,$R_{1b}$=H,$R_{1c}$=H

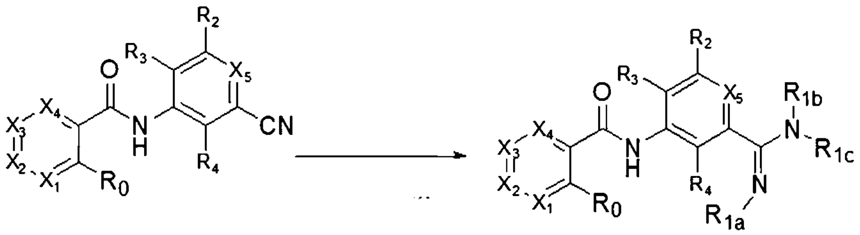

**III-a2**

**I-a2**

$R_{1a}$=OH,$R_{1b}$=H,$R_{1c}$=H

**III-a1** or **III-a2** is reacted with hydroxylamine or a salt of hydroxylamine to obtain **I-a1** or **I-a2;**
wherein, $R_{1a}$ is OH, $R_{1b}$ is H, $R_{1c}$ is H, and the remaining substituents are defined as those in the corresponding claim;

Route 3:

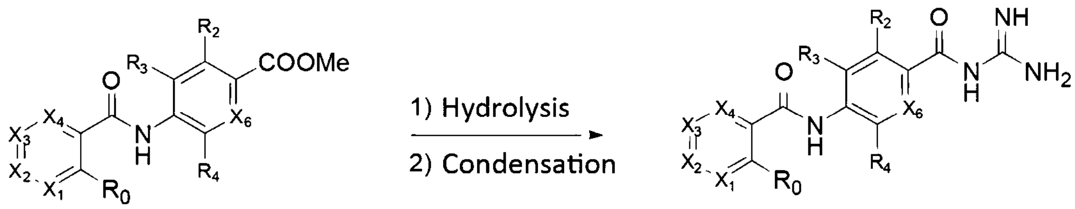


**III-a3** **I-b1**

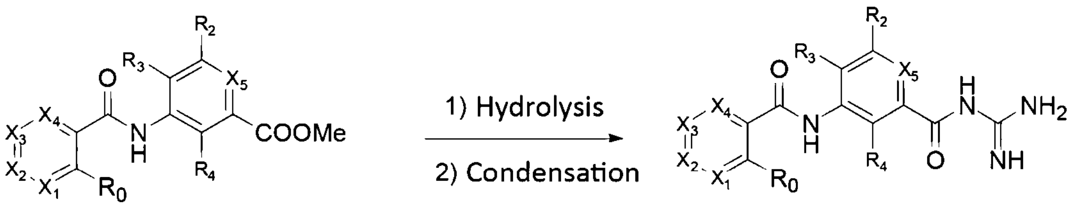


**III-a4** **I-b2**

**III-a3** or **III-a4** is hydrolyzed to produce an acid, which is then converted to an acid chloride, the acid chloride is

then reacted with guanidine via condensation to produce **I-b1** or **I-b2;** wherein, each of the substituents is defined as that in the corresponding claim;

Route 4:

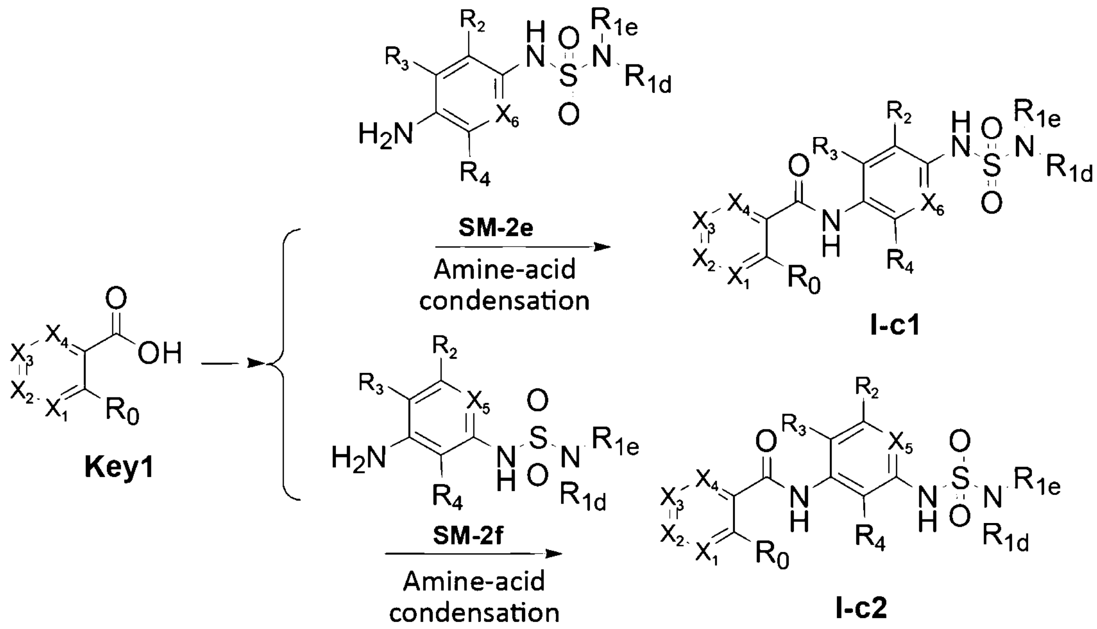


**Key1** is reacted with **SM-2e** or **SM-2f** via amine-acid condensation to produce **I-c1** or **I-c2;** wherein, each of the substituents is defined as that in the corresponding claim;

Route 5:

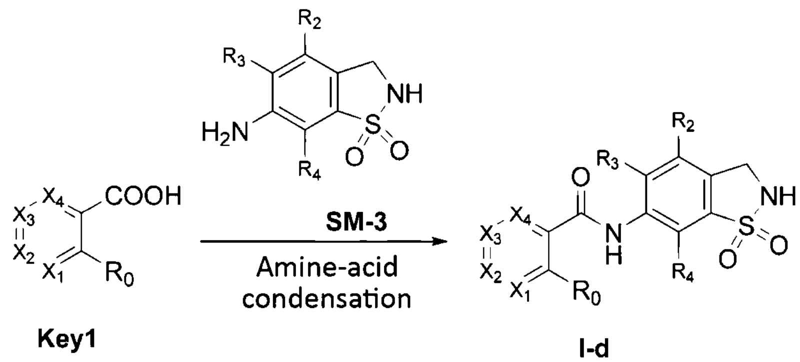


**Key1** is reacted with **SM-3** via amine-acid condensation to produce I-d; wherein, each of the substituents is defined as that in corresponding claim.

**8.** A pharmaceutical composition comprising one or more compounds selected from the compound, the tautomer, stereoisomer, deuterated substance, and pharmaceutically acceptable salt thereof of any one of claims 1 to 6, and optionally, a pharmaceutically acceptable excipient.

**9.** The compound, or the tautomer, stereoisomer, deuterated substance, or pharmaceutically acceptable salt thereof of any one of claims 1 to 6, or the pharmaceutical composition of claim 8, for use in preparation of a Nav1.8 inhibitor.

**10.** The compound, or the tautomer, stereoisomer, deuterated substance, or pharmaceutically acceptable salt thereof of any one of claims 1 to 6, or the pharmaceutical composition of claim 8, for use in preparation of a medicament for preventing and/or treating diseases associated with abnormal expression of Nav1.8 channel,
preferably, the diseases associated with abnormal expression of Nav1.8 channel include pain, pruritus, acute or chronic pruritus, asthma, multiple sclerosis, arrhythmias, atrial fibrillation, heart failure, Brugada syndrome, kidney

stones, epilepsy, convulsions, Charcot-Marie-Tooth syndrome, and incontinence.

## INTERNATIONAL SEARCH REPORT

International application No.
**PCT/CN2024/087393**

**A. CLASSIFICATION OF SUBJECT MATTER**

C07D401/04(2006.01)i; C07D401/12(2006.01)i; C07D213/78(2006.01)i; C07D213/82(2006.01)i; C07D317/68(2006.01)i; C07C257/18(2006.01)i; C07C259/18(2006.01)i; C07C257/20(2006.01)i; C07C261/04(2006.01)i; A61K31/44(2006.01)i; A61K31/36(2006.01)i; A61K31/167(2006.01)i; A61K31/277(2006.01)i; A61P29/00(2006.01)i; A61P25/04(2006.01)i; A61P19/02(2006.01)i

According to International Patent Classification (IPC) or to both national classification and IPC

**B. FIELDS SEARCHED**

Minimum documentation searched (classification system followed by classification symbols)

C07D,C07C,A61K,A61P

Documentation searched other than minimum documentation to the extent that such documents are included in the fields searched

Electronic data base consulted during the international search (name of data base and, where practicable, search terms used)

CNKI, CNTXT, ENTXT, DWPI, STN (REG, CAPLUS): 上海药物研究所, 电压门控钠离子通道, 钠通道, Nav1.8

**C. DOCUMENTS CONSIDERED TO BE RELEVANT**

| Category* | Citation of document, with indication, where appropriate, of the relevant passages | Relevant to claim No. |
|---|---|---|
| A | CN 101466665 A (VERTEX PHARMACEUTICALS, INC.) 24 June 2009 (2009-06-24)<br>entire document | 1-10 |
| A | CN 101855210 A (VERTEX PHARMACEUTICALS, INC.) 06 October 2010 (2010-10-06)<br>entire document | 1-10 |
| A | CN 105683157 A (VERTEX PHARMACEUTICALS, INC.) 15 June 2016 (2016-06-15)<br>entire document | 1-10 |
| Y | CN 112457294 A (SHANGHAI JEMINCARE PHARMACEUTICALS CO., LTD. et al.) 09 March 2021 (2021-03-09)<br>claims 1-13, and embodiments | 1-10 |
| A | CN 113272293 A (MERCK SHARP & DOHME CORP.) 17 August 2021 (2021-08-17)<br>entire document | 1-10 |
| A | CN 114031518 A (CHENGDU HYPERWAY PHARMACEUTICAL CO., LTD. et al.) 11 February 2022 (2022-02-11)<br>entire document | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 June 2024** | **24 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/087393**

**C. DOCUMENTS CONSIDERED TO BE RELEVANT**

| Category* | Citation of document, with indication, where appropriate, of the relevant passages | Relevant to claim No. |
|---|---|---|
| Y | CN 114437062 A (CHENGDU HYPERWAY PHARMACEUTICAL CO., LTD. et al.) 06 May 2022 (2022-05-06)<br>claims 1-32, and embodiments | 1-10 |
| PY | CN 116462662 A (CHENGDU KANGHONG PHARMACEUTICAL GROUP CO., LTD.) 21 July 2023 (2023-07-21)<br>claims 1-29, and embodiments | 1-10 |
| PY | CN 116655497 A (SHANGHAI INSTITUTE OF MATERIA MEDICA, CHINESE ACADEMY OF SCIENCES) 29 August 2023 (2023-08-29)<br>claims | 1-10 |
| PY | CN 116789644 A (SHANGHAI INSTITUTE OF MATERIA MEDICA, CHINESE ACADEMY OF SCIENCES) 22 September 2023 (2023-09-22)<br>claims | 1-10 |
| A | US 2007238733 A1 (JOSHI PRAMOD et al.) 11 October 2007 (2007-10-11)<br>entire document | 1-10 |
| Y | WO 2019014352 A1 (VERTEX PHARMACEUTICALS, INC.) 17 January 2019 (2019-01-17)<br>claims, and embodiments | 1-10 |
| X | WO 2020092187 A1 (MERCK SHARP & DOHME CORP. et al.) 07 May 2020 (2020-05-07)<br>embodiments 111-113 and 122 | 1-10 |
| A | WO 2022192487 A2 (LATIGO BIOTHERAPEUTICS, INC. et al.) 15 September 2022 (2022-09-15)<br>entire document | 1-10 |
| PY | WO 2023150201 A2 (LATIGO BIOTHERAPEUTICS, INC.) 10 August 2023 (2023-08-10)<br>embodiments, and claims 1-33 | 1-10 |
| PY | WO 2024032774 A1 (GUANGZHOU FERMION TECHNOLOGY CO., LTD.) 15 February 2024 (2024-02-15)<br>claims 1-39, and embodiments | 1-10 |
| Y | WO 2020092187 A1 (MERCK SHARP & DOHME CORP.; BRESLIN MICHAEL J. et al.) 07 May 2020 (2020-05-07)<br>embodiments 111-113 and 122 | 1-10 |

Form PCT/ISA/210 (second sheet) (July 2022)

**INTERNATIONAL SEARCH REPORT**
**Information on patent family members**

International application No.
**PCT/CN2024/087393**

| Patent document cited in search report | Publication date (day/month/year) | Patent family member(s) | Publication date (day/month/year) |
|---|---|---|---|
| CN 101466665 A | 24 June 2009 | NO 20084758 L | 09 January 2009 |
| | | CA 2648202 A1 | 25 October 2007 |
| | | WO 2007120647 A2 | 25 October 2007 |
| | | WO 2007120647 A3 | 29 November 2007 |
| | | RU 2008144415 A | 20 May 2010 |
| | | RU 2470012 C2 | 20 December 2012 |
| | | NZ 596413 A | 28 March 2013 |
| | | ZA 200808751 B | 30 December 2009 |
| | | AU 2007238857 A1 | 25 October 2007 |
| | | AU 2007238857 B2 | 02 May 2013 |
| | | EP 2004596 A2 | 24 December 2008 |
| | | JP 2009536616 A | 15 October 2009 |
| | | JP 5438504 B2 | 12 March 2014 |
| | | US 2007238733 A1 | 11 October 2007 |
| | | US 8841483 B2 | 23 September 2014 |
| | | MX 2008013194 A | 01 December 2008 |
| | | KR 20080109918 A | 17 December 2008 |
| CN 101855210 A | 06 October 2010 | ZA 201002471 B | 29 June 2011 |
| | | EP 2212292 A1 | 04 August 2010 |
| | | EP 2212292 B1 | 05 December 2012 |
| | | RU 2010118481 A | 20 November 2011 |
| | | KR 20100066583 A | 17 June 2010 |
| | | AU 2008310661 A1 | 16 April 2009 |
| | | NZ 584474 A | 29 June 2012 |
| | | JP 2011500599 A | 06 January 2011 |
| | | JP 5436434 B2 | 05 March 2014 |
| | | CA 2701766 A1 | 16 April 2009 |
| | | IL 204977 A0 | 30 November 2010 |
| | | US 2013231370 A1 | 05 September 2013 |
| | | US 8865771 B2 | 21 October 2014 |
| | | US 2009118333 A1 | 07 May 2009 |
| | | US 8389734 B2 | 05 March 2013 |
| | | MX 2010003864 A | 01 June 2010 |
| | | WO 2009049181 A1 | 16 April 2009 |
| CN 105683157 A | 15 June 2016 | CA 2918365 A1 | 22 January 2015 |
| | | CA 2918365 C | 07 September 2021 |
| | | MX 2016000646 A | 26 May 2016 |
| | | MX 364155 B | 12 April 2019 |
| | | WO 2015010065 A1 | 22 January 2015 |
| | | PE 20160548 A1 | 21 May 2016 |
| | | NZ 715751 A | 24 September 2021 |
| | | JP 2016525122 A | 22 August 2016 |
| | | JP 6337109 B2 | 06 June 2018 |
| | | SG 11201600383 SA | 26 February 2016 |
| | | HK 1223604 A1 | 04 August 2017 |
| | | AU 2014290411 A1 | 28 January 2016 |
| | | AU 2014290411 B2 | 15 November 2018 |
| | | ZA 201600380 B | 25 July 2018 |
| | | ECSP 16005566 A | 24 February 2017 |
| | | GEAP 202014060 A | 27 January 2020 |

Form PCT/ISA/210 (patent family annex) (July 2022)

**INTERNATIONAL SEARCH REPORT**
**Information on patent family members**

International application No.
**PCT/CN2024/087393**

| Patent document cited in search report | | | Publication date (day/month/year) | Patent family member(s) | | | Publication date (day/month/year) |
|---|---|---|---|---|---|---|---|
| | | | | GEP | 20207102 | B | 11 May 2020 |
| | | | | PH | 12016500105 | A1 | 18 April 2016 |
| | | | | RU | 2016105511 | A | 24 August 2017 |
| | | | | RU | 2016105511 | A3 | 25 April 2018 |
| | | | | RU | 2680401 | C2 | 21 February 2019 |
| | | | | US | 2016152561 | A1 | 02 June 2016 |
| | | | | US | 11203571 | B2 | 21 December 2021 |
| | | | | HUE | 037876 | T2 | 28 September 2018 |
| | | | | CL | 2016000106 | A1 | 29 July 2016 |
| | | | | ES | 2654393 | T3 | 13 February 2018 |
| | | | | KR | 20160032241 | A | 23 March 2016 |
| | | | | KR | 102215620 | B1 | 16 February 2021 |
| | | | | EP | 3022175 | A1 | 25 May 2016 |
| | | | | EP | 3022175 | B1 | 08 November 2017 |
| | | | | BR | 112016000825 | A2 | 25 July 2017 |
| | | | | BR | 112016000825 | A8 | 07 January 2020 |
| | | | | BR | 112016000825 | B1 | 25 April 2023 |
| | | | | UA | 119147 | C2 | 10 May 2019 |
| | | | | IL | 243671 | A0 | 21 April 2016 |
| | | | | IL | 243671 | B | 29 October 2020 |
| | | | | AP | 201609023 | D0 | 29 February 2016 |
| | | | | NO | 2918662 | T3 | 06 January 2018 |
| | | | | AP | 201609023 | A0 | 29 February 2016 |
| CN | 112457294 | A | 09 March 2021 | None | | | |
| CN | 113272293 | A | 17 August 2021 | AU | 2022287562 | A1 | 02 February 2023 |
| | | | | JP | 2022506146 | A | 17 January 2022 |
| | | | | JP | 7288051 | B2 | 06 June 2023 |
| | | | | AR | 116939 | A1 | 30 June 2021 |
| | | | | ZA | 202102628 | B | 25 October 2023 |
| | | | | JOP | 20210097 | A1 | 30 January 2023 |
| | | | | WO | 2020092667 | A1 | 07 May 2020 |
| | | | | WO | 2020092667 | A8 | 20 May 2021 |
| | | | | KR | 20210086687 | A | 08 July 2021 |
| | | | | MX | 2021005154 | A | 15 July 2021 |
| | | | | BR | 122023020853 | A2 | 16 January 2024 |
| | | | | EA | 202191177 | A1 | 28 July 2021 |
| | | | | BR | 112021008524 | A2 | 03 August 2021 |
| | | | | BR | 112021008524 | A8 | 07 February 2023 |
| | | | | CR | 20210209 | A | 20 May 2021 |
| | | | | CA | 3117927 | A1 | 07 May 2020 |
| | | | | PE | 20211693 | A1 | 01 September 2021 |
| | | | | MA | 54076 | A | 09 February 2022 |
| | | | | US | 2020140411 | A1 | 07 May 2020 |
| | | | | US | 11377438 | B2 | 05 July 2022 |
| | | | | US | 2021387966 | A1 | 16 December 2021 |
| | | | | TW | 202031643 | A | 01 September 2020 |
| | | | | TWI | 784207 | B | 21 November 2022 |
| | | | | CO | 2021005553 | A2 | 30 July 2021 |
| | | | | SG | 11202104326 | TA | 28 May 2021 |
| | | | | DOP | 2021000082 | A | 22 July 2021 |

Form PCT/ISA/210 (patent family annex) (July 2022)

**INTERNATIONAL SEARCH REPORT**
**Information on patent family members**

International application No.
**PCT/CN2024/087393**

| Patent document cited in search report | Publication date (day/month/year) | Patent family member(s) | Publication date (day/month/year) |
|---|---|---|---|
| | | AU 2019372057 A1 | 27 May 2021 |
| | | ECSP 21030066 A | 31 May 2021 |
| | | MY 197128 A | 26 May 2023 |
| | | NI 202100029 A | 13 August 2021 |
| | | US 2022289710 A1 | 15 September 2022 |
| | | EP 3873893 A1 | 08 September 2021 |
| | | IL 282468 A | 31 May 2021 |
| | | GEP 20237568 B | 27 November 2023 |
| | | CL 2021001078 A1 | 29 October 2021 |
| CN 114031518 A | 11 February 2022 | JP 2023548947 A | 21 November 2023 |
| | | WO 2022121517 A1 | 16 June 2022 |
| | | KR 20230093276 A | 27 June 2023 |
| | | TW 202222770 A | 16 June 2022 |
| | | TWI 793868 B | 21 February 2023 |
| | | EP 4230618 A1 | 23 August 2023 |
| | | EP 4230618 A4 | 15 May 2024 |
| | | US 2023303497 A1 | 28 September 2023 |
| | | CA 3197619 A1 | 16 June 2022 |
| | | AU 2021394226 A1 | 15 June 2023 |
| CN 114437062 A | 06 May 2022 | None | |
| CN 116462662 A | 21 July 2023 | WO 2023138599 A1 | 27 July 2023 |
| CN 116655497 A | 29 August 2023 | WO 2023160509 A1 | 31 August 2023 |
| CN 116789644 A | 22 September 2023 | None | |
| US 2007238733 A1 | 11 October 2007 | None | |
| WO 2019014352 A1 | 17 January 2019 | KR 20200026987 A | 11 March 2020 |
| | | CO 2020000145 A2 | 17 January 2020 |
| | | UY 37806 A | 31 January 2020 |
| | | AU 2018300150 A1 | 30 January 2020 |
| | | JP 2020526561 A | 31 August 2020 |
| | | JP 7277431 B2 | 19 May 2023 |
| | | CA 3069720 A1 | 17 January 2019 |
| | | DOP 2020000004 A | 15 July 2020 |
| | | CL 2020000075 A1 | 31 July 2020 |
| | | PE 20201164 A1 | 28 October 2020 |
| | | CR 20200064 A | 03 August 2020 |
| | | SG 11202000230 VA | 27 February 2020 |
| | | TN 2020000001 A1 | 04 October 2021 |
| | | JOP 20200001 A1 | 30 October 2022 |
| | | PH 12020500066 A1 | 28 September 2020 |
| | | BR 112020000553 A2 | 21 July 2020 |
| | | EP 3651752 A1 | 20 May 2020 |
| | | IL 271948 A | 27 February 2020 |
| | | MA 49566 A | 20 May 2020 |
| | | TW 201920081 A | 01 June 2019 |
| | | US 2023286907 A1 | 14 September 2023 |
| | | ECSP 20003147 A | 28 February 2020 |
| | | US 2019016671 A1 | 17 January 2019 |
| | | US 10647661 B2 | 12 May 2020 |
| | | US 2021094906 A1 | 01 April 2021 |

Form PCT/ISA/210 (patent family annex) (July 2022)

**INTERNATIONAL SEARCH REPORT**
**Information on patent family members**

International application No.
**PCT/CN2024/087393**

| Patent document cited in search report | Publication date (day/month/year) | Patent family member(s) | Publication date (day/month/year) |
|---|---|---|---|
| | | US 11603351 B2 | 14 March 2023 |
| WO 2020092187 A1 | 07 May 2020 | US 2022119363 A1 | 21 April 2022 |
| | | EP 3873468 A1 | 08 September 2021 |
| | | EP 3873468 A4 | 26 October 2022 |
| WO 2022192487 A2 | 15 September 2022 | AU 2022232502 A1 | 07 September 2023 |
| | | IL 305721 A | 01 November 2023 |
| | | EP 4304585 A2 | 17 January 2024 |
| | | WO 2022192487 A3 | 20 October 2022 |
| | | BR 112023018313 A2 | 12 December 2023 |
| | | CR 20230481 A | 26 February 2024 |
| | | KR 20230170654 A | 19 December 2023 |
| | | JP 2024509475 A | 01 March 2024 |
| | | CA 3212699 A1 | 15 September 2022 |
| WO 2023150201 A2 | 10 August 2023 | WO 2023150201 A3 | 07 September 2023 |
| WO 2024032774 A1 | 15 February 2024 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014120808 A

### Non-patent literature cited in the description


- **CATTERALL et al.** *Pharmacol Rev.*, 2005, vol. 57 (4), 397-409
- **BLACK et al.** *Neuron.*, 2013, vol. 80 (2), 280-91
- **CATTERALL et al.** *Annu Rev Pharmacol Toxicol.*, 2014, vol. 54, 317-38
- **BENNETT et al.** *Physiol Rev.*, 2019, vol. 99 (2), 1079-1151
- **GOODWIN et al.** *Nat Rev Neurosci.*, 2021, vol. 22 (5), 263-274
- **ALSALOUM et al.** *Nat Rev Neurol.*, 2020, vol. 16 (12), 689-705
- **FABER et al.** *Proc Natl Acad Sci USA.*, 2012, vol. 109 (47), 19444-9
- **KALUZA et al.** *Pflugers Arch.*, 2018, vol. 470 (12), 1787-1801
- **JARVIS et al.** *Proc Natl Acad Sci USA.*, 2007, vol. 104 (20), 8520-5
- **BIERHAUS et al.** *Nat Med.*, 2012, vol. 18 (6), 926-33
- **MERT et al.** *J Am Assoc Lab Anim Sci.*, 2012, vol. 51 (5), 579-85
- **CRANER et al.** *J Neuropathol Exp Neurol.*, 2003, vol. 62 (9), 968-75
- **ROOSTAEI et al.** *Neurology.*, 2016, vol. 86 (5), 410-7
- **SHIELDS et al.** *Ann Neurol.*, 2012, vol. 71 (2), 186-94
- **SHIELDS et al.** *PLoS One.*, 2015, vol. 10 (3), e0119067
- **ZHU et al.** *Elife*, 2020, vol. 9, e57656
- **VERKERK et al.** *Circ Res.*, 2012, vol. 111 (3), 333-43
- **HU et al.** *J Am Coll Cardiol.*, 2014, vol. 64 (1), 66-79
- **DYBKOVA et al.** *Cardiovasc Res.*, 2018, vol. 114 (13), 1728-1737
- **MUROI et al.** *Lung.*, 2014, vol. 192 (1), 15-20
- **KAMBOURIS et al.** *Ann Clin Transl Neurol.*, 2016, vol. 4 (1), 26-35